# NOUVEAU FASCICULE DE BREVET EUROPEEN

(11) **EP 0 432 040 B2**
(45) Date de publication et mention de la décision concernant l'opposition: **17.12.1997**
(45) Mention de la délivrance du brevet: 27.07.1994
(21) Numéro de dépôt: 90403458.4
(22) Date de dépôt: 05.12.1990
(51) Int. Cl.: C07D 417/12, C07D 495/04, A61K 31/425

(54) **Dérivés hétérocycliques d'acylaminothiazoles, leur préparation et compositions pharmaceutiques en contenant**
Heterozyklische Acylaminothiazolderivate, ihre Herstellung und diese enthaltende pharmazeutische Zubereitungen
Heterocyclic derivatives of acylaminothiazole, their preparation and pharmaceutical compositions containing them

(30) Priorité: 06.12.1989 FR 8916122; 04.05.1990 FR 9005669
(43) Date de publication de la demande: 12.06.1991
(73) Titulaire: SANOFI, 75008 Paris (FR)
(72) Inventeur: Bras, Jean-Pierre, F-31500 Toulouse (FR); Frehel, Daniel, F-31000 Toulouse (FR); Gully, Danielle, F-31600 Muret (FR); Valette, Gérard, F-31120 Lacroix (FR)
(74) Mandataire: Polus, Camille

(56) Documents cités:
- EP-A- 0 111 685
- EP-A- 0 169 755
- EP-A- 0 308 885
- EP-A- 0 334 491
- DE-A- 3 705 934
- Proceedings OF THE National Academy of Sciences OF THE UNITED STATES OF AMERICA vol. 83, no. 13, juillet 1986, USA pages 4918 - 4922; Ben E.Evans et al.: "Design of potent,orally effective,nonpeptidal antagonists of the peptide hormone cholecystokinin"

## Description

La présente invention concerne des dérivés hétérocycliques, antagonistes de la cholécystokinine et de la gastrine.

La cholécystokinine (CCK), est une hormone polypeptidique présente in vivo sous plusieurs formes comportant de 8 à 39 aminoacides. Elle a de nombreuses activités physiologiques sur les voies biliaires, le tractus gastrointestinal comme sur les systèmes nerveux central et périphérique et on peut se référer à l'article de J.E. Morley dans Life Sciences vol. 30, p. 479-493 (1982) qui fait une revue détaillée de ses propriétés. Deux populations différentes de récepteurs de la CCK ont été mises en évidence à l'aide d'antagonistes spécifiques; ceux de type A présents en particulier dans le pancréas, la vésicule biliaire et certaines zones de système nerveux central, tandis que ceux de type B se trouvent surtout dans le système nerveux central.

La gastrine est une hormone polypeptidique qui agit notamment sur la sécrétion acide de l'estomac; ses 5 aminoacides C-terminaux sont identiques à ceux de la CCK.

On a déjà décrit des composés antagonistes de la gastrine et/ou de la CCK, notamment le proglumide, le p-chlorobenzoyl-L-tryptophane, ou plus récemment, des dérivés de benzodiazépine antagonistes spécifiques soit des récepteurs CCK A, tel que le 3S(-)-N-[méthyl-1 oxo-2 phényl-5 dihydro-2,3 1H-benzodiazépine-1,4 yl-3]indolecarboxamide-2 (cf. Eur. J. Pharmacology 162, 273-280, (1989)), soit des récepteurs CCK B, tel que la 3R(+)-N-[méthyl-1 oxo-2 phényl-5 dihydro-2,3 1H-benzodiazépine-1,4-yl-3] N'-[méthyl-3 phényl]urée.

Les composés selon l'invention sont des dérivés hétérocycliques d'acylamino-2 thiazoles de formule I : 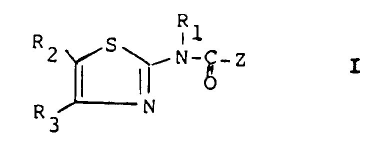 dans laquelle
R₁ représente l'atome d'hydrogène, un groupe alkyle en C₁ à C₄ ou un groupe phénylalkyle avec alkyle en C₁ à C₃; un groupe aminoalkyle de formule -Z₁-NR₄R₅ dans laquelle Z₁ représente un alkylène en C₂ à C₄ et R₄ et R₅ représentent indépendamment H ou un alkyle en C₁ à C₄ ou forment avec l'atome d'azote auquel ils sont liés un hétérocycle saturé tel que morpholino, pyrrolidinyle, piperidino, piperazinyle ou (C₁-C₃)alkyl-4 piperazinyle; un groupe carboxyalkyle éventuellement estérifié de formule -Z₂-COOR₆ dans laquelle Z₂ représente un alkylène en C₁ à C₄ et R₆ représente H ou un alkyle en C₁ à C₆; un groupe cyanoalkyle en C₂ à C₅; un groupe carbamoylalkyle de formule -Z₃-CONR₇R₈, dans laquelle Z₃ représente un alkylène en C₁ à C₄ et R₇ et R₈ représentent indépendamment H ou un alkyle en C₁ à C₄ ou avec N un hétérocycle comme NR₄R₅; un groupe hydroxyalkyle en C₂ à C₆, ou un groupe alkoxyalkyle en C₂ à C₁₀,
R₂ représente l'atome d'hydrogène ou un groupe alkyle en C₁ à C₄;
R₃ représente un groupe cycloalkyle en C₅ à C₈ éventuellement substitué par un ou des groupes alkyles en C₁ à C₄; un groupe aromatique tel qu'un phényle portant éventuellement un ou plusieurs substituants choisis parmi les atomes d'halogène, notamment le chlore ou le fluor, les groupes (C₁-C₆) alkyle, et alkoxy et thioalkoxy en C₁ à C₃, les groupes nitro et trifluorométhyle ou tel qu'un hétérocycle comportant au moins un hétéroatome choisi parmi O, S et N, notamment un furyle, thiényle, pyrrolyle, pyrazolyle, imidazolyle, pyridyle, pyrazinyle, oxazolyle et thiazolyle, éventuellement substitués par un groupe alkyle en C₁ à C₃ ou un atome d'halogène ou R₂ et R₃ considérés ensemble représentent le groupe 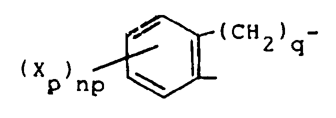 fixé par le carbone du phényle en position 4 du noyau thiazolyle et dans lequel q vaut 1 à 4, portant éventuellement un ou plusieurs (np) substituants Xp, identiques ou différents choisis parmi les atomes d'halogéne, les groupes alkyle et alkoxy en C₁ à C₃, les groupes nitro et trifluorométhyle, np valant de 0 à 3, et
Z représente un hétérocyle comportant un ou plusieurs hétéroatomes choisis parmi S et N, condensé avec un noyau aromatique qui peut aussi comporter un hétéroatome choisi parmi O, S, N et qui peut être substitué parun ou des groupes choisis parmi les atomes d'halogène, les groupes alkyle et alkoxy en C₁ à C₃, benzyloxy, nitro, amino et trifluorométhyle, ainsi que les sels d'addition de ces composés avec des acides et des bases minérales ou organiques; les sels non toxiques pharmaceutiquement acceptables sont préférés mais d'autres sels utilisables pour isoler ou purifier les composés de formule I sont aussi un objet de l'invention.

Les groupes alkyle, alkylène, alkoxy et thioalkoxy peuvent être linéaires ou ramifiés. Z représente notamment les groupes benzothiényle, benzimidazolyle, benzothiazolyle, indolyle, isoindolyle, indolinyle, isoindolinyle, quinolyle, isoquinolyle, quinoxalinyle, quinazolinyle, cinnolinyle et thiéno[2,3-c] ou [3,2-c] pyridyle.

Lorsque Z représente un groupe indolyle ou indolinyle de formule 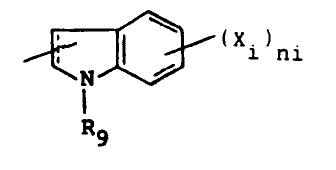 dans laquelle (Xᵢ)ₙᵢ représente les éventuels substituants du noyau aromatique, R₉ peut représenter H; un groupe alkyle en C₁ à C₄; un groupe hydroxyalkyle en C₁ à C₆; un groupe alkoxyalkyle en C₂ à C₁₀, éventuellement cyclisé, tel qu'un tétrahydropyrannyle;
un groupe aminoalkyle de formule -Z₄-NR₁₀R₁₁ dans laquelle Z₄ représente un alkylène en C₂ à C₄ et R₁₀ et R₁₁ représentent indépendamment H ou un alkyle en C₁ à C₄ ou forment avec l'atome d'azote auquel ils sont liés un groupe hétérocyclique saturé tel que morpholino, pyrrolidinyle, pipéridino, piperazinyle ou (C₁-C₃)alkyl-4 pipérazinyle; un groupe carboxyalkyle éventuellement estérifié de formule -Z₅-COOR₁₂ dans laquelle Z₅ représente un alkylène en C₁ à C₄ et R₁₂ représente H, un benzyle ou un alkyle en C₁ à C₆; un groupe cyanoalkyle avec alkyle en C₁ à C₄; un groupe carbamoylalkyle de formule -Z₆-CONR₁₃R₁₄ dans laquelle R₁₃ et R₁₄ représentent indépendamment H ou un alkyle en C₁ à C₆ ou forment avec N un hétérocycle saturé comme NR₁₀R₁₁, et Z₆ est un alkylène en C₁ à C₄; un groupe acyle de formule COR₁₅ dans laquelle R₁₅ représente un alkyle en C₁ à C₄ ou un phényle; un groupe alkoxycarbonyle de formule COOR₁₆ dans laquelle R₁₆ représente t-butyle ou benzyle.

Parmi les composés de formule I, on préfère ceux dans lesquels R₁ représente H, un alkyle ou un aminoalkyle et parmi ceux-ci plus particulièrement ceux dans lesquels Z représente un groupe indolyle substitué ou non sur l'azote; parmi les groupes R₃, on préfère les phényles au moins ortho substitués, lorsque R₂ représente H.

Les composés de formule I peuvent être préparés par condensation d'un aminothiazole de formule II 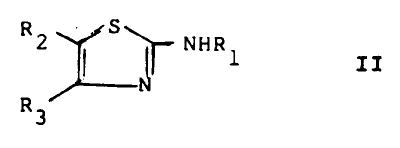 dans les conditions habituelles d'acylation d'une fonction amine, avec un acide de formule Z'COOH dans laquelle Z' représente Z ou un dérivé de Z dans lequel les fonctions sensibles de Z ont été protégées, et R₁, R₂, R₃ et Z ont la même signification que dans la formule I, ou avec une forme activée de l'acide Z'COOH, telle qu'un halogénure d'acide, un anhydride d'acide, et de préférence un anhydride mixte comme un anhydride carbonique, ou un ester activé, obtenu avec les réactifs couramment utilisés en synthèse peptidique.

Les composés de formule I dans laquelle Z est remplacé par Z', sont aussi un objet de l'invention en tant qu'intermédiaires de synthèse; en outre, certains présentent in vivo l'activité thérapeutique de leurs homologues, notamment du fait de leur métabolisation en composés de formule I.

Lorsque des fonctions ont été protégées, on effectue après la condensation la réaction de déprotection convenable, si nécessaire.

De nombreux aminothiazoles de formule II sont connus.

Les aminothiazoles nouveaux peuvent être préparés selon l'un des procédés précédemment décrits, notamment dans Bull. Soc. Chim. (C) p. 2498-2503 (1963).

De façon générale, on fera réagir une thiourée avec une cétone alpha-halogénée, et de préférence alphabromée, selon le schéma réactionnel : 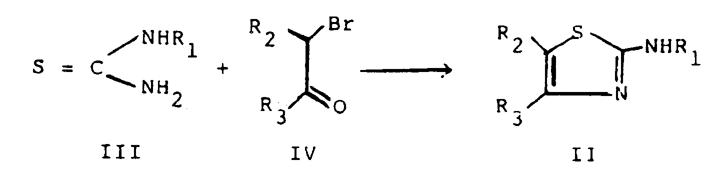 R₁, R₂ et R₃ ayant la même signification que dans la formule II.

La préparation de divers composés II dans lesquels R₁ représente un groupe aminoalkyle est décrite dans EP-A-0 283 390.

Les cétones alpha-halogénées et les thiourées peuvent être préparées pardes procédés dont les principes sont décrits dans les ouvrages généraux; ainsi les cétones alpha-bromées (IV) peuvent être préparées par action sur R₂CH₂COR₃ du brome en milieu acide acétique ou du bromure cuivrique dans un solvant organique tel que l'acétate d'éthyle, un solvant chloré ou leurs mélanges. Les cétones aromatiques de départ sont préparées en général par réaction de Friedel et Crafts, tandis que les méthylcétones aliphatiques peuvent être préparées par action du diazométhane sur les chlorures d'acides carboxyliques convenables suivie de l'hydrolyse de la diazocétone correspondante.

Les cétones aromatiques alpha-chlorées peuvent être préparées par réaction de Friedel et Crafts avec le chlorure d'acide alpha-chloré convenable, ou par chloroacétylation avec la N-N diméthylchloroacétamide lorsque R₂ = H.

Les thiourées substituées III de formule H₂NCSNHCH₂COOR₆ sont préparées par estérification de l'acide commercial et celles de formule H₂NCSHNCH₂₋CONR₄R₅ par amidification de l'acide; les autres peuvent être préparées par action de l'amine R₁NH₂ sur (CH₃)₃C-CO-N=C=S ou sur C₆H₅-CO-N=C=S.

Ces derniers composés sont obtenus respectivement par action des chlorures de pivaloyle ou de benzoyle sur le thiocyanate de potassium dans un solvant inerte anhydre, tel qu'une cétone; on peut effectuer la condensation avec l'amine R₁NH₂ sans isoler les acylisothiocyanates. Lorsque R₁ comporte un groupe alkoxycarbonyle, on préfère mettre en oeuvre le dérivé pivaloyle pour effectuer l'hydrolyse de l'acylthiourée intermédiaire en milieu acide fort anhydre, sans hydrolyse de la fonction alkoxycarbonyle; l'hydrolyse de la benzoylthiourée est en général réalisée par action d'une solution aqueuse d'une base minérale, telle que NaOH.

Certains des acides ZCOOH, ou Z'COOH, sont connus et même commerciaux; les autres sont préparés en utilisant les méthodes connues pour des molécules analogues.

Ainsi les acides indolecarboxyliques, de formule Z''COOH : 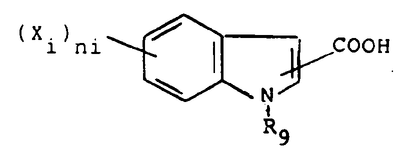 dans laquelle R₉ représente un groupe alkoxycarbonylalkyle peuvent être préparés à partir des acides indolecarboxyliques commerciaux ou obtenus par des procédés classiques, en suivant le schéma réactionnel (a) 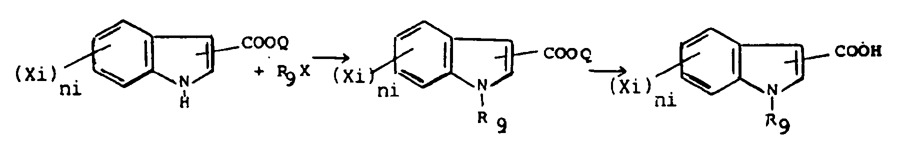 dans lequel X représente un atome d'halogène et Q représente le groupe benzyle.

Les esters benzyliques du schéma (a) sont préparés par action de l'acide correspondant sur l'alcool benzylique, en présence de l'un des agents d'activation de la fonction acide couramment utilisés en synthèse peptidique tel que :
- le carbonyl-1,1' diimidazole pour lequel on pourra se référer à Synthesis p. 833 (1982)
- le N,N'-dicyclohexylcarbodiimide en présence de diméthylamino-4 pyridine pour lequel on pourra se référer à J. Org. Chem. 55 (4) p. 1390 (1990)
- le N-éthyl N'-(diméthylamino-3 propyl) carbodiimide en présence de diméthylamino-4 pyridine pour lequel on pourra se référer à J. Org. Chem. 47 1962 (1982)
- le chlorure de N,N-bis(oxo-2 oxazolidinyl--3) phosphorodiamide pour lequel on pourra se référer à Synthesis p. 547 (1980)
- l'hexafluorophosphate de benzotriazolyloxy tris-(diméthylaminophosphonium) pour lequel on pourra se référer à Synthesis p. 413 (1977).

On peut aussi isoler le dérivé ainsi activé de l'acide avant de le faire réagir sur l'alcool benzylique.

Les esters benzyliques du schéma (a) peuvent aussi être préparés par réaction de l'acide indolecarboxylique et de l'alcool, activés sous forme de dérivés phosphonium comme il est décrit dans Tetrahedron 36 p. 2409 (1980) ou dans Synthesis p. 1 (1981).

La base mise en oeuvre lors de la fixation de R₉ sur l'azote de l'ester benzylique est de préférence une base forte anhydre telle qu'un hydrure alcalin; le milieu réactionnel est alors un solvant aprotique polaire, stable en présence d'une base forte, telle que le diméthylformamide ou le diméthoxyéthane; la réaction est effectuée à une température comprise entre 15°C et 80°C environ.

L'élimination du groupe benzyle, après la N-alkylation est effectuée de façon classique par action d'au moins un équivalent d'hydrogène, en présence d'un catalyseur tel que le palladium sur du charbon, sur l'ester en solution dans un alcool ou le diméthylformamide, éventuellement sous une légère pression.

Les acides indole carboxylique de formule Z''COOH dans laquelle R₉ représente un groupe hydroxyalkyle, alkoxyalkyle, aminoalkyle, cyanoalkyle ou carbamoylalkyle peuvent être préparées selon le schéma réactionnel (a) dans lequel Q représente un groupe alkyle en C₁ à C₃; l'hydrolyse de l'ester peut alors en effet être effectuée en milieu acide ou basique et par exemple par action d'une base minérale en milieu hydroalcoolique, à une température comprise entre 40°C et la température de reflux du solvant, sans modification de R₉.

Par ailleurs, certains des acides ZCOOH sont peu stables ou portent une fonction qui pourrait réagir lors de la condensation avec l'aminothiazole et il est préférable de les mettre en oeuvre sous une forme protégée Z'COOH.

Ainsi les dérivés (I) dans lesquels Z représente 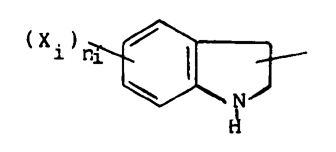 et dans laquelle (Xᵢ)ₙᵢ représente les éventuels substituants, peuvent être préparés à partir des composés obtenus par condensation de l'aminothiazole avec des composés de l'acide indolinylcarboxylique Z'COOH, de formule 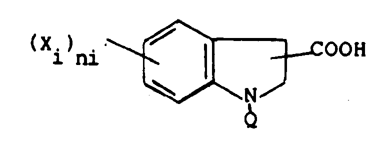 dans laquelle Q représente un groupe habituellement utilisé pour la protection des groupes NH₂ dans les réactions de condensation des acides aminés, tel que COO(t-C₄H₉); le groupe protecteur Q peut être éliminé du composé de formule V 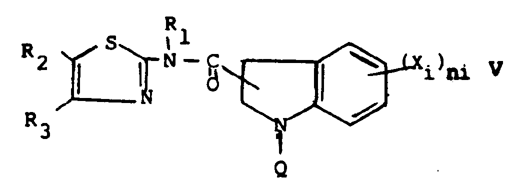 obtenu après la condensation avec le dérivé (II), par action d'un acide fort en milieu anhydre, tel que CF₃CO₂H dans CH₂Cl₂ ou HCl dans CH₃CO₂C₂H₅.

On a constaté que dans le cas où Z représente 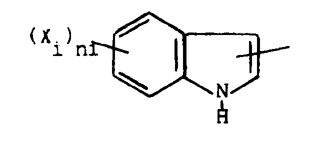 on peut protéger l'azote de l'acide indole carboxylique pour la condensation avec l'aminothiazole par un groupe tétrahydropyrannyle, un groupe acyle, tel qu'acétyle, ou un groupe carboxylique tel que benzyloxycarbonyle ou tertio-butyloxycarbonyle; ces groupes protecteurs sont fixés sur l'azote puis éliminés éventuellement après la condensation par des méthodes connues en soi, et par exemple par action d'une solution aqueuse acide diluée sur le dérivé tétrahydropyrannyle, par action d'un acide anhydre sur le t-butylcarbamate, par hydrogénation catalytique pour le benzylcarbamate ou par hydrolyse en milieu basique du dérivé acétyle.

Les acides Z''COOH dans lesquels R₉ est COOC(CH₃)₃ ou COOCH₂C₆H₅ peuvent être préparés par action du chloroformiate correspondant ClCOOC(CH₃)₃ ou ClCOOCH₂C₆H₅ sur Z"COOH dans lequel R₉ = H, en présence d'une base telle que la triéthylamine et la diméthylamino-4 pyridine, dans un solvant tel que l'acétonitrile ou le chlorure de méthylène.

Les acides Z''COOH dans lesquels R₉ est un groupe acyle peuvent être préparés par action du chlorure ou de l'anhydride d'acide sur Z''COOH dans lequel R₉ = H en présence d'un équivalent de triéthylamine et de diméthylamino-4 pyridine, par exemple dans le chlorure de méthylène.

Les chlorures d'acide de formule ZCOCl, Z'COCl, Z''COCl peuvent être préparés, notamment, par action de SOCl₂ ou d'un mélange de POCl₃ et P₂O₅ sur l'acide correspondant, en général en l'absence de solvant et à la température de reflux du milieu.

Les anhydrides mixtes de formule ZCOOCOY', Z'COOCOY' ou Z''COOCOY', dans lesquelles Y' représente un groupe alkyle en C₁ à C₄, peuvent être préparés par action d'un chloroformiate d'alkyle sur l'acide, en présence d'une base, généralement une amine tertiaire telle que la triéthylamine; cette réaction est le plus souvent effectuée dans un solvant tel que le dichlorométhane, le dichloroéthane ou le chloroforme.

Parmi les esters activés de formule ZCOOY'', Z'COOY'' ou Z''COOY'', on peut préparer ceux dans lesquels
- Y'' représente 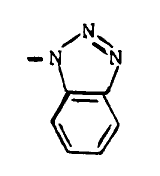 par action de l'hydroxy-1 benzotriazole sur l'acide en présence de dicyclohexylcarbodiimide selon le mode opératoire décrit dans J. Am. Chem. Soc. 93, 6318-6319 (1971), ou par action de l'hexafluorophosphate de benzotriazolyl-1-oxytris(diméthylamino)phosphonium selon le mode opératoire décrit dans Synthesis 751-752 (1976); ceux dans lesquels
- Y'' représente 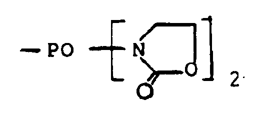 par action du chlorure de N,N-bis(oxo-2 oxazolidinyl--3)phosphorodiamide selon le mode opératoire décrit dans 3. Am. Chem. Soc. 107, 4342-4343 (1985).

La condensation de l'aminothiazole (II) avec l'acide sous forme d'ester activé peut être effectuée dans un solvant dont la nature est choisie selon la solubilité des composés et le type d'activation de la fonction acide, de préférence en présence d'une base, par exemple une amine tertiaire telle que la triéthylamine; la réaction est en général effectuée à une température comprise entre 0°C et 30°C.

Lorsque les composés de formule I comportent dans R₁ ou Z un groupe acide carboxylique, ceux-ci sont préparés par hydrolyse d'un ester de formule I correspondant soit en milieu acide, soit de préférence en milieu basique, par exemple par action d'une base minérale, tel qu'un hydroxyde alcalin, en milieu hydroalcoolique.

Il est aussi avantageux dans le cas où Z représente le groupe indolyle non substitué sur l'azote de préparer le composé de formule I par déshydrogénation du dérivé indolinyle correspondant de formule VI 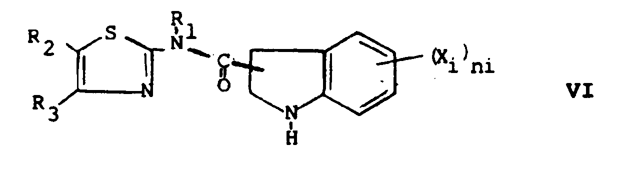 dans
laquelle R₁, R₂,R₃, (Xᵢ)ₙᵢ ont les mêmes significations que précédemment.

La réaction est effectuée par action sur le reste indoline des réactifs déshydrogénants classiques, tel que la tétrachloro-2,3,5,6 benzoquinone-1,4 (p-chloranile), la dichloro-2,3 dicyano-5,6 benzoquinone-1,4 (DDQ) ou le cyclohexène en présence de Pd dans des solvants inertes à haut point d'ébullition tels que le diphényléther, le xylène, le diméthoxy-1,2 éthane ou le méthoxy-2 éthyléther à température élevée, et de préférence à la température de reflux du solvant.

Les sels d'addition des composés de formule I avec des acides ou des bases sont préparés de la façon habituelle par introduction de l'acide, ou de la base dans une solution du composé de formule I. Le sel est isolé, selon ses caractéristiques de solubilité, après évaporation du solvant ou addition d'un non-solvant.

Les composés de formule I et leurs sels sont des antagonistes de cholécystokinine, plus ou moins sélectifs des récepteurs de type A ou B, et des antagonistes de la gastrine plus ou moins puissants.

Leur affinité pour le récepteur CCK A a été déterminée, in vitro, en utilisant la méthode décrite dans ce qui suit, dont le principe est celui mentionné dans Life Sciences 37(26) 2483-2490 (1985); elle consiste à déterminer le déplacement de la CCK 8S iodée de ses sites de fixation sur un homogénat de pancréas de rat : des quantités aliquotes de suspension membranaire pancréatique (100 µg de protéines par ml) dans un tampon TRIS, HCl (50 mM), de pH 7,4 contenant MgCl₂ (5mM), bacitracine (0,1 mg/ml), fluorure d'acide méthylphénylméthane sulfonique (0,1 mg/ml), sont incubés 40 minutes à 25°C en présence de CCK 8S iodée (2000 Ci/mmole, soit 50 pM de concentration finale) et de concentrations croissantes de la substance à étudier; la réaction est arrêtée au bout de 40 minutes par centrifugation. Après élimination du surnageant, la radioactivité du culot est mesurée. Par ailleurs, la fixation non spécifique est déterminée en présence de CCK 8S à la concentration de 1 µM.

Dans ces conditions, la concentration inhibitrice de 50% de la fixation (CI₅₀) est pour les produits de l'invention inférieure à 10⁻⁷M, et pour un grand nombre de l'ordre de 10⁻⁹M, alors que dans les mêmes conditions celle de la benzodiazépine carboxamique citée dans l'introduction de la description est d'environ 10-⁸M.

Leur affinité pour les récepteurs CCK B a été déterminée en étudiant le déplacement de la CCK 8S iodée de ses sites de fixation spécifiques présents sur des homogénats de cortex de cobaye en utilisant le même mode opératoire que pour les récepteurs CCK A, mais pour une suspension membranaire à 600 µg de protéines/ml et avec un tampon HEPES (10 mM) à pH 6,5 contenant NaCl (130 mM), Mg Cl₂(5 mM), EDTA (1 mM) et bacitracine (250 mg/l) et l'incubation étant de 2 heures.

A la concentration de 10⁻⁵M tous les produits déplacent plus de 25% de la CCK 8S marquée du récepteur B; certains ont des CI₅₀ de l'ordre de 10⁻⁸M, inférieures à celles de la benzodiazépine urée racémique citée dans l'introduction.

L'affinité pour le récepteur de la gastrine des composés les plus spécifiques des CCK B a été étudiée, selon la méthode décrite dans ce qui suit, dont le principe est celui mentionné dans J. Receptor. Res. 3(5) 647-655 (1983); : des aliquotes de glandes gastriques de cobaye dans un tampon à pH 7,4 HEPES (24,5 mM) comprenant NaCl (98 mM), KCl (6 mM), NaH₂PO₄ (2,5 mM), pyruvate (5 mM), glutamate (5 mM), CaCl₂(0,5 mM), MgCl₂(1mM), glucose (11,5 mM), glutamine (1 mM), albumine bovine (0,4 g/100 ml) ont été incubées 90 minutes à 37°C dans un bain marie en présence de gastrine (2-17) iodée (2000 Ci/mmol; 70 pM) et de concentrations croissantes des produits à étudier. La réaction a été arrêtée par centrifugation et la radioactivité vité du culot mesurée; la fixation non spécifique a été déterminée en présence de gastrine (2-17) à 1 µM. Les composés de l'invention ont une CI₅₀ comprise entre 10⁻⁵M et 10⁻⁸M.

On a aussi montré que les composés de l'invention ont une activité antagoniste de celle de CCK. Ceci a été mis en évidence in vitro par la mesure de l'inhibition par les produits à tester, de la sécrétion d'amylase par des acini de rats stimulée par la CCK 8S, selon une méthode analogue à celle décrite dans J. Biol. Chem. 254(12) 5321-5327 (1979) mais avec des tissus pancréatiques de cobaye. Les composés ont une CI₅₀ de 10⁻⁶ a 10⁻⁷M, ordre de grandeur de la CI₅₀ de la benzodiazépine carboxamide racémique précédemment citée.

Enfin, in vivo, chez la souris, les composés ayant une bonne affinité pour les récepteurs de la gastrine, ont déclenché l'activité de vidange gastrique inhibée par l'administration sous-cutanée de CCK 8S dans le protocole décrit dans Life Sciences, 39 1631-1638 (1986); la DE₅₀ (dose efficace 50) ainsi déterminée est nettement inférieure à celle du proglumide, antagoniste connu de la gastrine.

Comme ces composés sont peu toxiques, ils sont utilisables comme médicaments, pour le traitement des désordres physiologiques résultant d'une hypersécrétion de ces peptides ou d'une dysrégulation des systèmes hormonaux biologiques dans lesquels ils sont impliqués, au niveau de la sphère intestinale ou dans le système nerveux central, suivant leur spécificité. On peut se référer à la revue des applications thérapeutiques des antagonistes de CCK et de gastrine publiée dans "Proceedings of International Symposium on Gastrin and cholecystokinin" -7-11 sept. 1987 - Ed. J.P. Bali, J. Martinez - Elsevier Science Pub. BV.

Notamment les antagonistes de la CCK seront utiles dans le traitement des dyskinésies intestinales, comme le syndrome du colon irritable, dans le traitement des pancréatites aiguës ou chroniques ou dans celui des carcinomes pancréatiques, mais aussi pour régulariser l'appétit, ou, associé aux analgésiques opiacés, dans le traitement de la douleur.

Quant aux antagonistes plus sélectifs de la gastrine, ils seront utiles dans le traitement et la prévention des ulcères gastriques, dans le traitement du syndrome de Zollinger-Ellison, dans celui des hyperplasies des cellules G de l'antre ou pour les malades ayant des tumeurs cancéreuses de l'oesophage, de l'estomac ou de l'intestin.

Parmi les antagonistes de la cholecystokinine au niveau des récepteurs A, on préfère les composés :
- N-[(triméthyl-2,4,6 phényl)-4 thiazolyl-2] indole carboxamide-2 et les dérivés substitués sur l'azote de l'indole, notamment par (C₁-C₄) alkyle tel que CH₃, CH₂COOR, avec R étant H ou (C₁-C₄) alkyle, notamment CH₃ et (CH₂)₂NR₁₀R₁₁ avec R₁₀ et R₁₁ étant (C₁-C₄)alkyle, tel que CH₃,
- N[(triméthoxy-2,4,6 phényl)-4 thiazolyl-2] indole carboxamide-2 et les dérivés substitués sur l'azote de l'indole, notamment par (C₁-C₄) alkyle tel que CH₃, CH₂COOR où R est H ou (C₁-C₄) alkyle, tel que CH₃,
- N-[(diméthyl-2,6 phényl)-4 thiazolyl-2] indole carboxamide-2 et les dérivés substitués à l'azote de l'indole notamment par CH₂COOR, R étant H ou (C₁-C₄) alkyle, tel que CH₃,
- N-[(diméthoxy-2,6 phényl)-4 thiazolyl-2] indole carboxamide-2 et les dérivés substitués à l'azote de l'indole, notamment par CH₂COOR, R étant H ou (C₁-C₄) alkyle tel que CH₃,
- N-[(dichlor-2,6 phényl)-4 thiazolyl-2] indole carboxamide-2 et les dérivés substitués à l'azote de l'indole, notamment par CH₂COOH et (CH₂)₂-NR₁₀R₁₁ avec R₁₀ et R₁₁ étant (C₁-C₄) alkyle, tel que CH₃,
- N-[(méthyl-2 phényl)-4-thiazolyl-2] indole carboxamide-2 et les dérivés substitués à l'azote l'indole, notamment par CH₂COOH,
- N-[(méthoxy-2 phényl)-4 thiazolyl-2] indole carboxamide-2 et les dérivés substitués à l'azote de l'indole, notamment par CH₂COOH,
- N-[(chloro-2 phényl)-4 thiazolyl-2] indole carboxamide-2 et les dérivés substitués à l'azote de l'indole notamment par CH₂COOR, avec R étant H ou (C₁-C₄) alkyle tel que CH₃,
- N-[(méthyl-4 phényl)-4 thiazolyl-2] indole carboxamide-2,
- N-[(méthoxy-4 phényl)-4 thiazolyl-2] indole carboxamide-2.

Parmi les antagonistes de la cholecystokinine au niveau des récepteurs B et des antagonistes de la gastrine, on préfère les composés :
- N[(triméthoxy-2,4,6 phényl)-4 thiazolyl-2] indole carboxamide-2 et les dérivés substitués à l'azote de l'indole, par CH₂COOR, avec R étant H ou (C₁-C₄) alkyle, tel que CH₃,
- N-[(diméthoxy-2,6 phényl)-4 thiazolyl-2] indole carboxamide-2 et les dérivés substitués à l'azote de l'indole, par CH₂COOR, avec R étant H ou (C₁-C₄) alkyle tel que CH₃.

Les médicaments selon l'invention comprennent au moins l'un des composés de formule I ou l'un de ses sels avec un acide ou une base pharmaceutiquement acceptable, éventuellement associé avec les excipients habituels pour constituer une forme pharmaceutique administrable de façon classique par voie orale, transmuqueuse, parentérale ou rectale. Les doses administrées dépendent de la nature et de l'importance de la maladie, du composé et de la voie d'administration. Elles seront généralement comprises entre 20 et 100 mg par jour chez l'homme adulte par voie orale et 3 à 10 mg en injection.

Les compositions pharmaceutiques selon l'invention pourront être, pour l'administration orale, présentées sous forme de comprimés, de pilules, de gélules ou de granulés ou encore de solution, de suspension ou de gel. Pour l'administration parentérale, les compositions de l'invention se présenteront sous forme de solution, de suspension ou d'émulsion dans une huile ou tout solvant injectable, éventuellement à base aqueuse contenant les adjuvants classiques dans ce type de formulation.

Pour l'administration locale, sur la peau ou sur les muqueuses, les compositions selon l'invention se présenteront en crème, pommade ou sous forme de dispositif transdermique, tandis que pour l'administration rectale, elles seront sous forme de suppositoire ou de capsule rectale.

Dans ce qui suit, on décrit des exemples de mise en oeuvre de l'invention, ainsi que les procédés de préparation de certains intermédiaires de synthèse de formule II et IV. Les points de fusion indiqués ont été déterminés en capillaire. Les spectres de résonance magnétique nucléaire, (RMN) ont été enregistrés par rapport au tétraméthylsilane.

### Préparation d'alphabromocétones de formule IV

A) (Triméthyl-2,4,6 phényl)(bromométhyl)cétone (IV : R₂ = H; R₃ = 2,4,6-(CH₃)₃C₆H₂-)
   On dissout 50 g de (triméthyl-2,4,6 phényl)(méthyl)cétone dans 200 ml d'acide acétique glacial et on ajoute goutte à goutte, en maintenant le milieu réactionnel à une température inférieure à 10°C, 31,8g de brome. En fin d'addition, on laisse revenir à température ambiante et abandonne à cette température pendant 2 heures. On verse le milieu réactionnel sur 500 ml d'eau glacée et extrait la phase aqueuse avec de l'éther diéthylique. Les extraits organiques sont lavés avec une solution aqueuse saturée de bicarbonate de sodium, puis avec de l'eau salée et séchés sur du sulfate de magnésium anhydre. L'évaporation du solvant laisse une huile qui est utilisée sans autre purification dans l'étape ultérieure.
B)(Triméthoxy-2,4,6 phényl)(bromométhyl) cétone (IV : R₂ = H; R₃ = 2,4,6-(OCH₃)₃C₆H₂-)
   On porte au reflux une suspension de 45,3g de bromure cuivrique CuBr₂ dans 150 ml d'acétate d'éthyle et ajoute rapidement A cette température 25,1g de (triméthoxy-2,4,6,phényl)(méthyl)cétone en solution dans 150 ml de chloroforme. On note l'apparition d'un abondant précipité jaune verdâtre. On laisse au reflux le milieu réactionnel pendant 2 H 30. On laisse ensuite revenir à température ambiante et filtre les sels insolubles que l'on lave avec de l'acétate d'éthyle. Les phases organiques sont traitées avec du noir animal : après élimination du solide par filtration, on concentre sous pression réduite pour obtenir une huile que l'on purifie par chromatographie sur une colonne de silice (éluant : cyclohexane/acétate d'éthyle 6/4-V/V).
   Rendement : 60%. Huile
C) (Cyclohexyl)(bromométhyl)cétone (IV : R₂ = H; 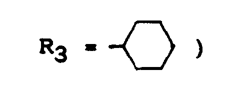
   On dissout 7,2 g de chlorure de l'acide cyclohexanecarboxylique dans 50 ml d'éther diéthylique. Après refroidissement à 0°C, on ajoute une solution de 0,1 mole de diazométhane dans 100 ml d'éther diéthylique, préparée extemporanément à partir de 21,5 g de p-tolylsulfonylméthylnitrosamide (Diazald^{R}) selon le procédé décrit dans Organic Synthesis Coll. Vol IV p. 250. On abandonne pendant 24 h à température ambiante.
   A la solution de diazocétone ainsi obtenue, on ajoute 9,1 ml d'une solution aqueuse d'acide bromhydrique à 48% (p/V), en maintenant la température du milieu réactionnel à 0°C. On laisse environ 12 heures sous agitation à température ambiante eton verse le milieu réactionnel dans de l'eau. On décante la phase organique et la sèche sur du sulfate de sodium anhydre. L'évaporation du solvant laisse une huile que l'on utilise sans purification dans l'étape ultérieure.
D) (diméthoxy-2,6 éthyl-4 phényl) (chlorométhyl) cétone (IV R₂=H; R₃ = 2,6 -(OCH₃)₂ 4-C₂H₅C₆H₂, et Cl au lieu de Br)
   On dissout dans 100 ml d'hexane, 8,3 g de diméthoxy-3,5 éthylbenzène et 6,1 g de tétraméthylèthylène-diamine et on ajoute à 0°C 32,8 ml de butyllithium. Après 1 heure à 10°C, on introduit la suspension crème obtenue dans une solution à -10°C de 6,1 g de N-méthyl N-méthoxy chloroacétamide dans 50 ml de tétrahydrofuranne. Après 1 heure à une température inférieure à 0°C, on laisse revenir à température ambiante avant d'ajouter 100 ml d'eau. Le produit cherché est extrait dans l'éther éthylique et purifié par chromatographie sur gel de silice. F = 72°C.
E) (N-méthyl pyrrolyl) (chlorométhyl) cétone (IV R₂ = H 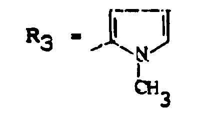 et Cl au lieu de Br)
   préparé selon la méthode décrite dans Synthesis p. 212-213 (1990).
F) (diméthoxy-2,6 hydroxy-4 phényl) (chlorométhyl) cétone et (diméthoxy-2,4 hydroxy-6 phényl) (chlorométhyl) cétone préparées selon la méthode décrite dans J. Chem. Soc. p. 3112 (1957)

Les cétones bromées du tableau I ont été préparées en utilisant l'un des procédés mis en oeuvre selon A ou B

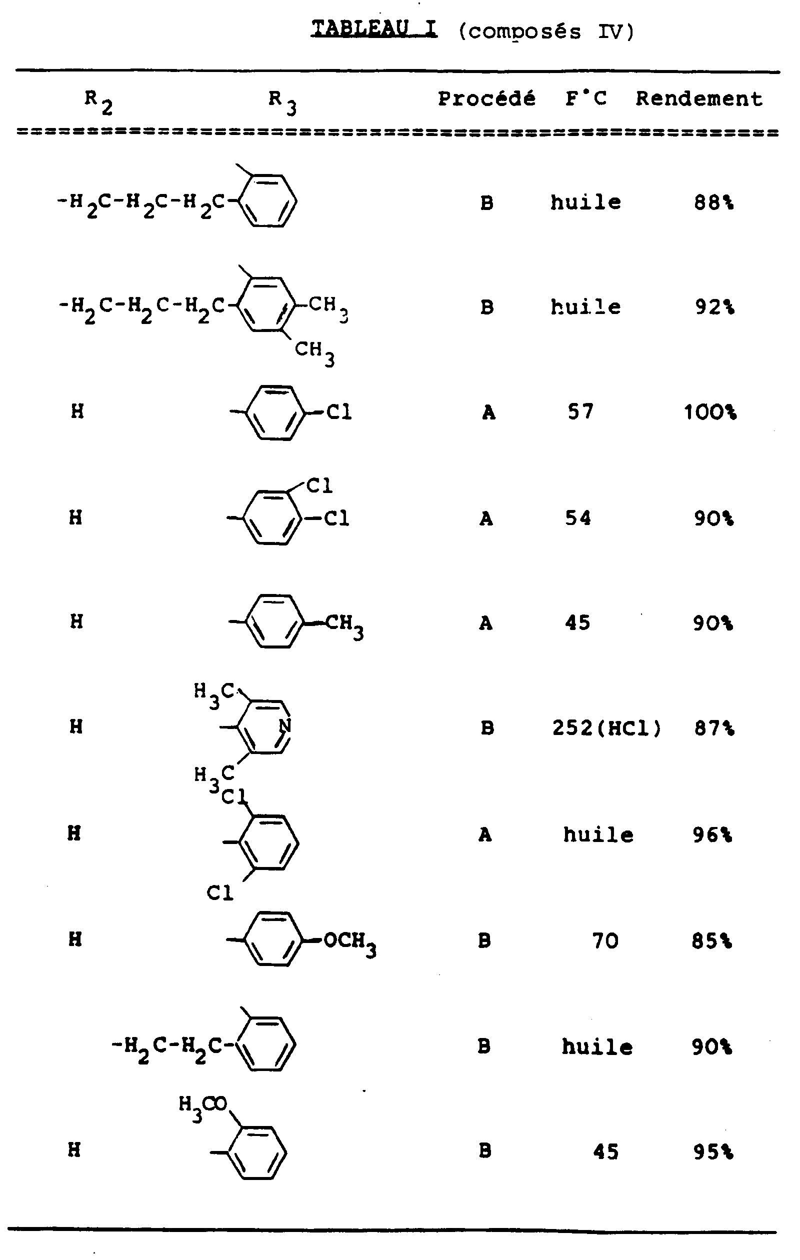

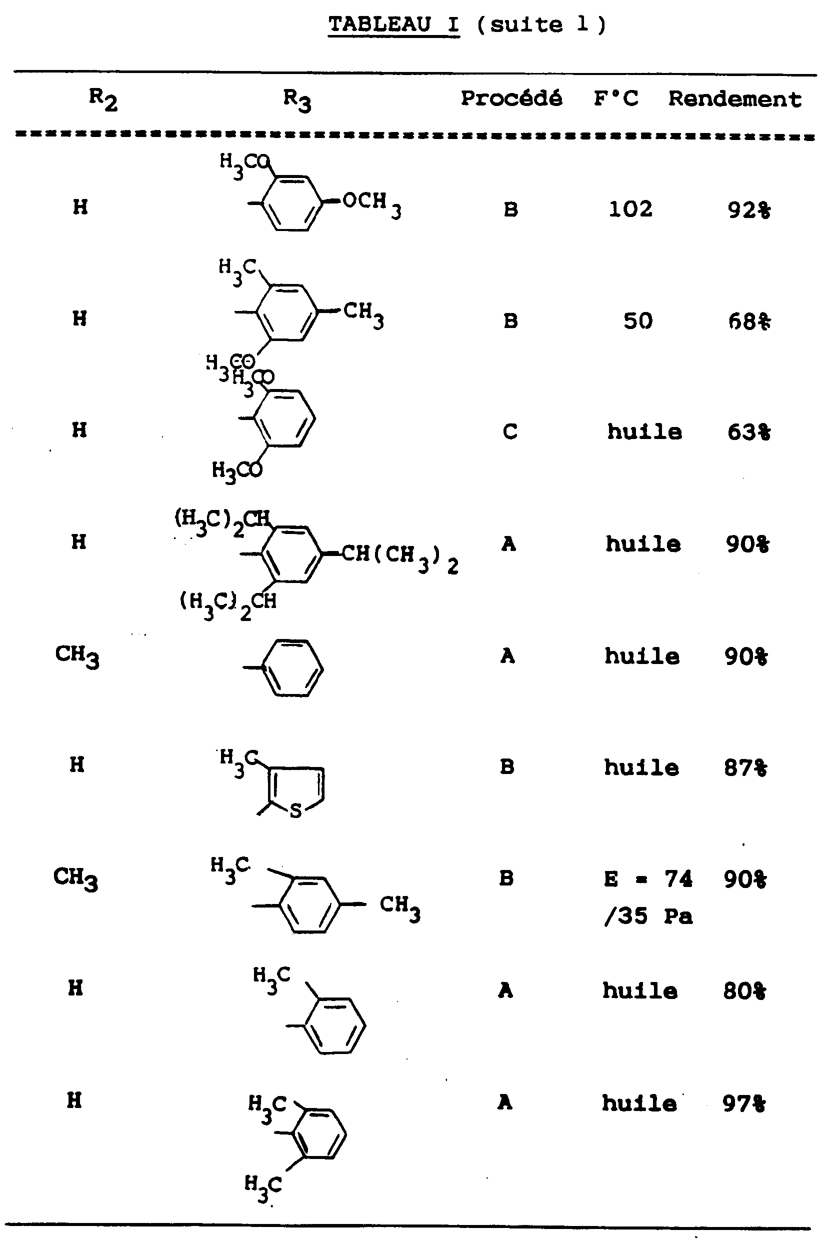

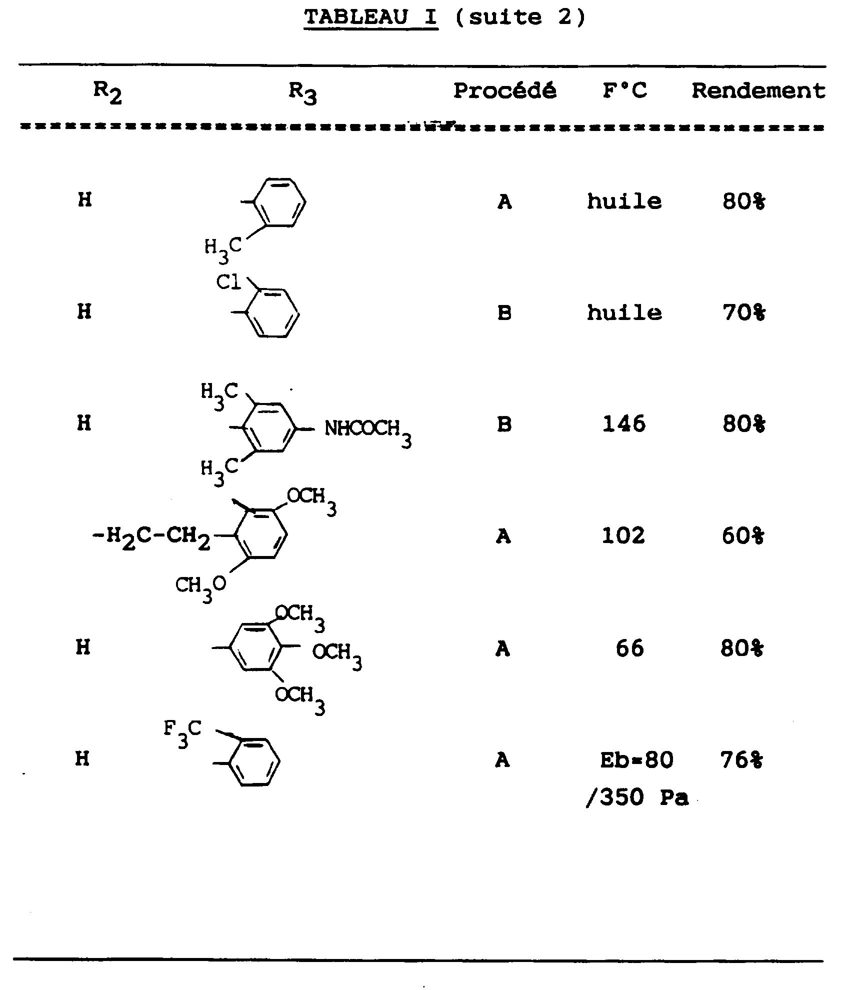

### Préparation d'aminothiazoles de formule II

a) Amino-2 (triméthyl-2,4,6 phényl)-4 thiazole (II : R₁ = R₂ = H; 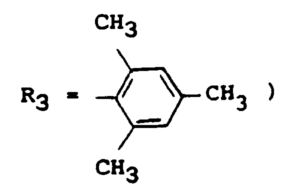
   On porte au reflux pendant 3 heures une solution de 80 g de (triméthyl-2,4,6 phényl)(bromométhyl)cétone et de 35 g de thiourée dans 250 ml de méthanol. Après refroidissement du milieu réactionnel, on filtre le précipité et le lave abondamment avec de l'éther diéthylique. Après concentration du filtrat au tiers du volume initial on récupère un second jet de cristaux. Rendement : 70%.
   F = 168°C. Le bromhydrate préparé par action de HBr dans l'éthanol fond à 295°C.
b) (Triméthoxy-2,4,6 phényl)-4 méthylamino-2 thiazole (II : R₁ = CH₃; R₂ = H; 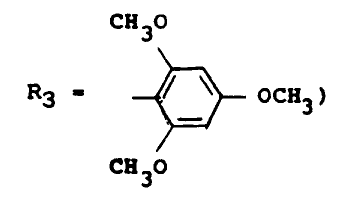
   On porte au reflux pendant 8 heures, un mélange de 5 g de (triméthoxy-2,4,6 phényl)(bromométhyl)cétone et de 1,72 g de N-méthyl thiourée dans 40 ml de méthanol. On évapore à sec le milieu réactionnel et recristallise les cristaux obtenus dans l'éthanol. Rendement : 83%.
   Point de fusion du bromhydrate 246°C.

Les aminothiazoles de formule II dans laquelle R₁ = H, figurant dans le tableau II, ont été préparés en appliquant les procédés précédents.

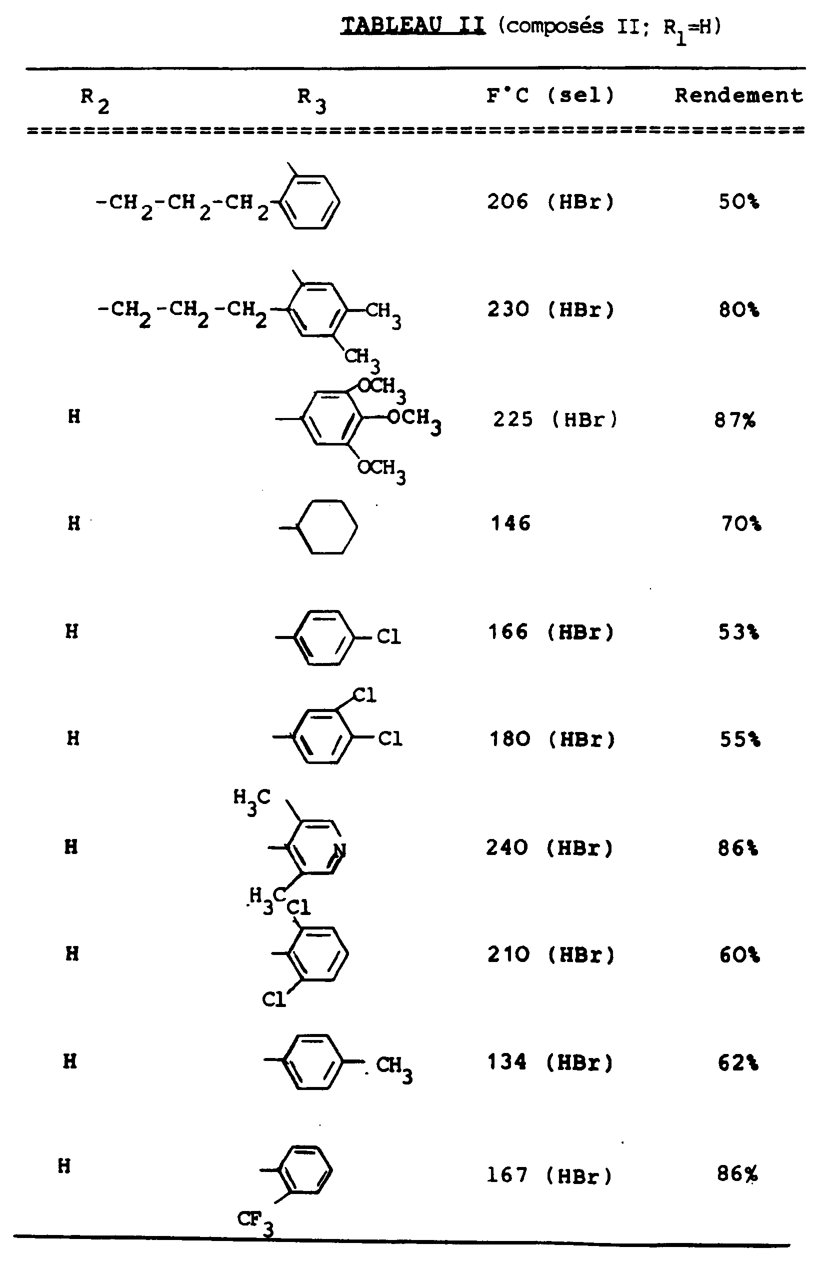

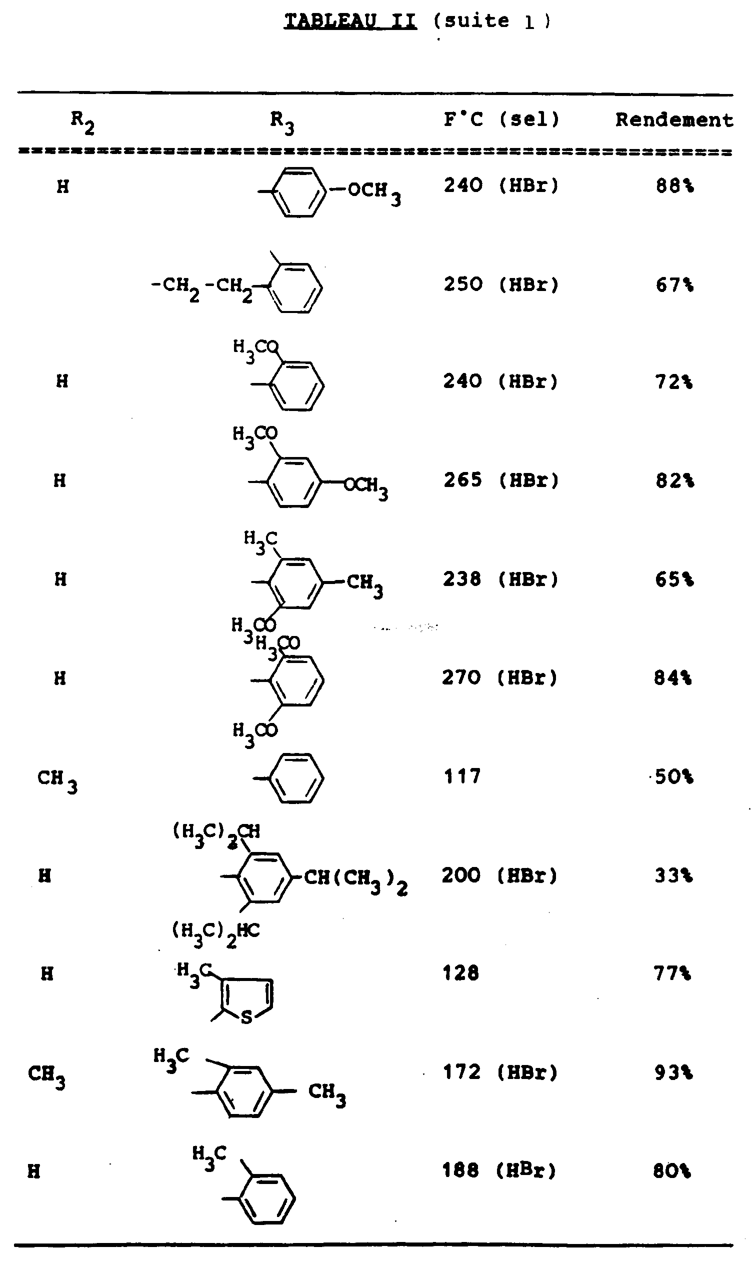

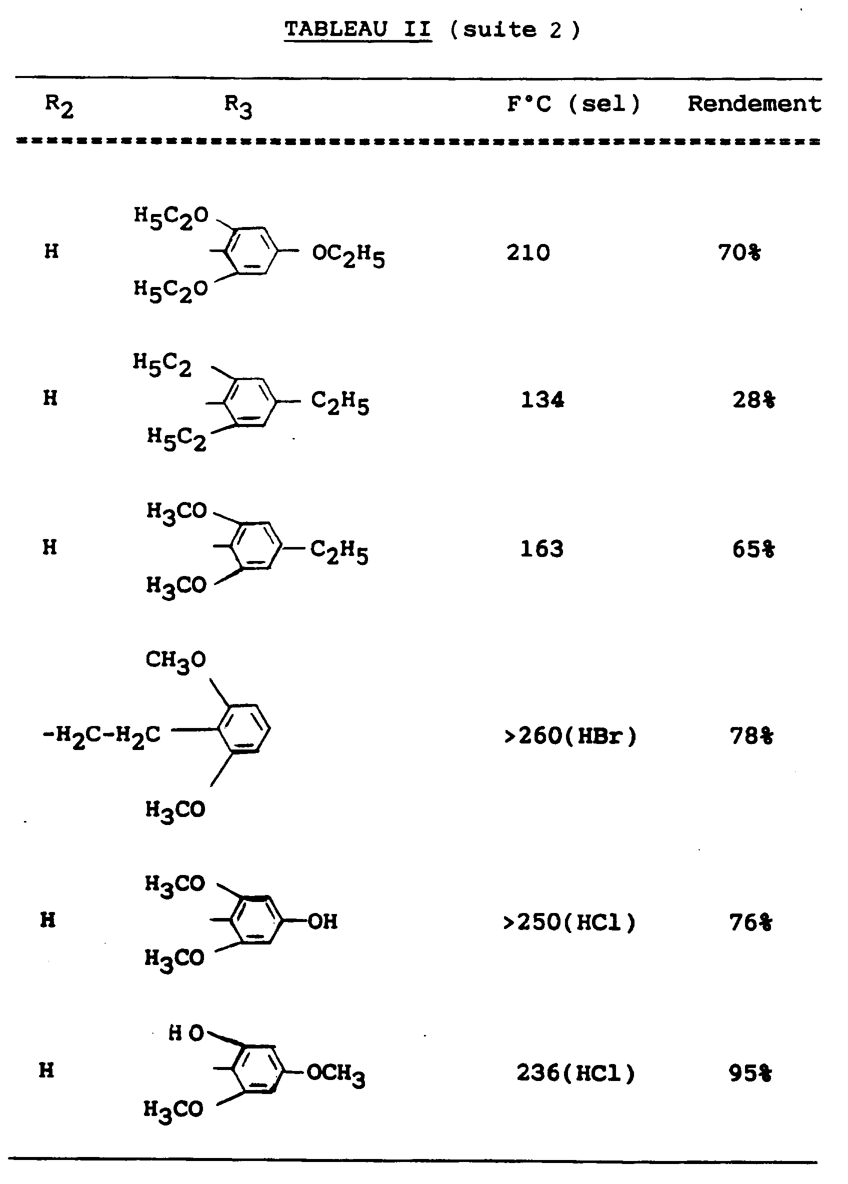

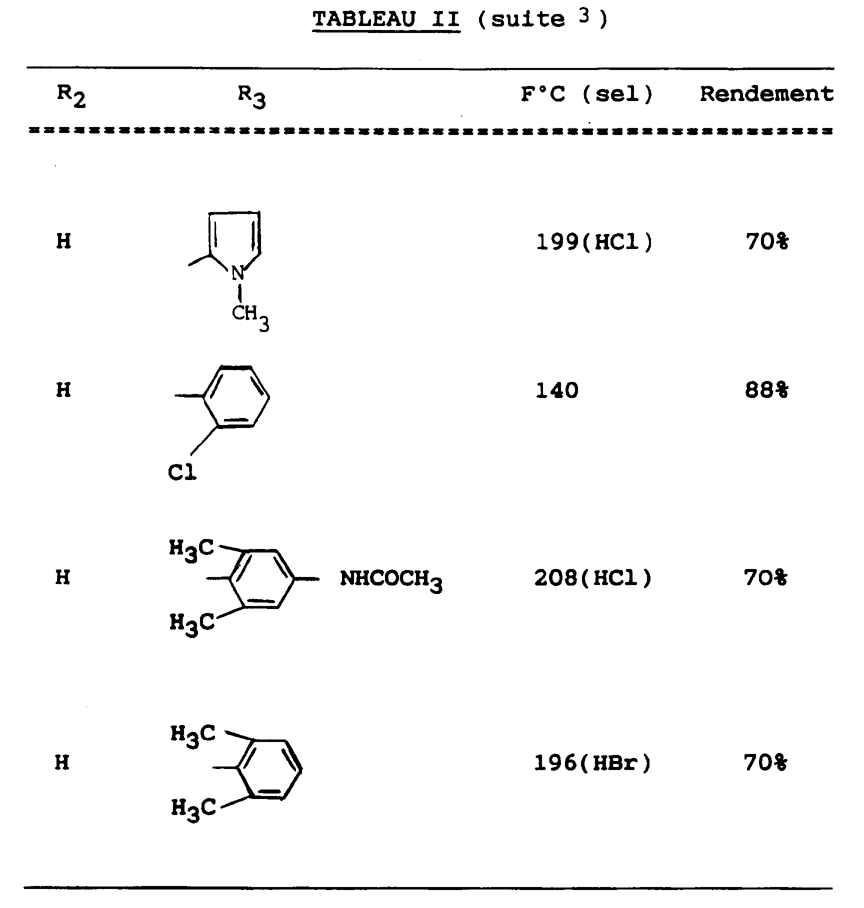

### Préparation des acides indolecarboxyliques :

A') Indolecarboxylate-2 de benzyle
   On introduit 5 g de N,N'-carbonyldiimidazole dans une solution de 5 g d'acide indole-2 carboxylique dans 50 ml de tétrahydrofuranne sec; après 12 heures d'agitation à température ambiante, on ajoute 3,7 g d'alcool benzylique et on porte le milieu réactionnel à sa température de reflux; celle-ci est maintenue durant 8 heures, avant d'éliminer le solvant par distillation sous pression réduite. Le résidu est dissous dans l'acétate d'éthyle et la phase organique lavée par une solution aqueuse de NaOH N puis séchée avant évaporation du solvant.
   Le résidu jaune est recristallisé dans l'isopropanol, F = 136°C; rendement 85%.
B')(méthoxycarbonylméthyl-1) indolecarboxylate-2 de benzyle (Z''COOQ : 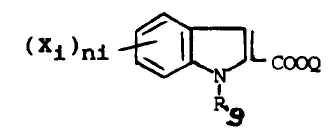 R₉ = CH₂COOCH₃; Xᵢ = H; Q = C₆H₅).
   On introduit lentement 5 g d'indolecarboxylate-2 de benzyle en solution dans 20 ml de diméthylformamide, dans 30 ml d'une suspension de 1 g de NaH dans 30 ml de diméthylformamide puis 3,1 g de bromoacétate de méthyle. Après 12 heures sous agitation à température ambiante, le milieu est versé dans un volume d'eau glacée puis extrait par de l'acétate d'éthyle. La phase organique, séchée, est concentrée et le résidu recristallisé dans de l'éthanol aqueux (95% - V/V). F = 94°C - rendement 87%.
C') Acide(méthoxycarbonylméthyl)-1 indole carboxylique-2 (Z''COOH : R₉ = CH₂COOCH₃).
   On dissout 3g de l'ester obtenu selon B') dans un mélange de 50 ml d'éthanol et 10 ml de diméthylformamide et on ajoute 300 mg de palladium sur charbon (5%). Le milieu est hydrogéné à température ambiante sous une pression de 0,1 MPa. Lorsque l'absorption d'hydrogène a cessé, on dégaze le milieu et le filtre sur un lit de talc. Le résidu obtenu après évaporation des solvants est lavé avec de l'éther diisopropylique. F = 194°C; rendement 90%.
D') Acide (N,N diméthylamino-2 éthyl)-1 indolecarboxylique-2 (Z''COOH : R₉ = (CH₂)₂N(CH₃)₂)
   a) ester éthylique
      On introduit en atmosphère inerte, par portions 2,8 g de NaH dans une solution de 5 g d'indolecarboxylate-2 d'éthyle dans 40 ml de diméthylformamide; à la fin du dégagement de H₂, on ajoute par portions 4,2 g de chlorhydrate de N-(chloro-2 éthyl)-N,N-diméthylamine. Après 12 heures d'agitation à température ambiante, le milieu réactionnel est versé dans un volume d'eau glacée, puis extrait par l'acétate d'éthyle. La phase organique, séchée est évaporée à siccité. Huile - rendement 90%.
   b) acide
      6 g de l'huile obtenue en a) sont dissous dans 50ml d'éthanol aqueux (95% - V/V) avec 2g de KOH en pastilles. Le milieu réactionnel est maintenu 1 heure à sa température de reflux, puis le solvant est éliminé sous pression réduite. Le résidu est dissous dans 150 ml d'eau et on fait barboter du gaz carbonique dans le milieu durant 1 heure. Le précipité formé est isolé. F = 228°C; rendement 83%.
E') Acide(N,N-diméthylamino-3 propyl)-1 indolecarboxylique-2 (R₉ = (CH₂)₃N(CH₃)₂) préparé selon le procédé décrit en D'). F = 160°C; rendement 70%.
F') Acide(méthoxy-2 éthyl)-1 indolecarboxylique-2 (Z''COOH : R₉ = CH₂CH₂OCH₃).
   On meten suspension 1,4 g d'hydrure de sodium dans 10 ml de diméthylformamide eton ajoute goutte à goutte une solution de 5 g d'indolecarboxylate-2 d'éthyle dissous dans 25 ml de diméthylformamide. Après une heure à température ambiante, le milieu est refroidi à 5°C, et on ajoute 4,04 g de méthoxy-1 bromo-2 éthane; après 12 heures à température ambiante, le milieu est porté à 60°C pendant 1 heure, puis après refroidissement, versé dans un volume d'eau glacée; on extrait avec de l'acétate d'éthyle, et après séchage, on concentre la phase organique.
   Le résidu obtenu est repris par 50 ml d'éthanol contenant 1,7 g d'hydroxyde de sodium et le mélange porté à la température de reflux du solvant durant 2 heures.
   La solution est alors versée dans un volume d'eau et acidifiée jusqu'à pH = 2 par addition d'une solution aqueuse de HCl N. L'acide cherché précipite. F = 150°C; rendement 82%.
G') Acide(tétrahydropyrannyl-2)-1 indolecarboxylique-2 (Z''COOH : R₉ = tétrahydropyrannyl-2).
   On ajoute, par portions, 1,5 g d'hydrure de sodium dans une solution de 5 g d'indolecarboxylate-2 d'éthyle dans 40 ml de diméthylformamide; lorsque le dégagement gazeux est terminé, le milieu est refroidi à 0°C et on y introduit lentement 4,5 g de chloro-2 tétrahydropyranne dissous dans 10 ml de diméthylformamide. Après 12 heures d'agitation à température ambiante, le milieu est versé dans un volume d'eau glacée puis extrait avec de l'acétate d'éthyle. La phase organique est séchée et concentrée pour donner avec 95% de rendement une huile, constituée de (tétrahydropyrannyl-2)-1 indolecarboxylate-2 d'éthyle.
   Le chloro-2 tétrahydropyranne a été préparé par saturation à 0°C de dihydropyranne avec HCl; il distille à 40°C sous 2000 Pa.
   L'ester hui eux est introduit dans 80 ml d'éthanol contenant 1,6 g de NaOH en pastilles; le milieu est porté 1 heure à sa température de reflux puis le solvant est distillé sous pression réduite. Le résidu est dissous dans 50 ml d'eau, puis traité par 10 g d'une résine échangeuse d'ion cationique sous forme H⁺ (Amberlite^{R} IRN77), avant extraction par l'acétate d'éthyle.
   La phase organique, séchée, est amenée à siccité et le résidu recristallisé dans l'acétate d'éthyle. F = 216°C; rendement 86%.
H') Acide t-butyloxycarbonyl-1 indolecarboxylique-2
   On introduit goutte à goutte 30 ml d'une solution de 6g de dicarbonate de di-tertiobutyle dans 30 ml d'une solution de 4 g d'acide indole-2 carboxylique, 4 ml de triéthylamine et 0,4 g de diméthylamino-4 pyridine dans l'acétonitrile. Après 2 heures d'agitation à température ambiante et élimination du précipité formé, l'acétonitrile est éliminé par distillation et le résidu est dissous dans le chlorure de méthylène. La phase organique est lavée à l'eau, séchée, et concentrée à sec.
   F = 117°C ; Rendement: 66%.
I') Acide benzyloxycarbonyl-1 indolecarboxylique-2
   En appliquant le mode opératoire précédent, on obtient ce composé qui fond à moins de 40°C.

### EXEMPLE 1

### N-[(méthoxy-4 phényl)-4 thiazolyl-2](méthyl-1) indolecarboxamide-2 (I : R₁ = R₂ = H;

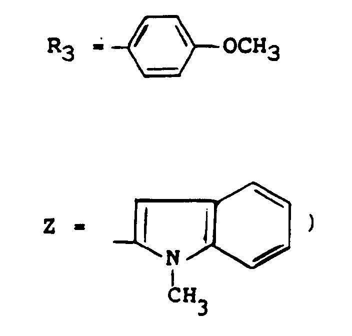

On dissout 1g d'amino-2 (méthoxy-4 phényl)-4 thiazole dans 20 ml de diméthylformamide eton ajoute successivement, au milieu réactionnel et à température ambiante, 0,6 g d'acide méthyl-1 indolecarboxylique-2, puis 1,6g d'hexafluorophosphate de benzotriazolyl-1 oxytris(diméthylamino)phosphonium (BOP), puis 0,7 g de triéthylamine. On laisse sous bonne agitation à température ambiante pendant 12 heures environ avant de verser le milieu réactionnel dans 100 ml d'eau glacée; la phase aqueuse obtenue est ensuite extraite 2 fois par 50 ml d'acétate d'éthyle. Les extraits organiques sont séchés sur du sulfate de magnésium anhydre et évaporés à siccité. Le solide résiduel est purifié par chromatographie sur colonne de silice (éluant : CH₂Cl₂). Rendement 25%. F : 100°C.
RMN¹H : (250 MHz,DMSOd6) : δ (ppm) : 3,8(s,3H); 4,1(s,3H); 6,9-8,0 (m,10H); 12,7(s,1H).

### EXEMPLE 2

### N-méthyl N[(triméthyl-2,4,6 phényl)-4 thiazolyl-2] quinoléinecarboxamide-3 (I : R₁ = CH₃; R₂ = H;

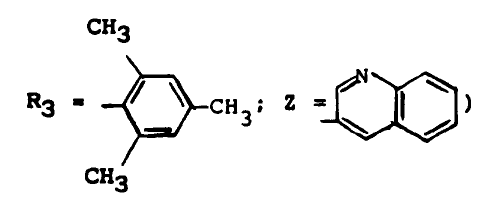

On dissout 1 g de [N-méthyl[(triméthyl-2,4,6 phényl)-4]-amino]-2 thiazole dans 20 ml de diméthylformamide et ajoute 1,6 g de triéthylamine, puis on introduit goutte à goutte sous agitation une solution de 0,73 g du chlorhydrate du chlorure de l'acide quinolylcarboxylique-3 dissous dans 10 ml de diméthylformamide. On porte ensuite à 50°C pendant 2 heures avant d'évaporer à sec le milieu réactionnel et de reprendre le résidu dans 100 ml de dichlorométhane. La solution organique est lavée à l'eau et séchée sur du sulfate de magnésium anhydre avant d'évaporer le solvant; le résidu huileux est purifié par chromatographie flash (silice, éluant : acétate d'éthyle/dichlorométhane-1/9). Rendement : 45%

Le trifluoroacétate préparé par action de un équivalent de CF₃COOH sur l'amine en solution dans le dichlorométhane fond à 160°C.
RMN¹H : 80 MHz(DMSOd6) δ (ppm) : 2,2(s,6H); 2,4(s,3H); 3,8(s,3H); 7,0(s,2H); 7,3(s,1H); 7,7-8,3(m,4H); 8,9(s,1H); 9,3(s,1H).

Les exemples suivants décrits dans le tableau III, ont été préparés en appliquant le procédé de l'exemple 1.

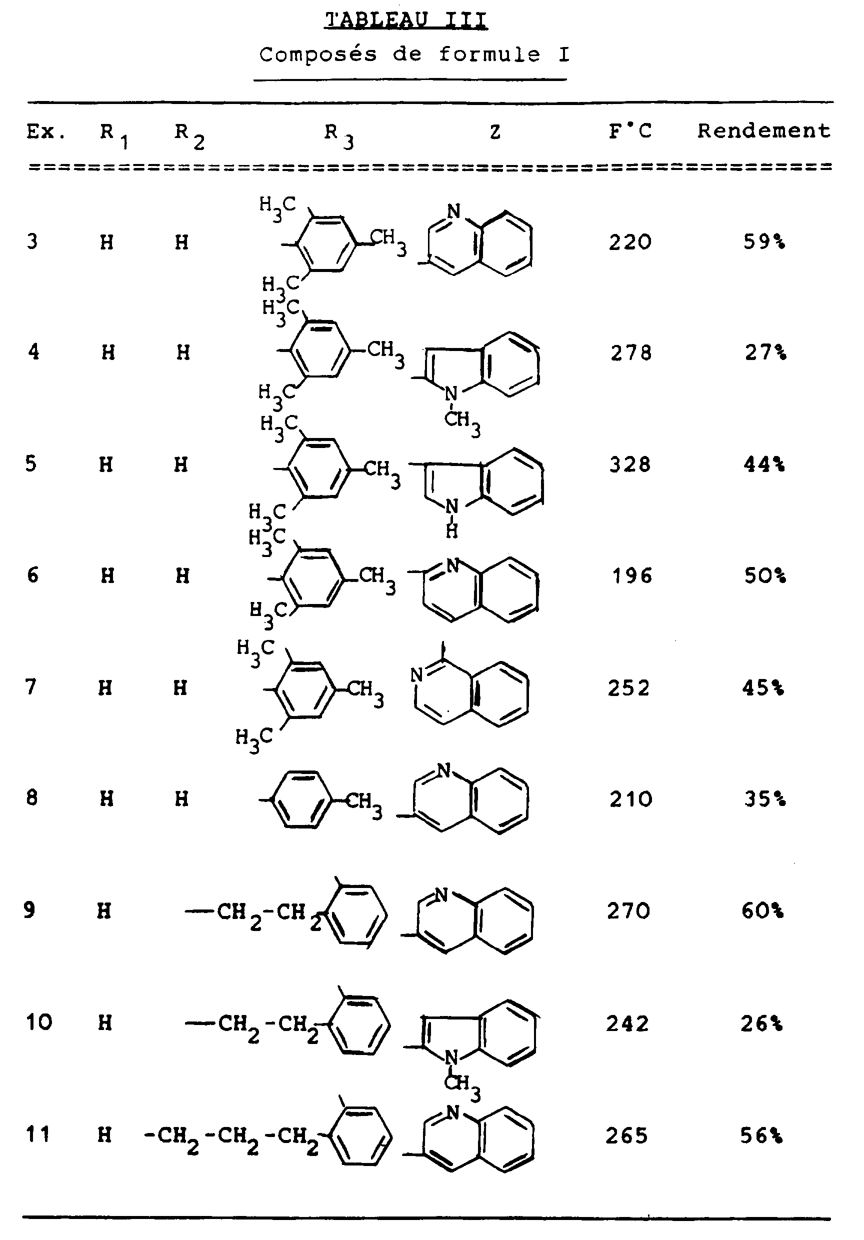

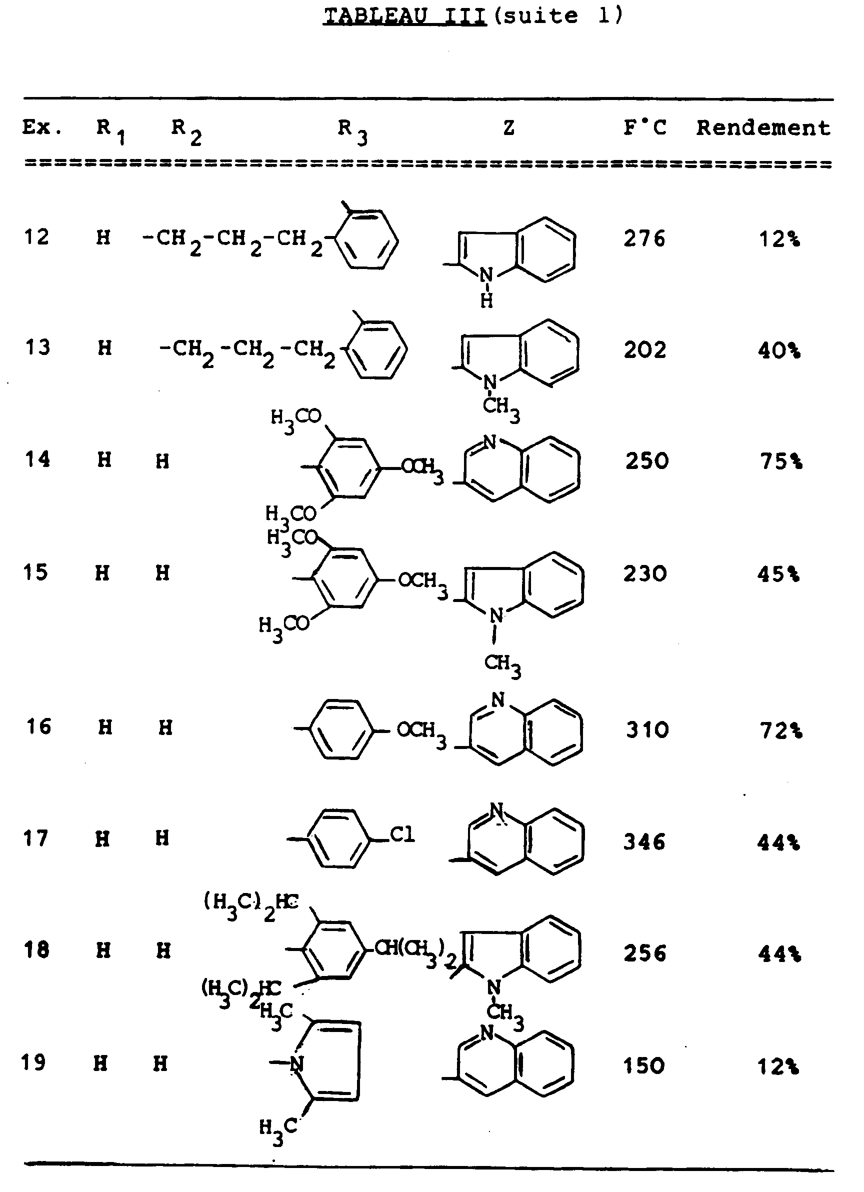

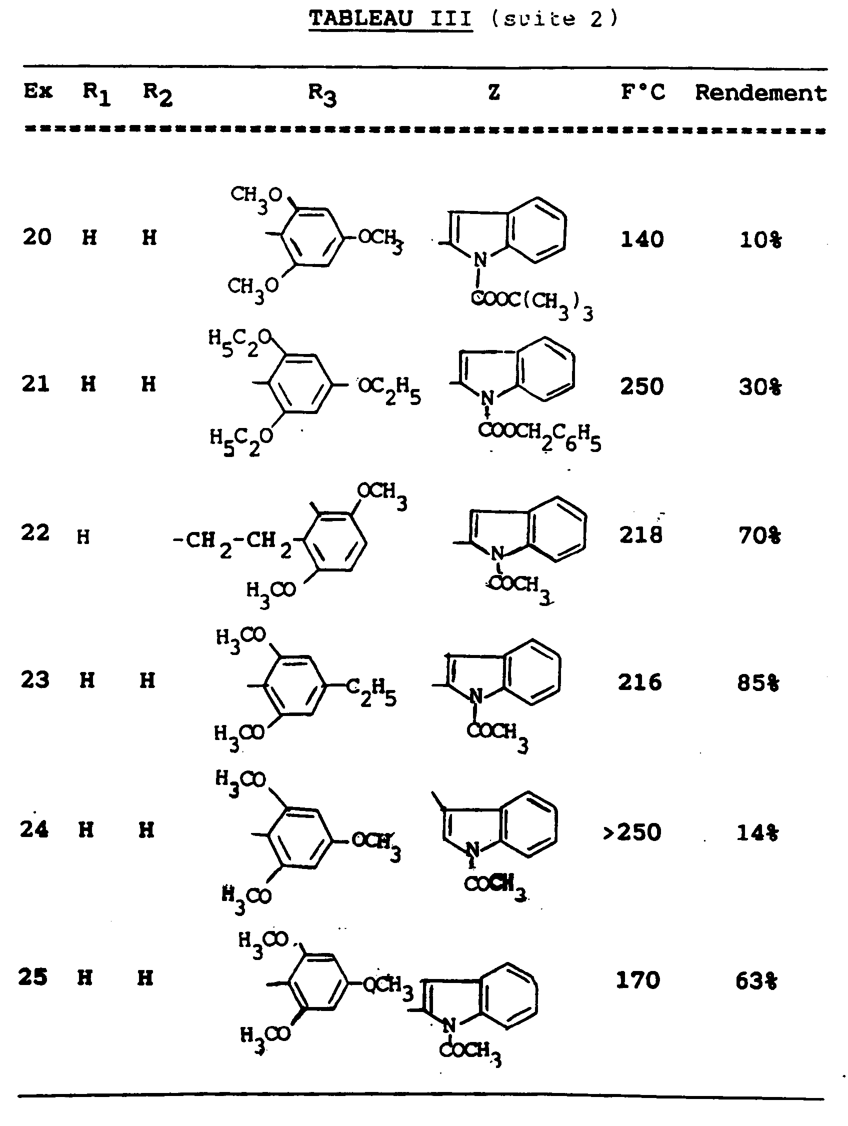

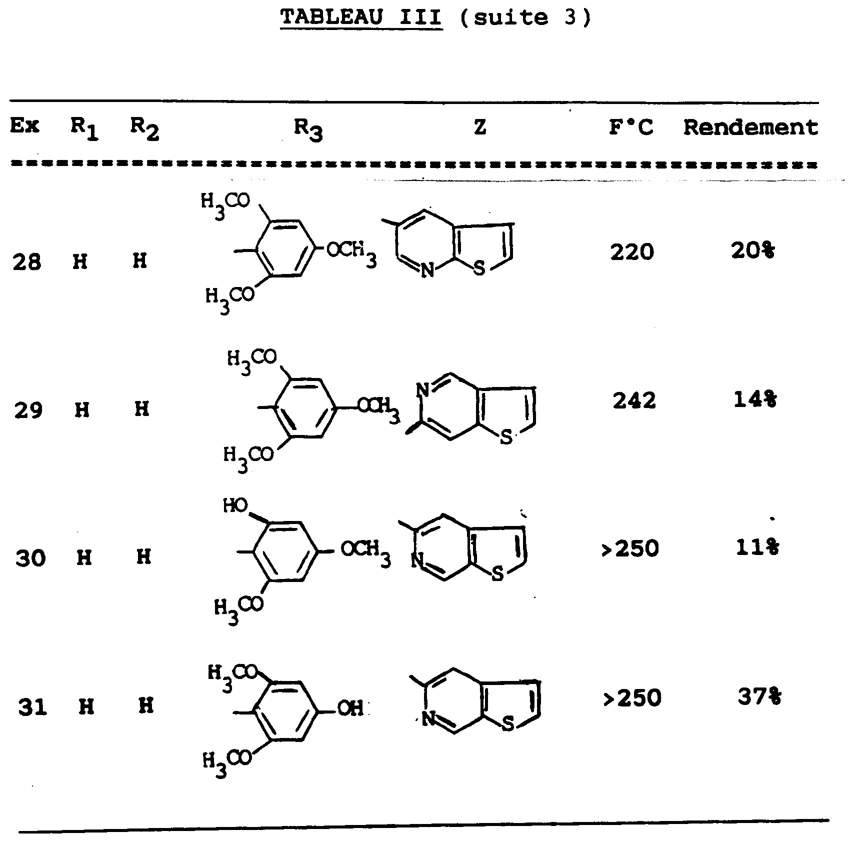

### EXEMPLE 32

### N-[(diméthoxy-2,6 éthyl-4 phényl)-4 thiazolyl-2] indole carboxamide-2 (I : R₁ = R₂ = H

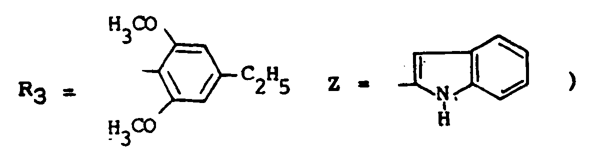

On introduit 10 ml de solution aqueuse de NaOH 2N dans une suspension de 1,4 g du composé de l'exemple 23 (composé I avec 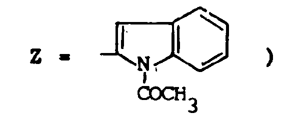 dans 150 ml d'éthanol aqueux (96% - v/v). Après 2 heures d'agitation à température ambiante on ajoute 1,9 ml d'acide chlorhydrique ensolution aqueuse concentrée. Le précipité formé est isolé et lavé à l'éthanol puis à l'éther isopropylique.
F >260°C Rendement : 85%.

### EXEMPLE 33

### N-[(triéthoxy-2,4,6 phényl)-4 thiazolyl-2] indole carboxamide-2(I R₁ = R₂ = H;

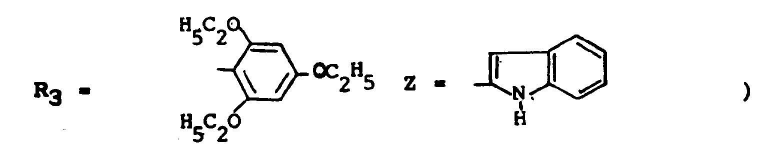

composé obtenu en appliquant la méthode décrite dans l'exemple 32 au composéde l'exemple 21.
F = 270°C Rendement : 90%.

### EXEMPLE 34

### I : R₁ = H

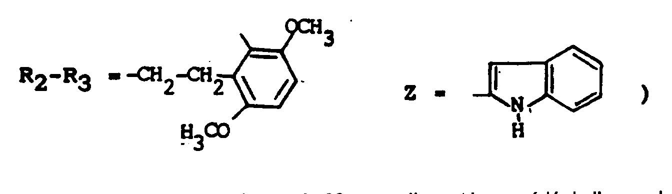

composé obtenu à partir du composé de l'exemple 22 en appliquant le procédé de l'exemple 32.
F > 260°C. Rendement : 80%.

### EXEMPLE 35

### * N[(triméthyl-2,4,6 phényl)-4 thiazolyl-2](ter-butyloxycarbonyl-1) indolinecarboxamide-2. (V : R₁ = H; R₂ = H;

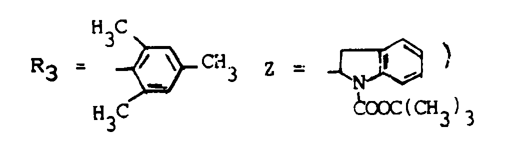

A une solution de 23,5 g du bromhydrate de l'amino-2 (triméthyl-2,4,6 phényl)-4 thiazole dans 250 ml de diméthylformamide, on additionne successivement 21 g de l'acide ter-butyloxycarbonyl-1 indolinecarboxylique-2, puis 35,2 g d'hexafluorophosphate de benzotriazolyl-1 oxytris (diméthylamino) phosphonium (BOP) puis 24,2 g de triéthylamine. On agite à température ambiante le milieu réactionnel pendant 12H. On évapore ensuite le solvant sous pression réduite et on verse sur le résidu 150 ml d'acétate d'éthyle. La solution organique est lavée avec une solution aqueuse saturée de NaCl et séchée sur du sulfate de magnésium anhydre. Le solide obtenu après évaporation du solvant est lavé avec de l'éther diisopropylique. Rendement : 95%. F = 206°C.

On prépare selon ce procédé les composés de formule V du tableau IV, pour lequel
Z représente

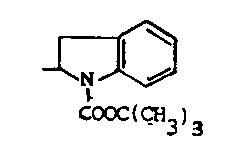

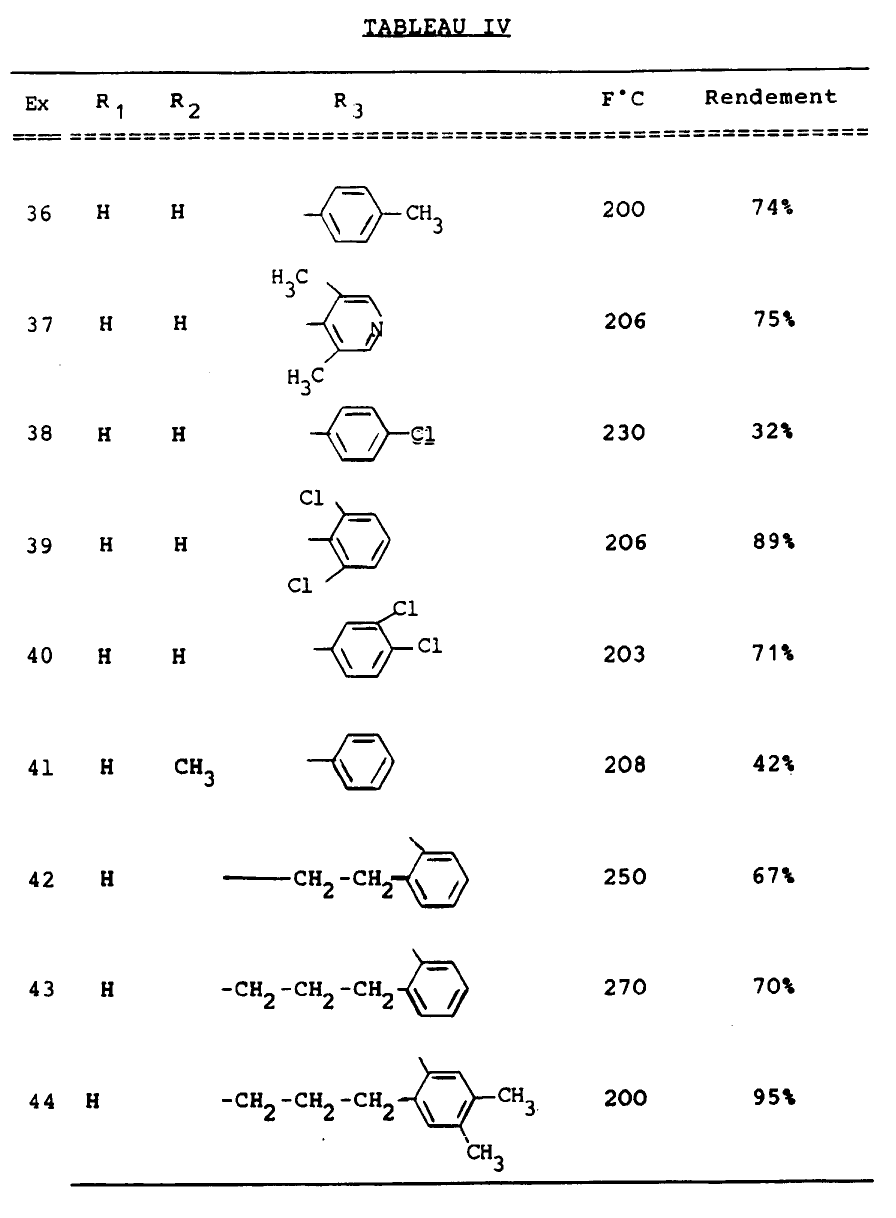

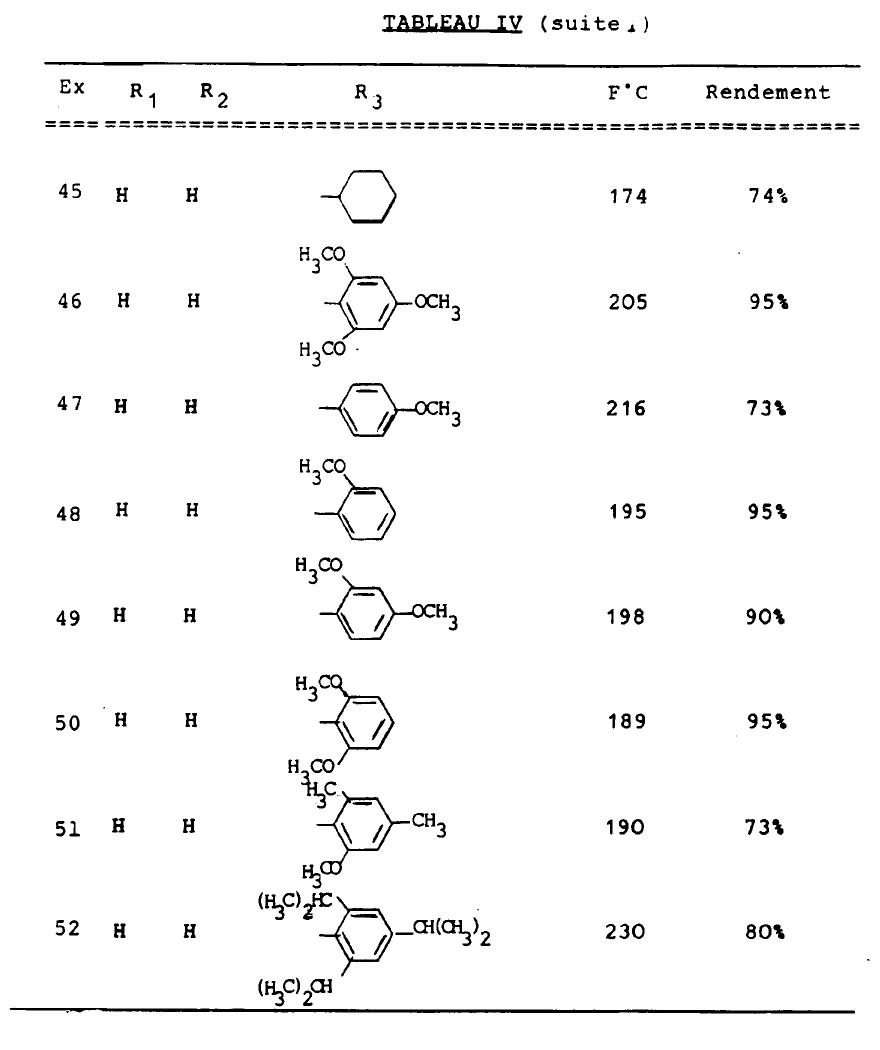

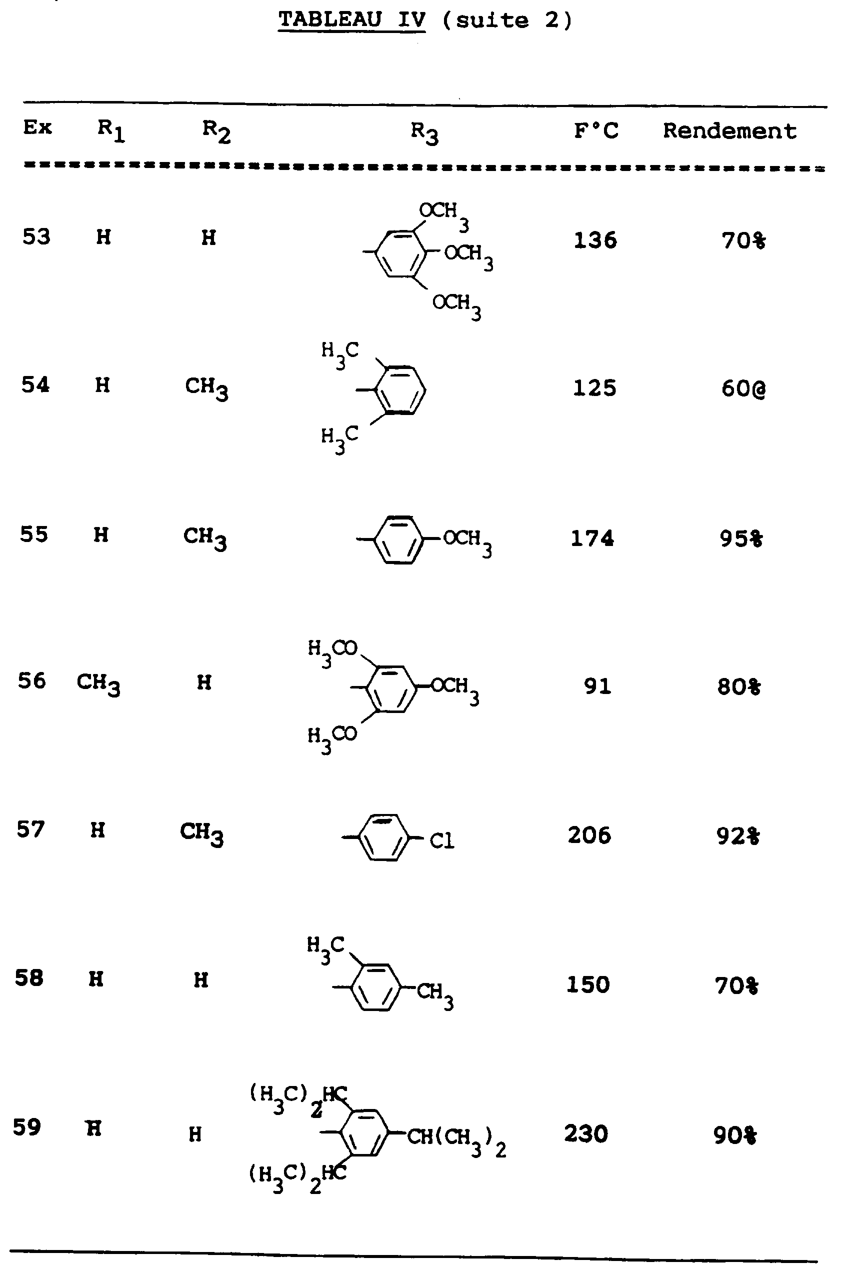

### EXEMPLE 60 N[(triméthyl-2,4,6 phényl)-4 thiazolyl-2] indolinecarboxamide-2 (I : R₁ = R₂ = H;

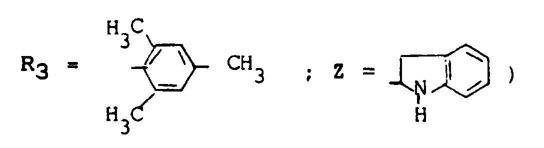

On dissout 35g de N[(triméthyl-2,4,6 phényl)-4 thiazolyl-2](tertiobutyloxycarbonyl-1) indolinecarboxamide-2 dans 200 ml de dichlorométhane sec et ajoute goutteà goutte 40 ml d'acide trifluoroacétique. On maintient le milieu réactionnel à température ambiante pendant 2 heures puis on évapore à siccité. Le résidu obtenu est repris dans 150 ml d'acétate d'éthyle. La solution organique est lavée avec une solution aqueuse 1N d'hydroxyde de sodium, puis avec de l'eau saturée de NaCl et séchée sur du sulfate de magnésium anhydre. Après élimination de l'acétate d'éthyle, on obtient le produit final, dont on prépare le sel par action de HCl gazeux dans l'isopropanol. Le chlorhydrate fond à 212°C. Rendement 88%.
Sel : RMN¹H : 80 MHz(DMSOd6) δ (ppm) : 2,1(s,6H); 2,4(s,3H); 3,2-3,8(m,2H); 5,0(m,1H); 6,0(s,1H); 6,9-7,6(m,7H); 12,1(s,1H).

### EXEMPLE 61 : N[(méthoxy-4 phényl)-4 thiazolyl-2] indolinecarboxamide-2 (I : R₁ = R₂ = H;

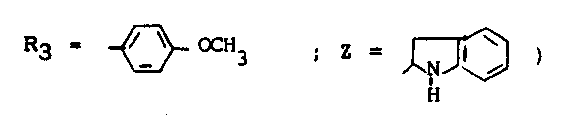

On dissout 2,9 g de N-[(méthoxy-4 phényl)-4 thiazolyl-2](terbutyloxycarbonyl-1)indolinecarboxamide -2 dans 200 ml d'acétate d'éthyle et on ajoute goutte à goutte 50 ml d'une solution 5N de HCl anhydre dans l'acétate d'éthyle. On agite à température ambiante pendant 2 heures. Le solide obtenu est séparé par filtration et lavé avec de l'éther diéthylique. On isole ainsi avec 75% de rendement le dichlorhydrate du produit final, qui fond à 214°C.
Sel : RMN¹H (250 MHz,DMSOd6) δ (ppm) : 3,2-3,7(m,2H); 3,8(s,3H); 5,0(m,1 H); 6,0(s,1H); 6,7-7,9(m,9H); 12,1(s,1H).

Les exemples du tableau V ont été préparés en appliquant l'un des procédés de déprotection décrit dans les exemples 60 et 61, aux indolines correspondantes substituées sur l'azote par COOC(CH₃)₃.

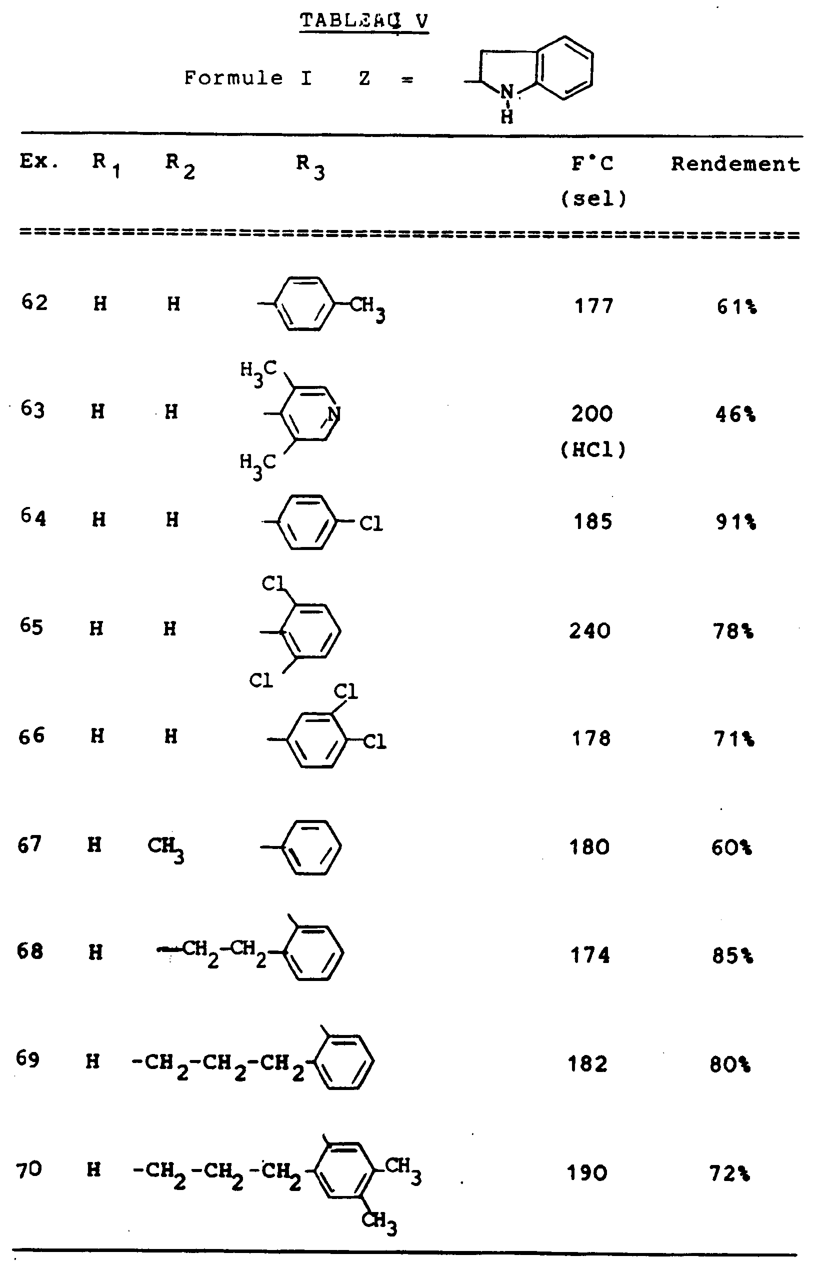

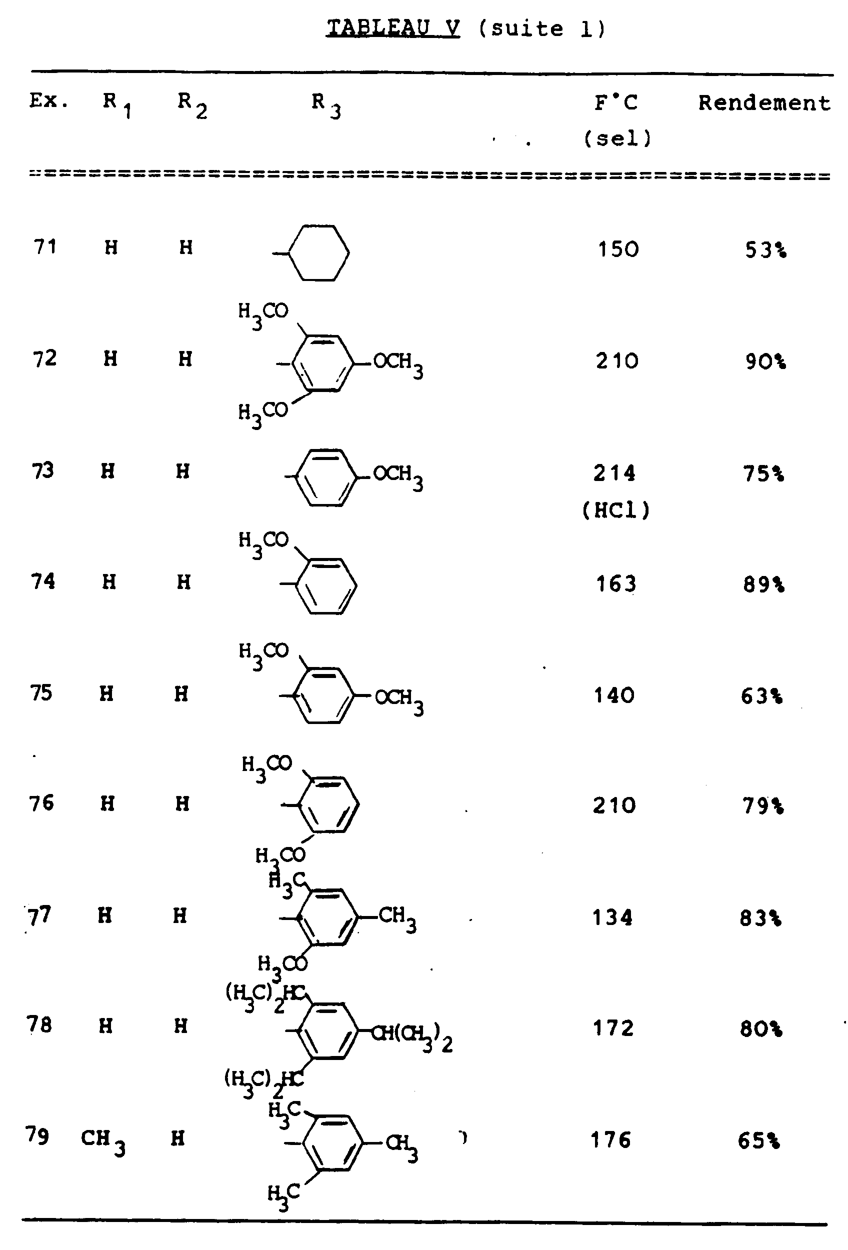

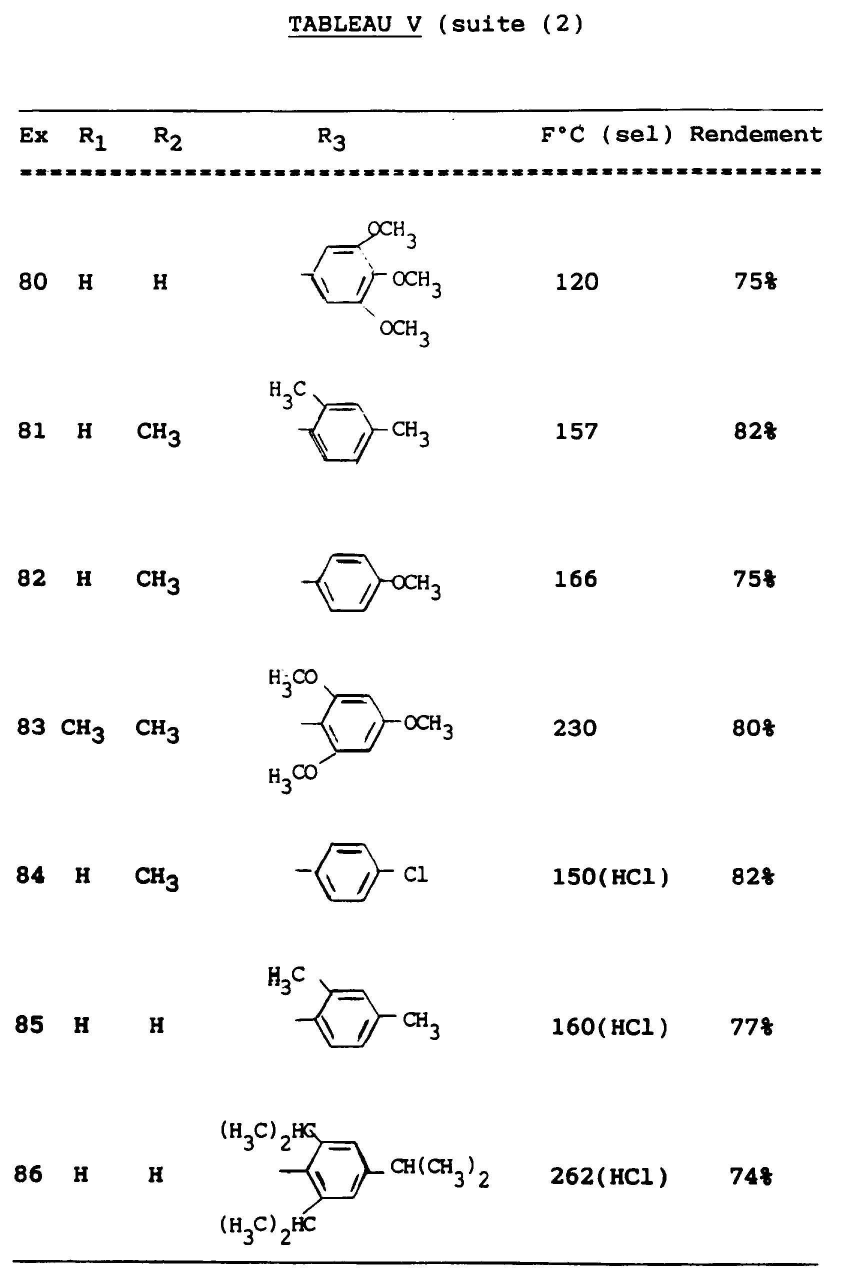

### EXEMPLE 87 : N-[(méthoxy-4 phényl)-4 thiazolyl-2] indolecarboxamide-2 (I : R₁ = R₂ = H;

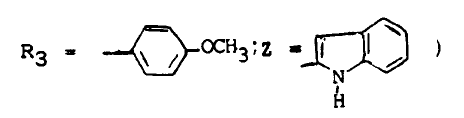

a) Action de Pd/C/Cyclohexène
   On dissout 0,5g de N-[(méthoxy-4 phényl)-4 thiazolyl-2] indolinecarboxamide-2 dans 50 ml de diphényléther, puis ajoute au milieu réactionnel 0,3 g de Pd sur charbon à 10%, puis 2 ml de cyclohexène et on porte le milieu réactionnel à 160°C pendant 5 heures. On filtre à chaud le catalyseur et le lave avec du diméthylformamide. On concentre les filtrats et le résidu obtenu est purifié par chromatographie sur colonne de silice (éluant : dichlorométhane). Rendement : 50%. PF = 252°C.
   RMN¹H : (250 MHz,DMSOd6) δ (ppm) : 3,8(s,3H); 7,0-7,9-(m,10H); 11,9(s,1H); 12,1(s,1H).
b) Oxydation avec le choranile (ou tétrachloro-2,3,5,6 benzoquinone-1,4).
   On dissout 0,2 g de N-[(méthoxy-4 thiazolyl)-2] indolinecarboxamide-2 dans 20 ml de xylène, puis ajoute 0,2 g de chloranil et on porte le milieu réactionnel au reflux pendant 3 heures. On évapore alors le solvant et on redissout le résidu dans du dichlorométhane. La solution organique est lavée successivement par une solution aqueuse 1 N d'hydroxyde de sodium, puis par de l'eau, et séchée sur du sulfate de magnésium anhydre. Le résidu obtenu après évaporation du solvant est concrétisé par trituration avec de l'éther diéthylique et lavé abondamment avec de l'éther éthylique.
   Cristaux blanc cassé. Rendement : 60%. PF = 252°C.

### EXEMPLE 88 : N-[(triméthyl-2,4,6 phényl)-4 thiazolyl-2]indolecarboxamide-2 (I : R₁ = R₂ = H;

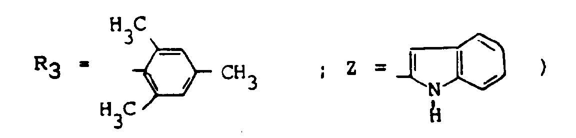

On dissout 6 g de N-[(triméthyl-2,4,6 phényl)-4 thiazolyl-2] indolinecarboxamide-2 dans 50 ml de diméthoxy-1,2 éthane et on ajoute 4,1 g de dichlor-2,3 dicyano-5,6 benzoquinone (DDQ). On agite à température ambiante pendant 3 heures. On évapore à siccité le milieu réactionnel et on reprend le résidu par de l'acétate d'éthyle. La solution organique est lavée successivement par une solution aqueuse 1 N d'hydroxyde de sodium puis par de l'eau saturée en NaCl et séchée sur du sulfate de magnésium anhydre. Le résidu obtenu après évaporation du solvant est trituré avec de l'éther diisopropylique, et le solide lavé abondamment avec ce solvant. On isole des cristaux blanchâtres. Rendement : 82%. PF = 265°C.
RMN¹H : (250 MHz,DMSOd6) δ (ppm) : 2,07(s,6H); 2,69(s,3H), 6,92-7,69(m,8H); 11,92(s,1H); 12,77(s,1H).

Les produits de formule I dans laquelle Z représente 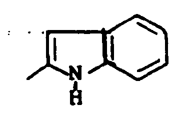 qui sont décrits dans le tableau VI ont été préparés à partir des indolines en appliquant l'un des procédés des exemples 87 et 88.

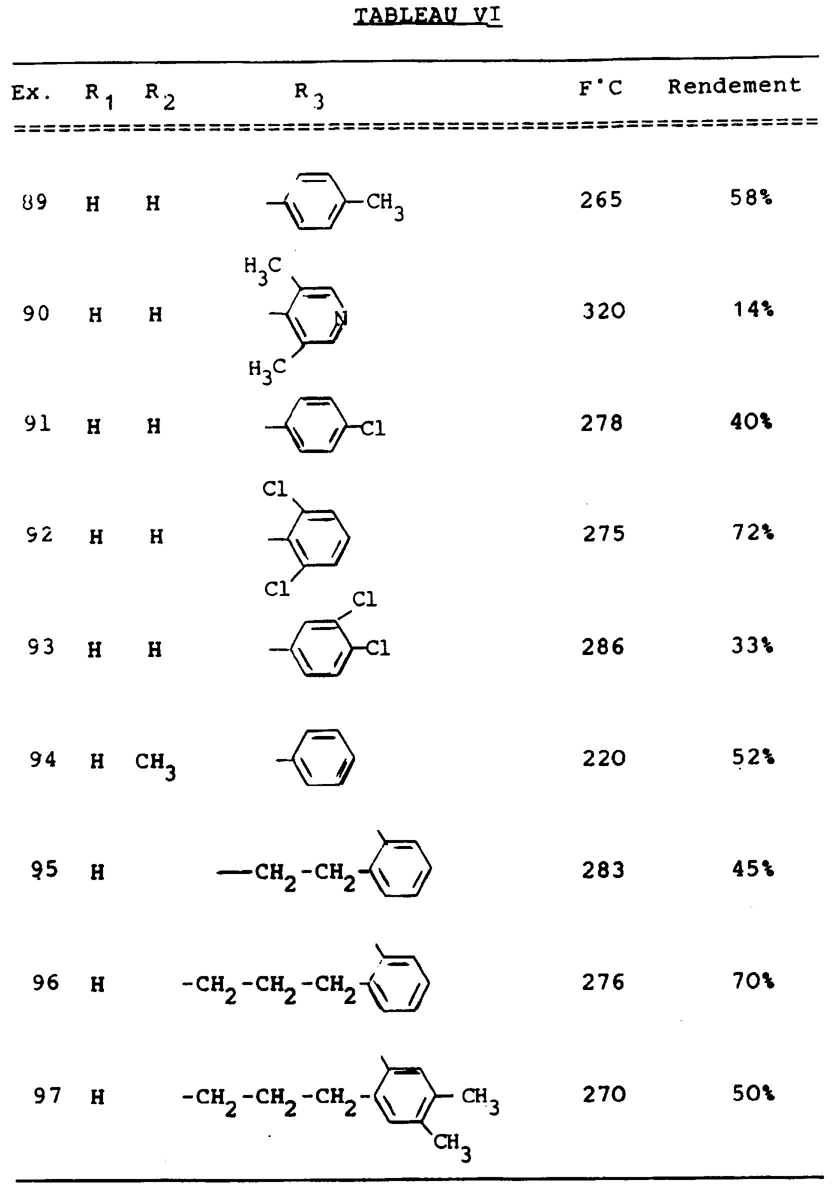

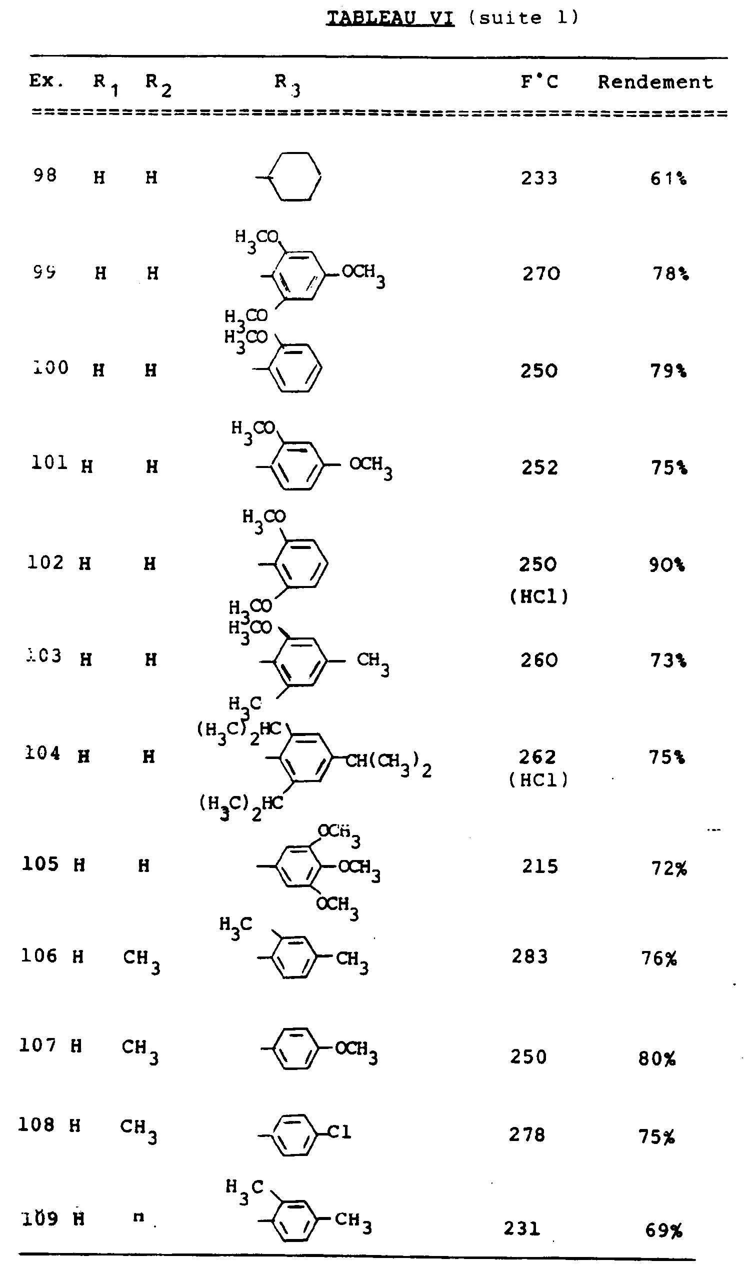

les exemples suivants concernent des composés de formule I dans laquelle

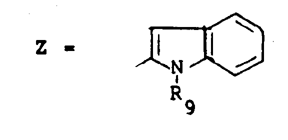

### EXEMPLE 110

### [(triméthyl-2,4,6 phényl)-4 thiazolyl-2]aminocarbonyl-2 indolyl-1 acétate de méthyle (I: R₁ = R₂ = H; R₃ = 2,4,6-(CH₃)₃C₆H₂; R₉ = CH₂COOCH₃)

A une solution de 1,7 g de bromhydrate de (triméthyl-2,4,6 phényl)-4 amino-2 thiazole dans 30 ml de diméthylformamide, on ajoute successivement 1,34 g de l'acide méthoxycarbonylméthyl-1 indolecarboxylique-2, 1,9 g de triéthylamine et 2,7 g d'hexafluorophosphate de benzotriazolyloxytris(diméthylamino)phosphonium (BOP).

On laisse le milieu réactionnel sous agitation vers 20°C pendant 1 nuit, puis on le verse dans un volume d'eau glacée avant d'extraire par de l'acétate d'éthyle. Les extraits organiques séchés sont évaporés à sec et le résidu recristallisé dans l'acétate d'éthyle. F = 206°C, rendement : 82%.

### EXEMPLE 111

### Acide [(triméthyl-2,4,6 phényl)-4 thiazolyl-2]aminocarbonyl-2 indolyl-1 acétique (I: R₁ = R₂ = H; R₃ = 2,4,6-(CH₃)₃C₆H₂; R₉ = CH₂COOH)

On dissout 1 g de l'ester obtenu à l'exemple 110, dans 15 ml de méthanol et on introduit dans le milieu 1,8 ml d'une solution aqueuse d'hydroxyde de sodium 2N; après agitation vers 20°C pendant 3 heures, on porte à 60°C pendant une heure puis on élimine le solvant, et on reprend le résidu par 15 ml d'eau. La solution aqueuse est acidifiée à pH = 4 par addition d'une solution aqueuse d'acide chlorhydrique; on isole le précipité formé par filtration. F = 244°C, rendement 81%.

### EXEMPLE 112

### N[(triméthyl-2,4,6 phényl)-4 thiazolyl-2](tétrahydropyrannyl-2)-1 indolecarboxamide-2 (I : R₁ = R₂ = H; R₃ = 2,4,6-(CH₃)₃C₆H₂;

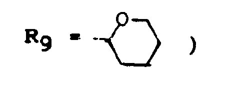

On introduit successivement dans une solution de 2,44 g de brOmhydrate de (triméthyl-2,4,6 phényl)-4 amino-2 thiazole dans 30 ml de diméthylformamide, 2,45 g de triéthylamine, 2 g d'acide (tétrahydropyrannyl-2)-1 indole carboxylique-2 et 3,6 g de BOP. Après 12 heures d'agitation vers 20°C, le milieu réactionnel est versé dans un volume d'eau glacée. Le précipité formé est isolé par filtration et recristallisé dans l'éthanol. F = 188°C, rendement 80%.

### EXEMPLE 113

### N[(triméthyl-2,4,6 phényl)-4 thiazolyl-2] indolecarboxamide-2 (I: R₁ = R₂ = R₉ = H; R₃ = 2,4,6-(CH₃)₃C₆H₂)

On maintient durant 4 heures à 60°C une solution de 1 g du composé obtenu selon l'exemple 112, dans 50 ml de méthanol et 5 ml d'une solution aqueuse d'acide chlorhydrique 6N. Le précipité formé, après retour à 20°C environ est isolé. F = 265°C, rendement 95%.

### EXEMPLE 114

### N[(triméthyl-2,4,6 phényl)-4 thiazolyl-2](diméthylamino-2 éthyl)-1 indolecarboxamide-2 (I : R₁ = R₂ = H; R₃ = 2,4,6-(CH₃)₃C₆H₂; R₉ = (CH2)₂ N(CH₃)₂)

A une solution de 3 g d'acide (N,N-diméthylamino-2 éthyl)-1 indolecarboxylique-2 dans 75 ml de diméthylformamide, on ajoute successivement 3,86 g de bromhydrate de (triméthyl-2,4,6 phényl)-4 amino-2 thiazole, 2,59 g de triéthylamine, et 5,7 g de BOP. On laisse le milieu réactionnel sous agitation à température ambiante pendant une nuit eton le verse dans un volume d'eau glacée avant d'extraire avec de l'acétate d'éthyle. Après séchage, l'extrait organique est évaporé à sec. Le solide obtenu est recristallisé dans l'acétate d'éthyle. F= 100°C, rendement: 72%.

On prépare le chlorhydrate dans l'éthanol par action de HCl. Chlorhydrate, F = 270°C.

Les produits de formule I dans laquelle 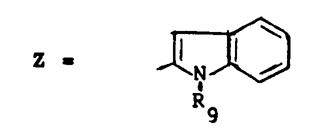 décrits dans le tableau VII ont été préparés en appliqant l'une des méthodes des exemples 110 à 114.

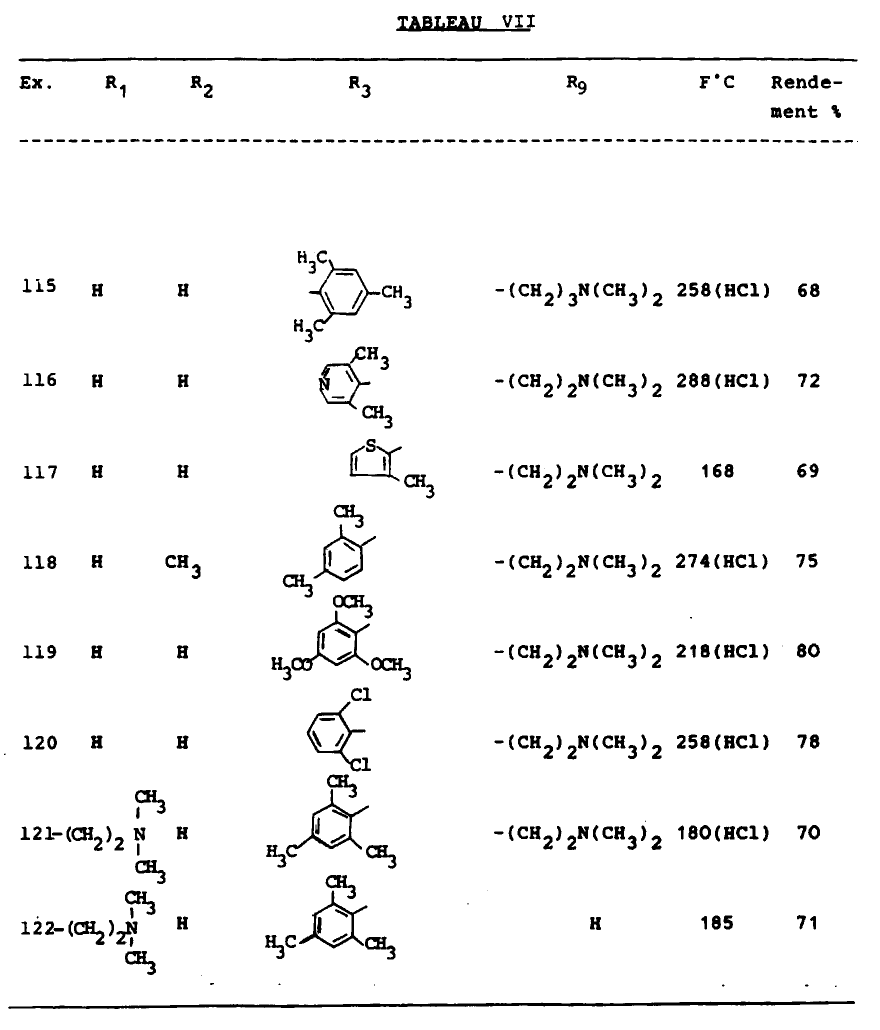

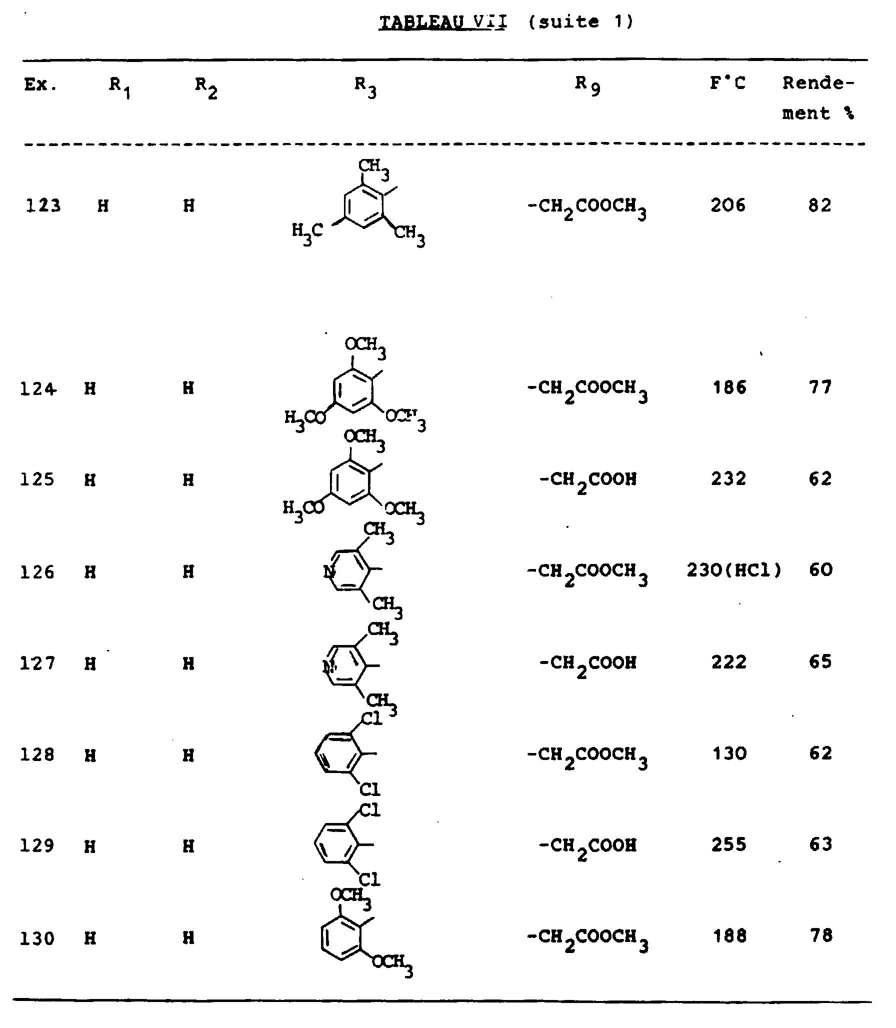

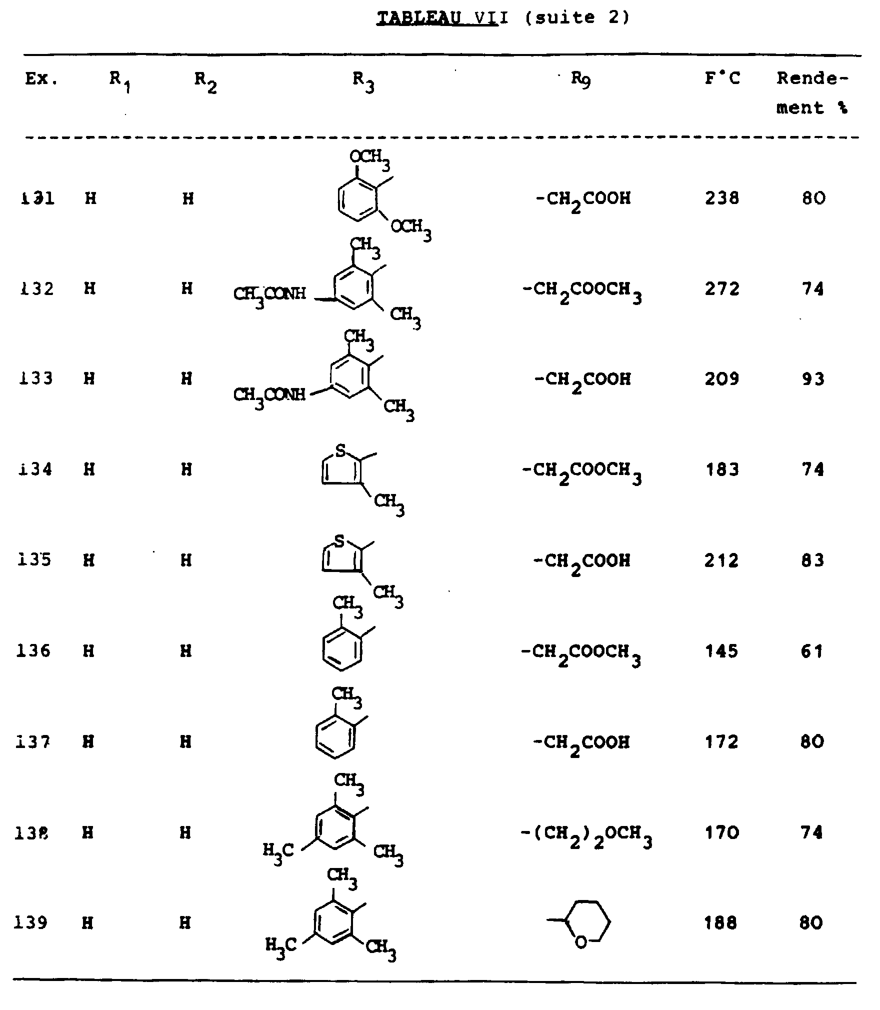

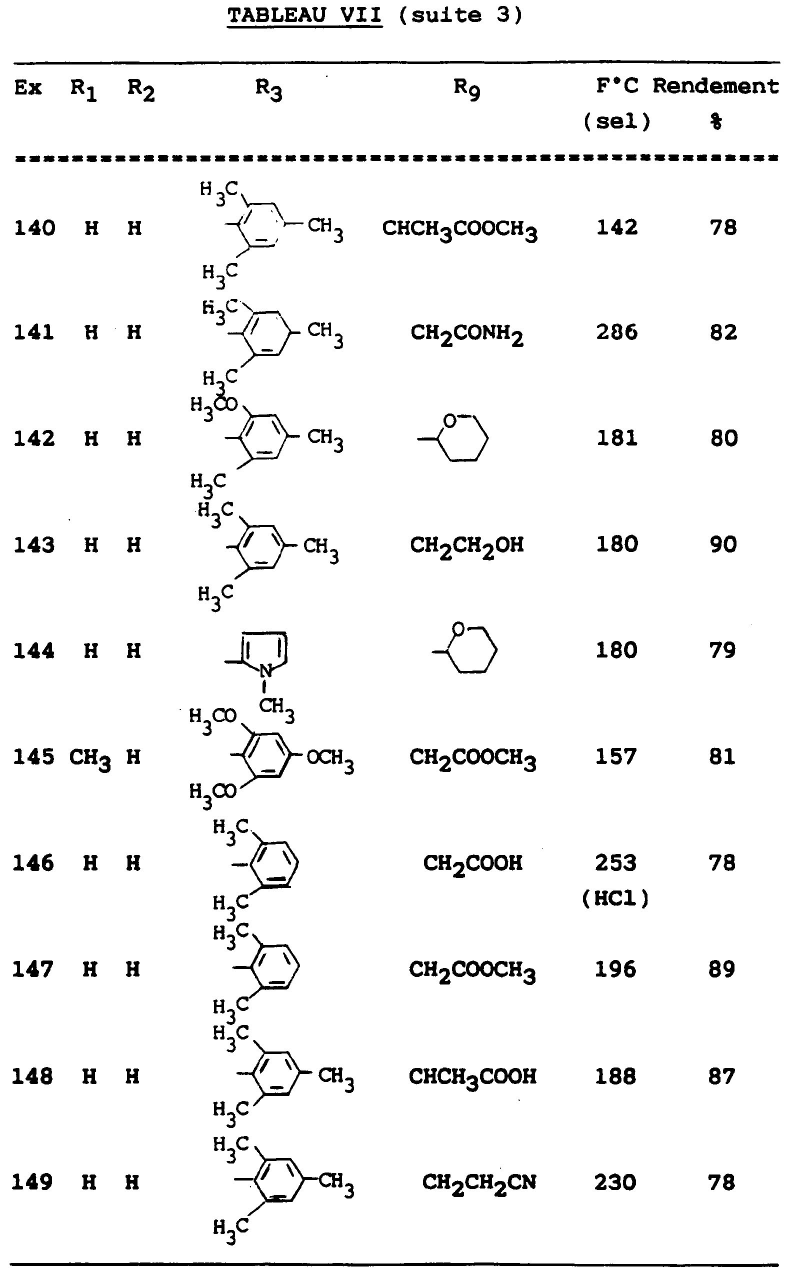

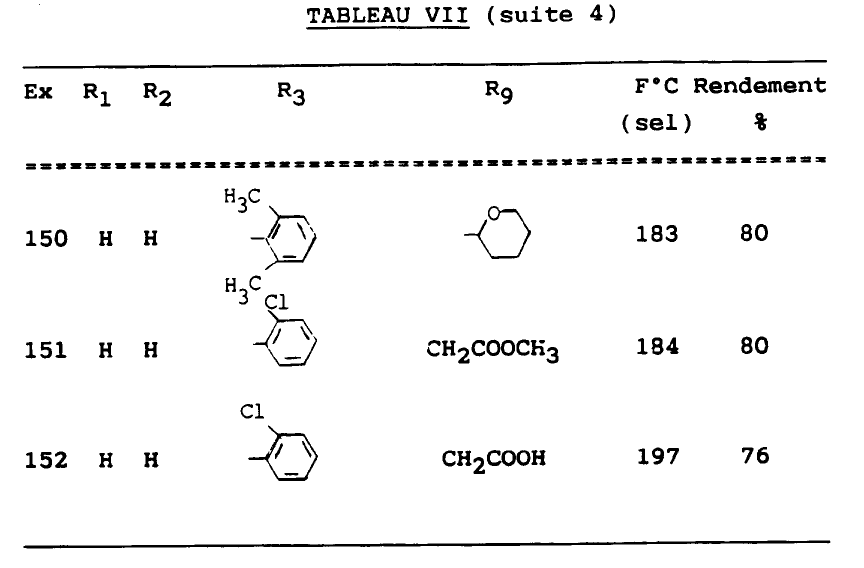

D'autres exemples de composés de formule I figurent dans le tableau VIII suivant :

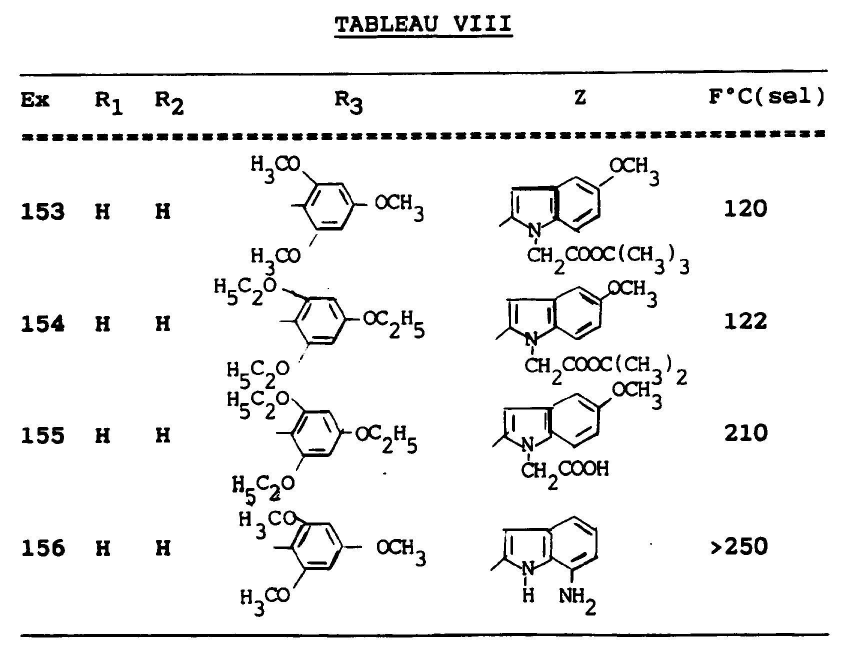

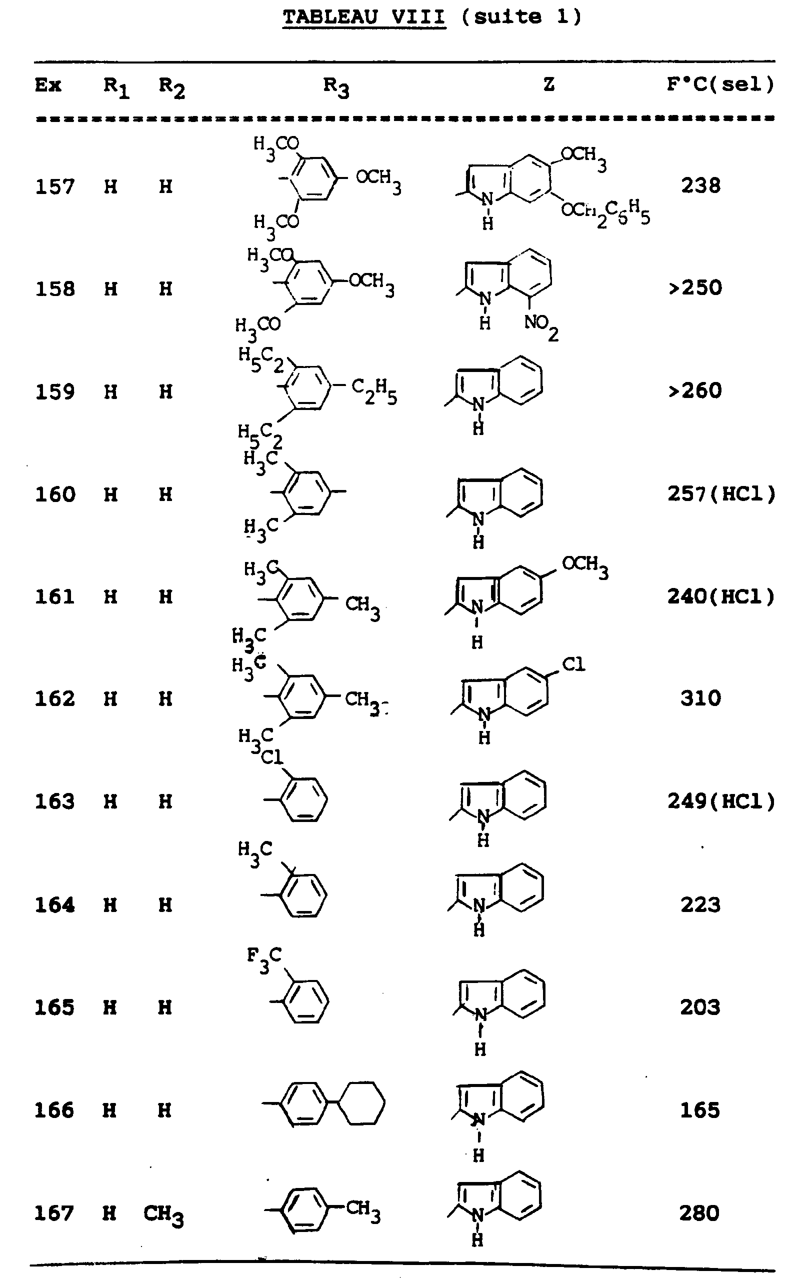

Les spectres de résonance magnétique nucléaire des composés des exemples précédents ont été enregistrés. Dans le tableau IX, on indique les déplacements chimiques observés en précisant la fréquence appliquée et le solvant.

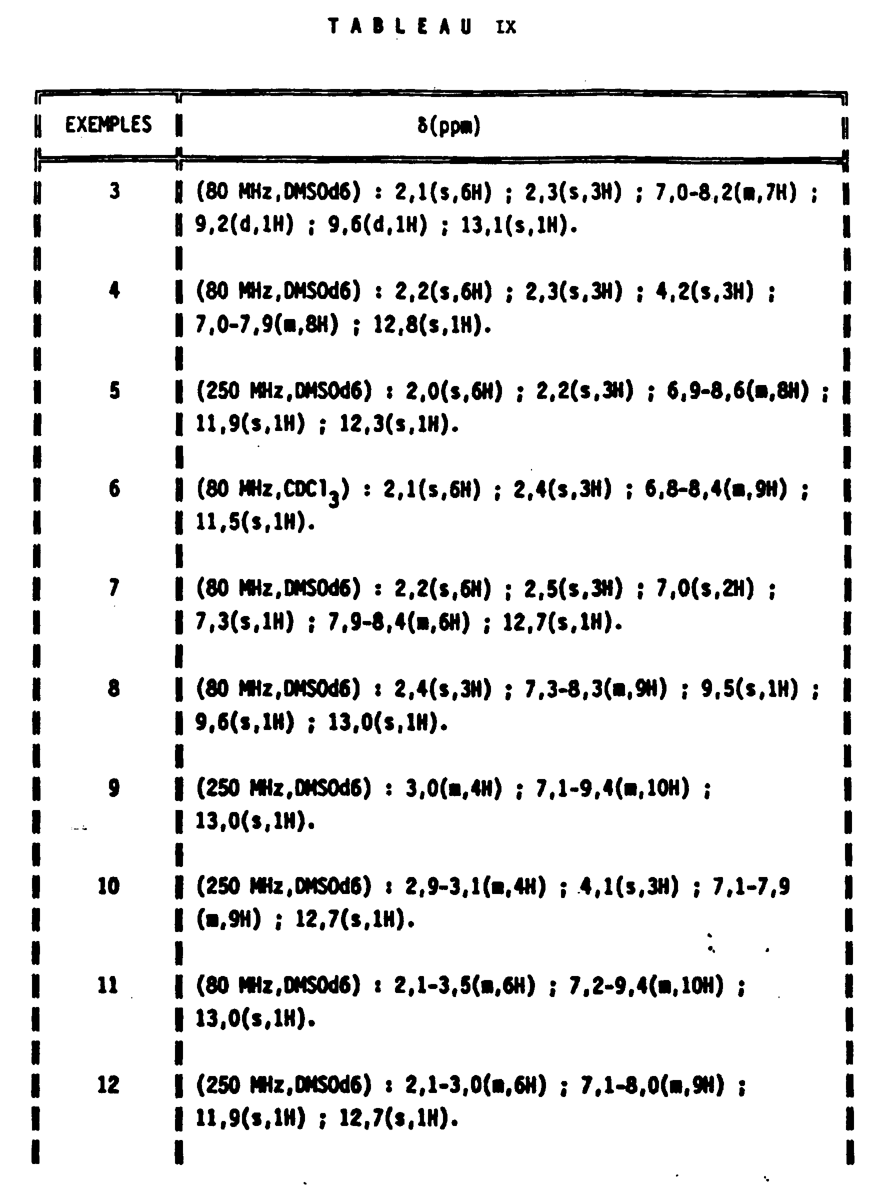

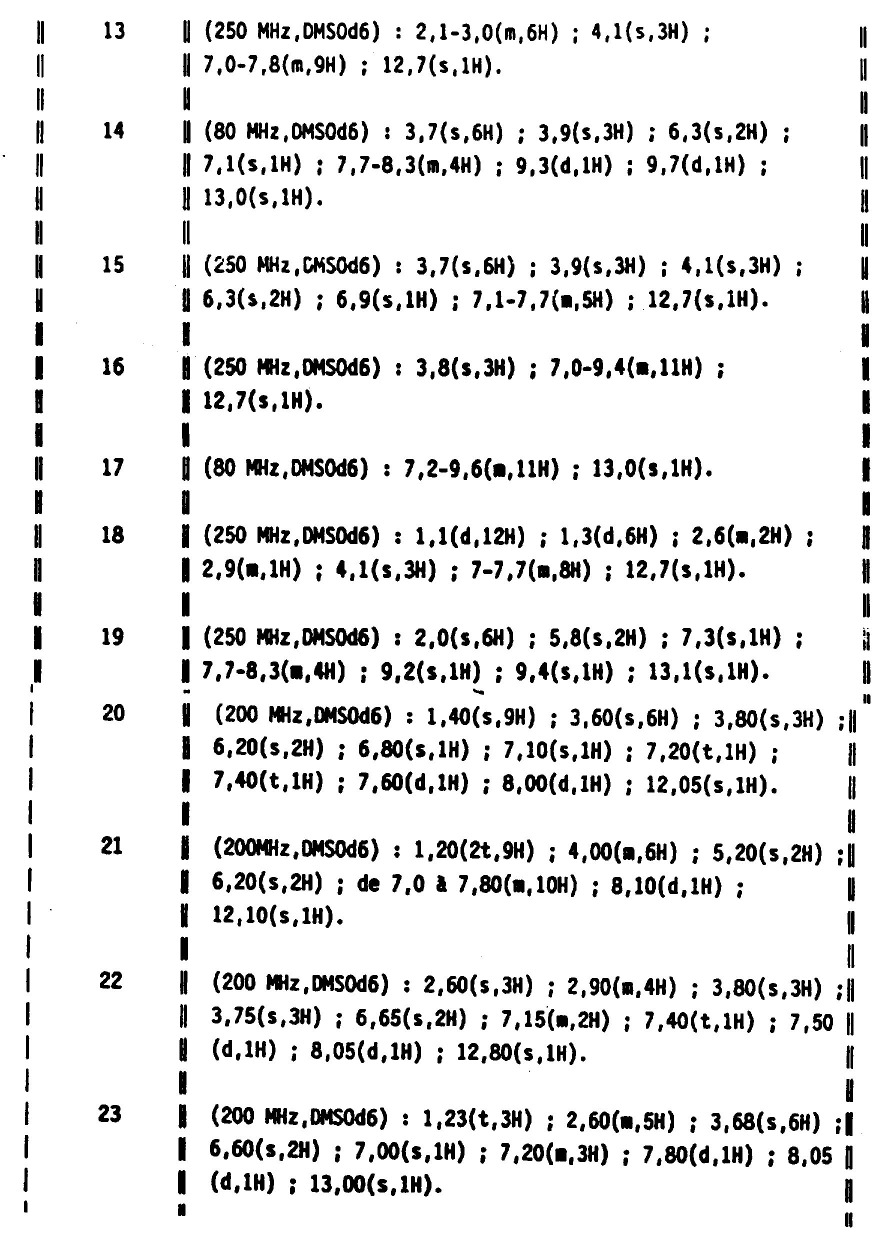

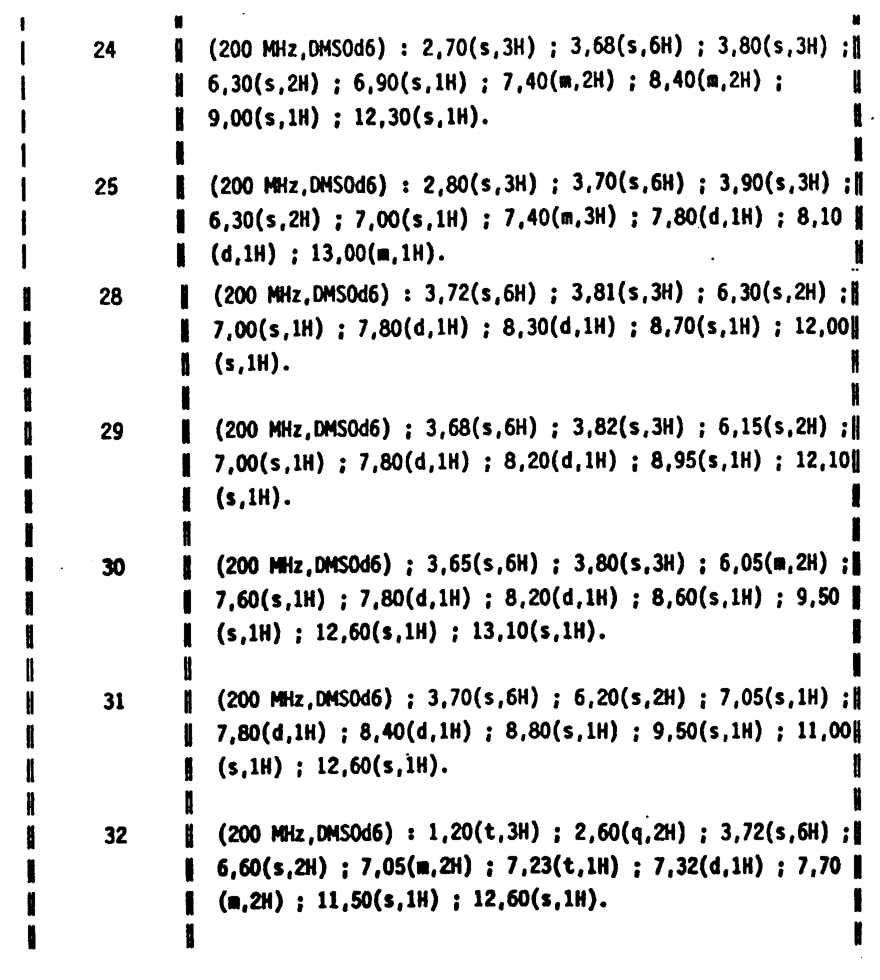

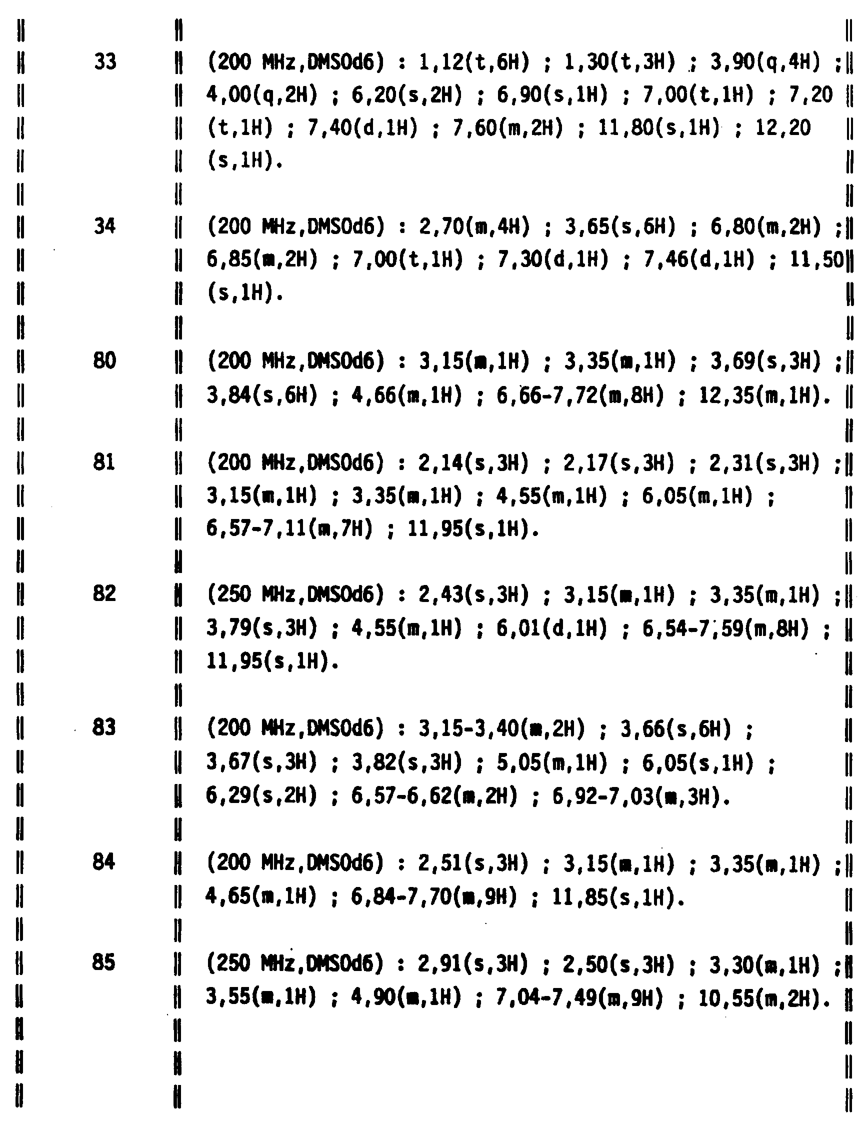

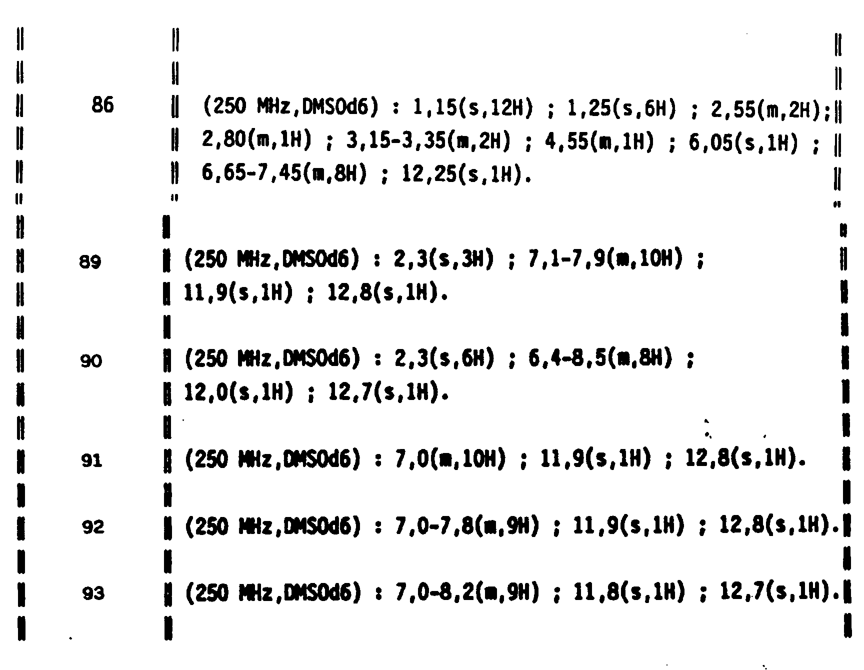

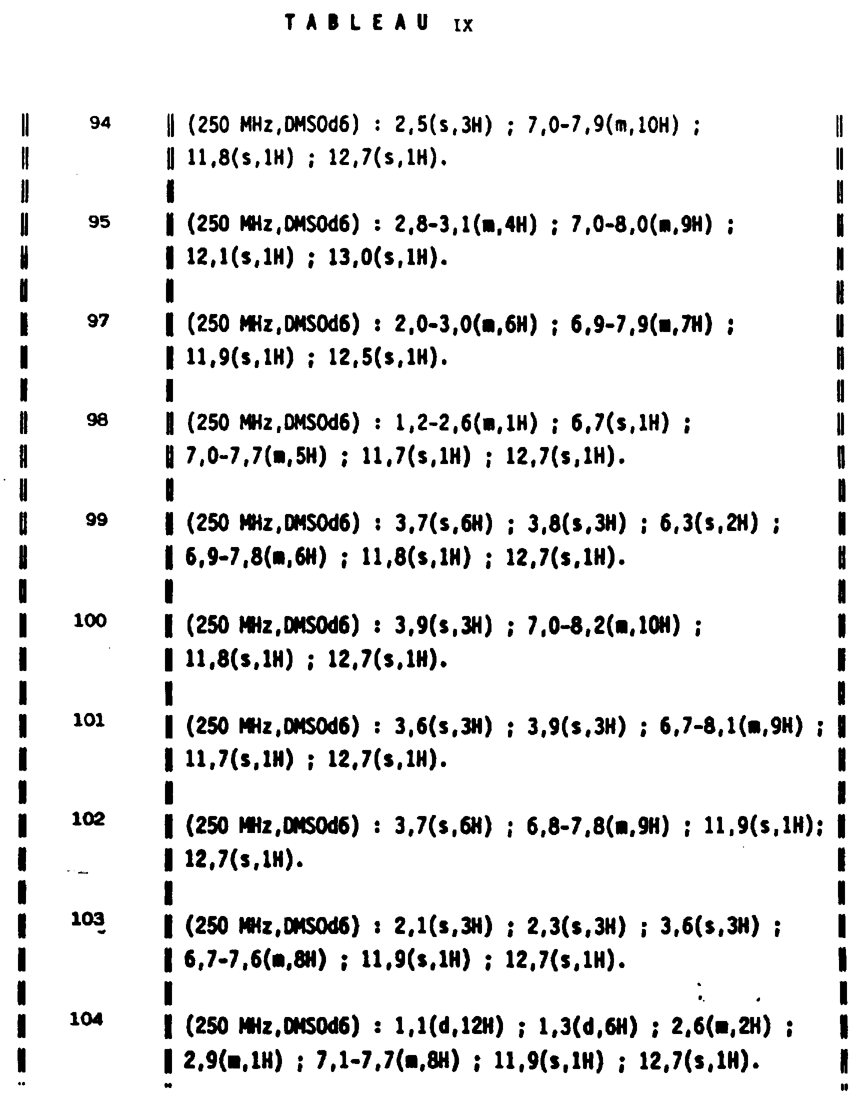

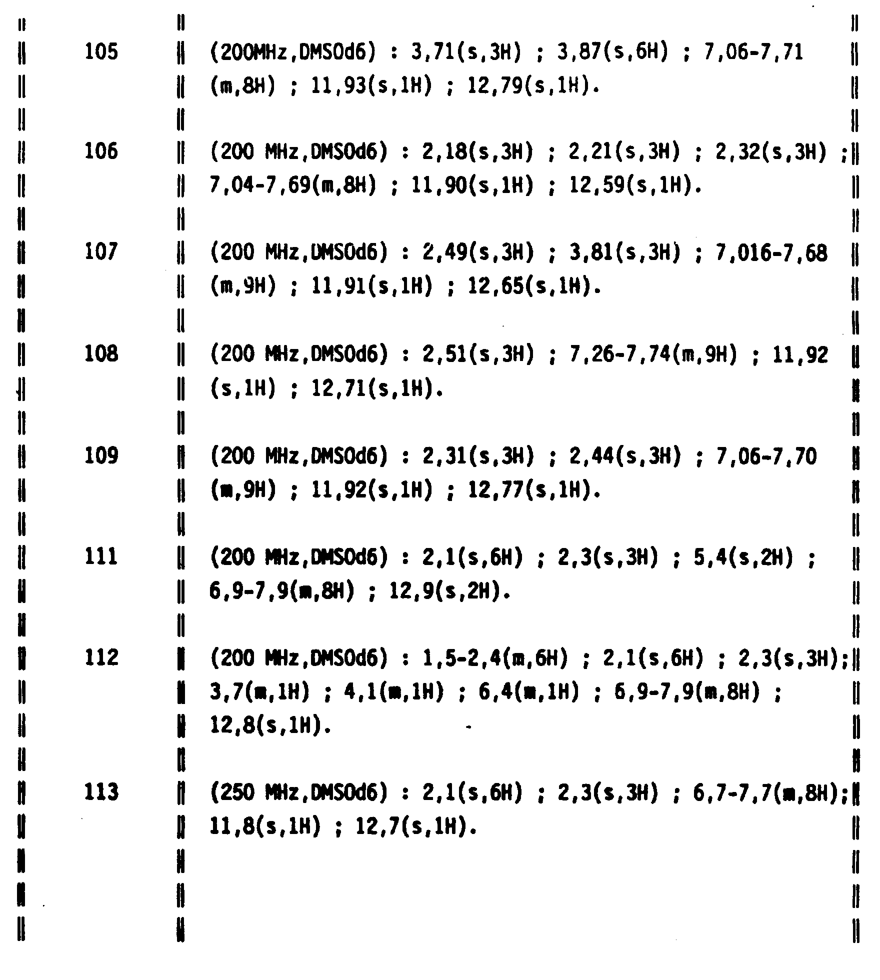

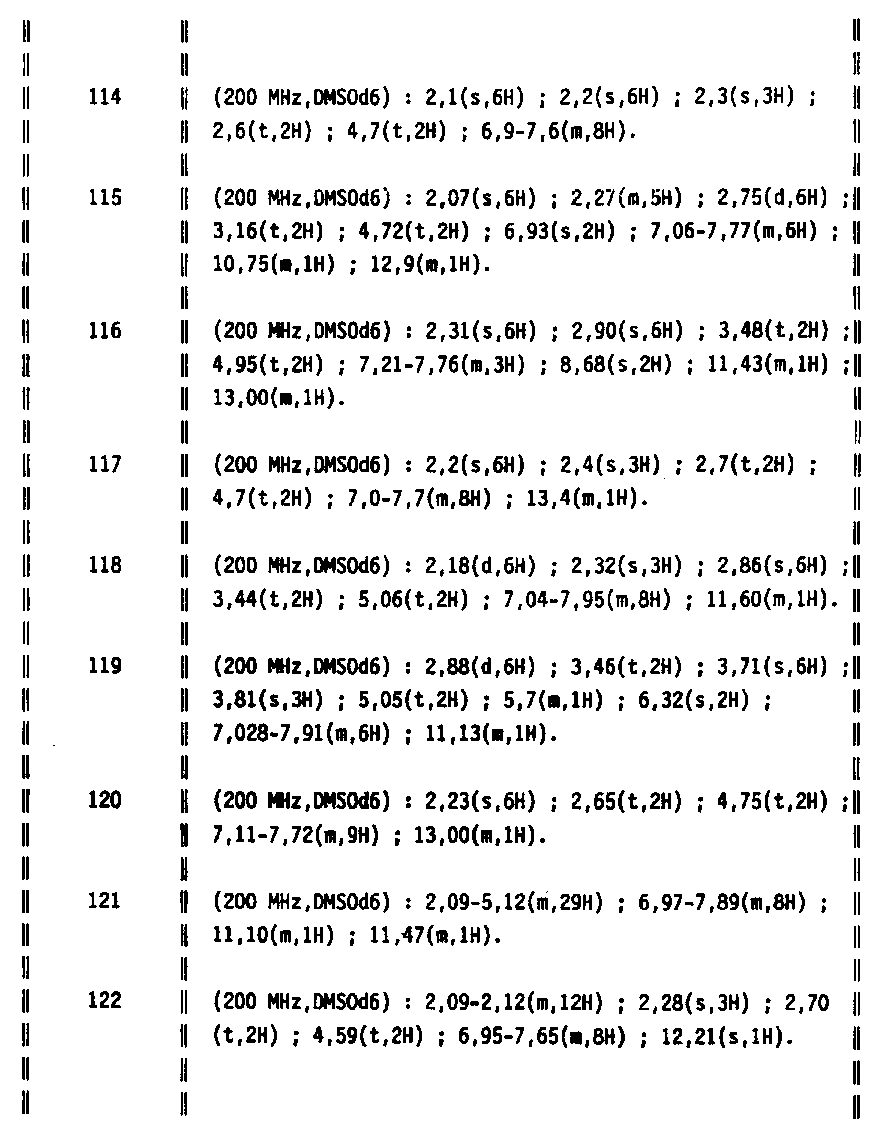

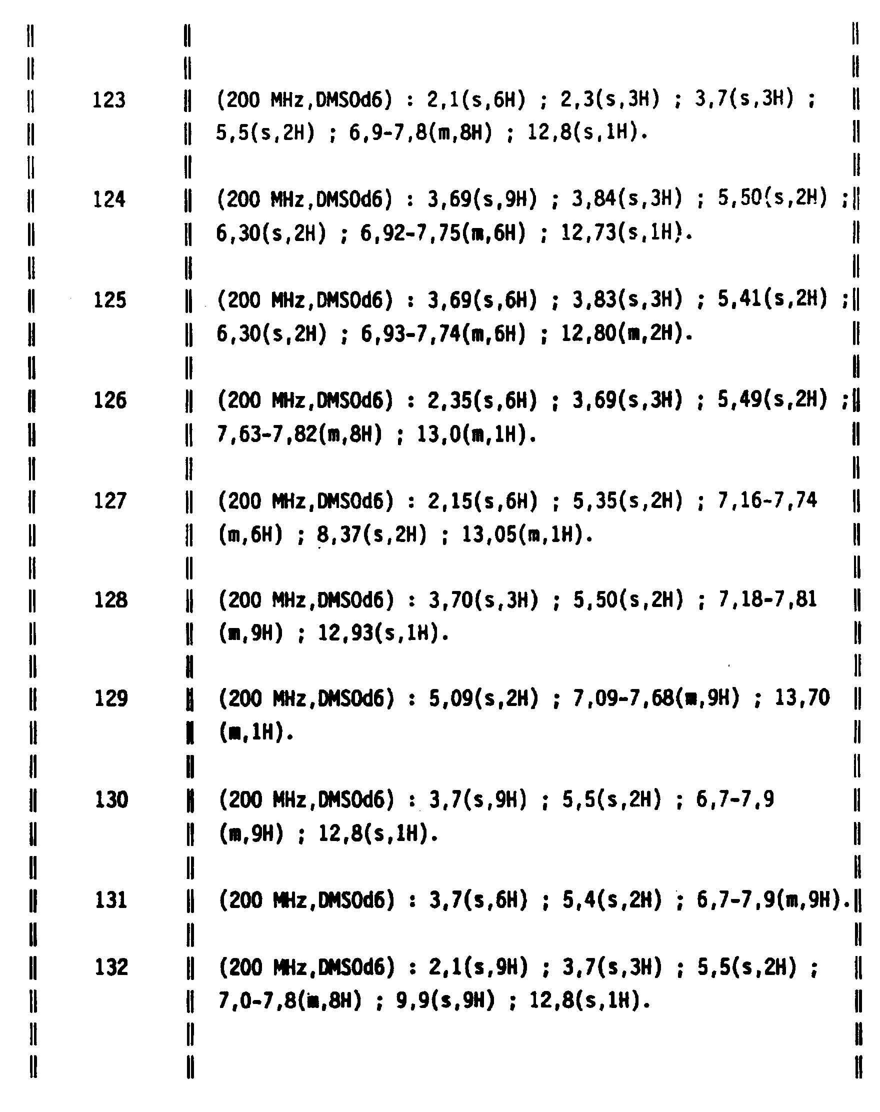

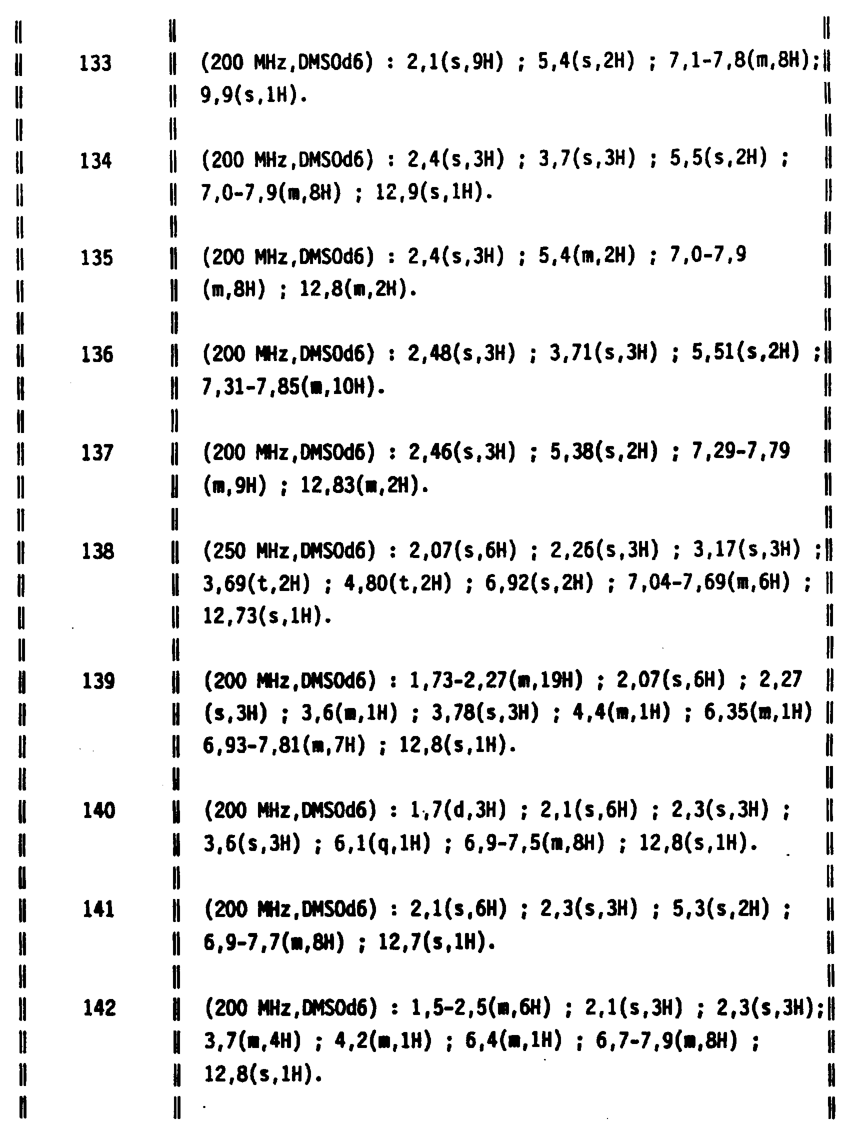

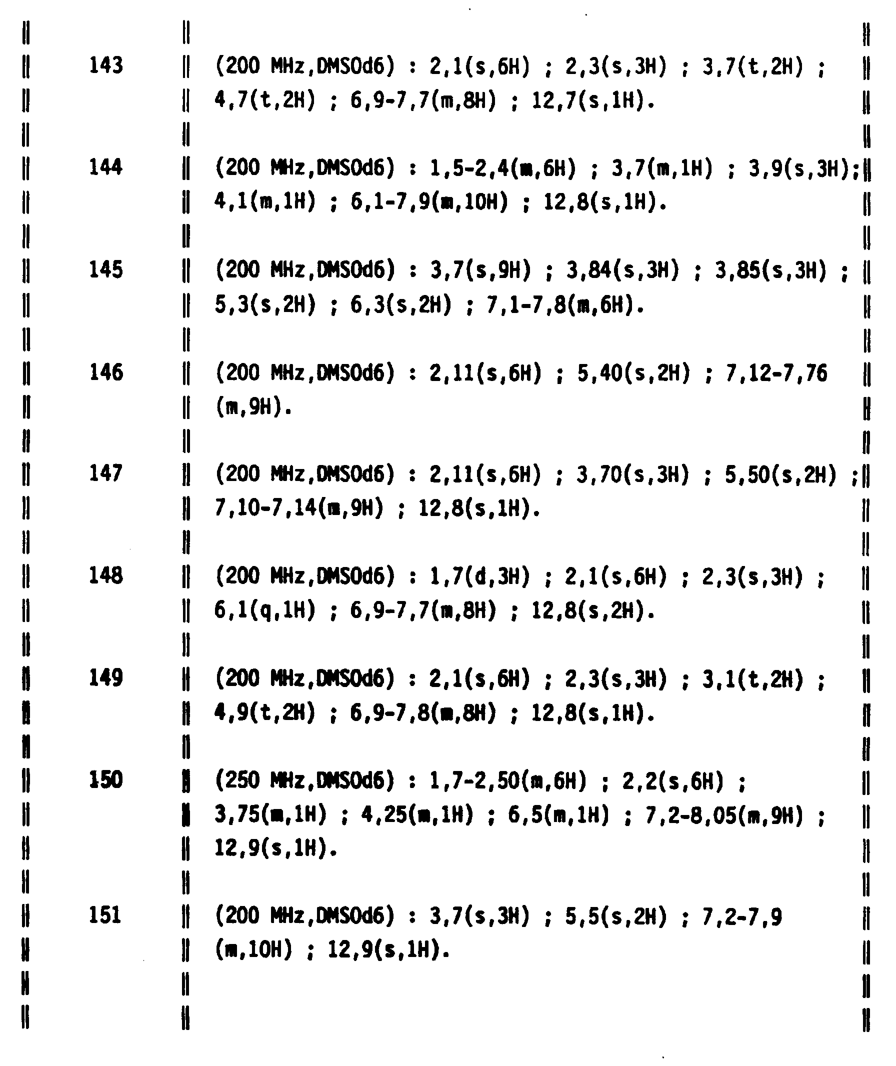

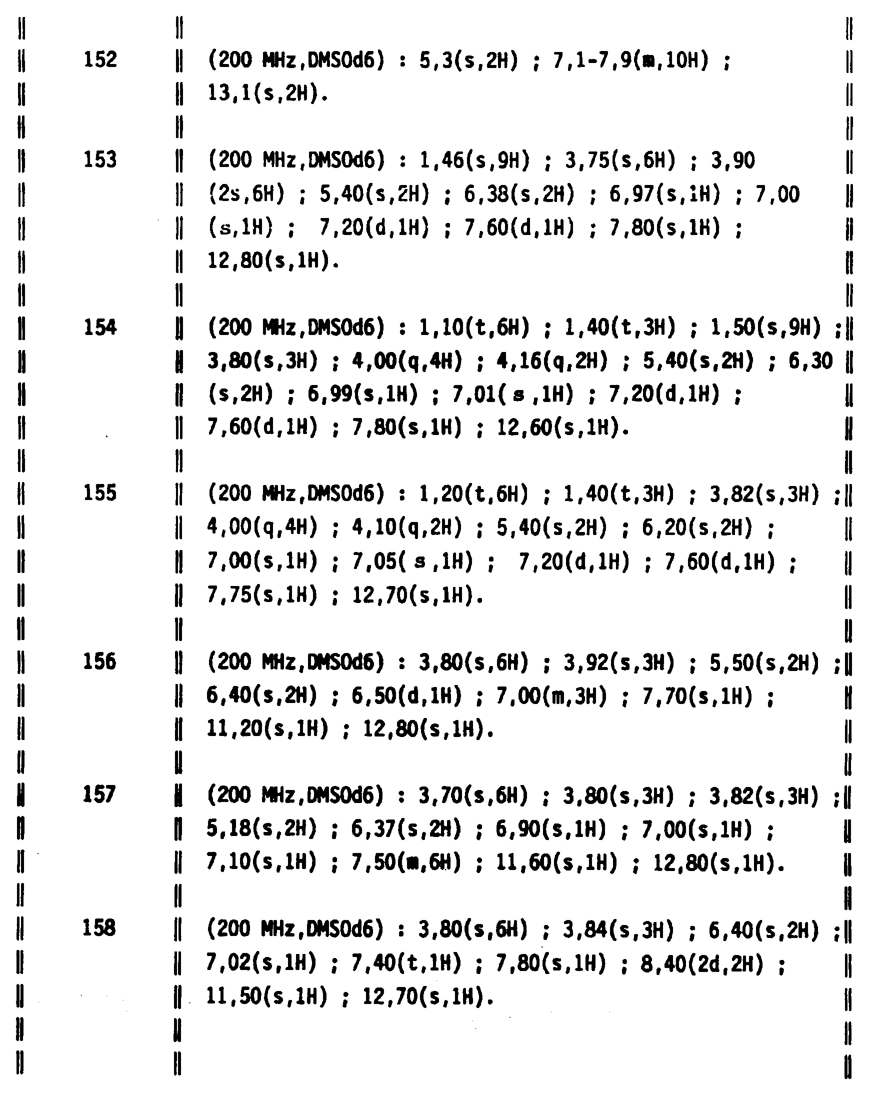

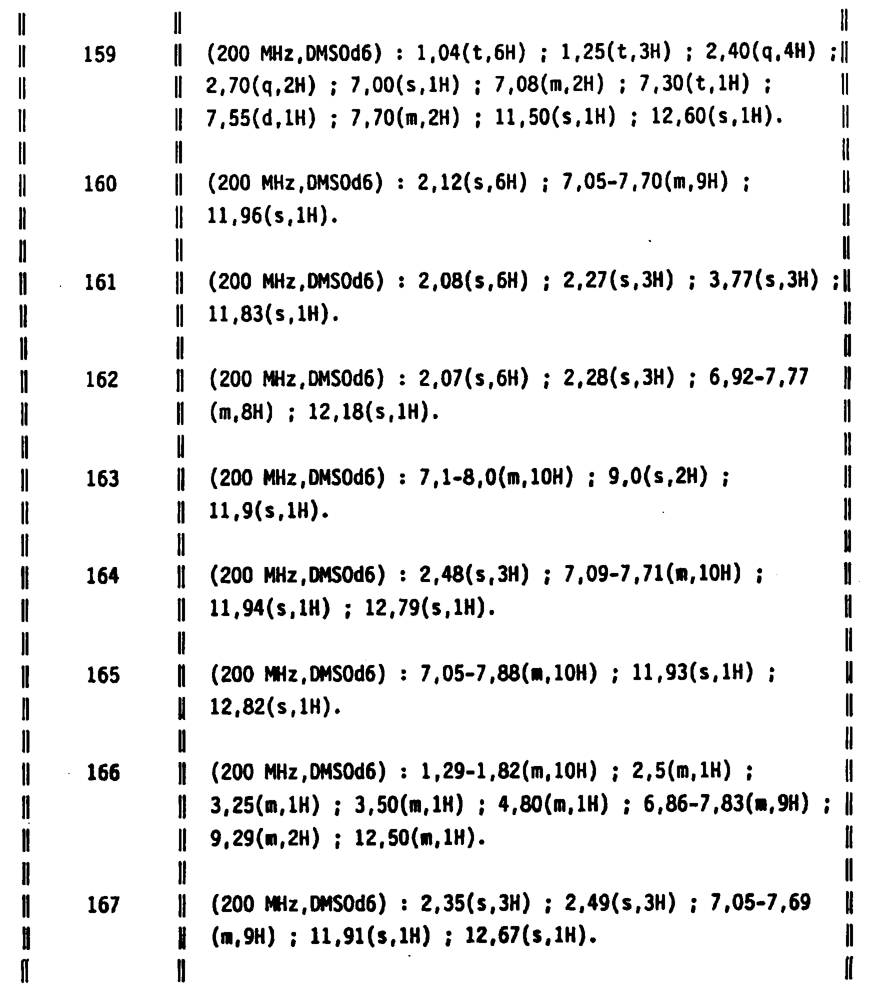

## Revendications (Revendications pour l'(les) Etat(s) contractant(s) suivant(s): AT, BE, CH, DE, DK, FR, GB, IT, LI, LU, NL, SE)

1. Acylamino-2 thiazoles de formule 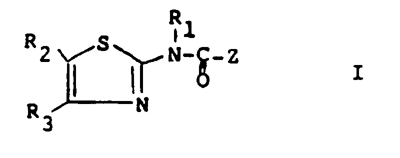 dans laquelle
R₁ représente H; alkyle en C₁ à C₄, phénylalkyle (C₁-C₃); un aminoalkyle -Z₁-NR₄R₅ dans laquelle Z₁, représente un alkylène en C₂ à C₄ et R₄ et R₅ représentent indépendamment H ou alkyle en C₁ à C₄ ou forment avec N un hétérocycle saturé et représentent morpholino, pyrrolidinyle, pipéridino, pipérazinyle ou (C₁-C₃)alkyl-4 pipérazinyle; carboxyalkyle -Z₂-COOR₆ dans laquelle Z₂ représente alkylène en C₁ à C₄ et R₆ représente H ou alkyle en C₁ à C₆; cyanoalkyle en C₂ à C₅; carbamoylalkyle -Z₃-CONR₇R₈ dans laquelle Z₃ représente un alkylène en C₁ à C₄ et R₇ et R₈ représentent indépendamment H ou un alkyle en C₁ à C₄ ou, avec N, un hétérocycle comme NR₄R₅; hydroxyalkyle en C₂ à C₆ ou alkoxyalkyle en C₂ à C₁₀;
R₂ représente H ou alkyle en C₁ à C₄;
R₃ représente un cycloalkyle en C₅ à C₈, éventuellement substitué par un ou des alkyles en C₁ à C₄; un groupe aromatique, tel qu'un phényle portant éventuellement un ou plusieurs substituants choisis parmi halogène, (C₁-C₆)alkyle, (C₁-C₃)alkoxy et (C₁-C₃)thioalkoxy, nitro, trifluorométhyle ou tel qu'un hétérocycle comportant au moins un hétéroatome choisi parmi O, S et N, et R₃ représente alors furyle, thiényle, pyrrolyle, pyrazolyle, imidazolyle, pyridyle, pyrazinyle, oxazolyle et thiazolyle, éventuellement substitués par (C₁ à C₃) alkyle ou halogène
ou R₂ et R₃ considérés ensemble représentent le groupe 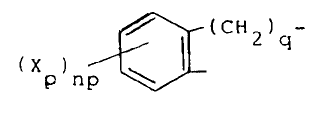 fixé par le carbone du phényle en position 4 du thiazolyle et dans lequel q vaut 1 à 4, et Xₚ représente les éventuels substituants choisis parmi halogène, (C₁-C₃)alkyle, (C₁-C₃)alkoxy, nitro et trifluorométhyle et np représente 0 à 3, et
Z représente un hétérocycle comportant un ou plusieurs hétéroatomes choisis parmi S et N, condensé avec un noyau aromatique qui peut comporter un hétéroatome et qui peut être substitué par un ou des groupes choisis parmi halogène, (C₁-C₃)alkyle, (C₁-C₃) alkoxy, benzyloxy, nitro, amino et trifluorométhyle, ainsi que les sels d'addition des composés de formule I avec des acides et des bases minérales ou organiques,

2. Composés selon la revendication 1 de formule I dans laquelle Z représente benzothiényle, benzimidazolyle, benzothiazolyle, quinolyle, isoquinolyle, quinoxalinyle, quinazolinyle, cinnolinyle et thieno[2,3-c] ou [3,2-c] pyridyle, isoindolyle, isoindolinyle, indolyle ou indolinyle, éventuellement substitués, et les groupes indolyle et indolinyle étant éventuellement substitués sur l'azote par (C₁-C₄)alkyle, (C₂-C₆)hydroxyalkyle ; (C₂-C₁₀)alkoxyalkyle, aminoalkyle-Z₄-NR₁₀R₁₁ dans laquelle Z₄ représente (C₂-C₄)-alkylène et R₁₀ et R₁₁ représentent indépendamment H, (C₁-C₄) alkyle ou R₁₀ et R₁₁ représentent avec N un hétéro-saturé tel que morpholino, pyrrolidinyle, pipéridino, piperazinyle ou (C₁-C₃)alkyl-4 pipérazinyle; carboxyalkyle-Z₅-COOR₁₂ dans laquelle Z₅ représente (C₁-C₄) alkylène R₁₂ est H, benzyle ou (C₁-C₆) alkyle: carbamoylalkyle -Z₆-CONR₁₃R₁₄ dans laquelle Z₆ représente (C₁-C₄)alkylène et R₁₃ et R₁₄ représentent indépendamment H, (C₁-C₆) alkyle, ou forment avec N un hétérocycle saturé comme NR₁₀R₁₁; acyle-COR₁₅ avec R₁₅ représente (C₁-C₄)alkyle ou phényle; alkoxycarbonyle -COOR₁₆ avec R₁₆ étant tert-butyle ou benzyle; et leurs sels.

3. Composés selon la revendication 2 de formule I dans laquelle R₂ représente H, R₁ représente H, (C₁-C₄)alkyle ou -Z₁-NR₄R₅ avec Z_{1,} R_{4,} R₅ ayant les mêmes significations que dans la revendication 1, et Z représente un groupe indolyle substitué ou non sur l'azote comme à la revendication 2 et R₃ représente un phényle au moins orthosubstitué.

4. N-[(triméthyl-2,4,6 phényl)-4 thiazolyl-2] indole carboxamide-2 et les composés substitués sur l'azote de l'indole par CH₃, CH₂COOH, CH₂COOCH₃, (CH₂)₂ N(CH₃)₂ et leurs sels pharmaceutiquement acceptables.

5. N-[(triméthoxy-2,4,6 phényl)-4 thiazolyl-2] indole carboxamide-2 et les composés substitués sur l'azote de l'indole par CH₃, CH₂COOH, CH₂COOCH₃, (CH₂)₂ N(CH₃)₂ et leurs sels pharmaceutiquement acceptables.

6. N-[(diméthyl-2,6 phényl)-4 thiazolyl-2] indole carboxamide-2 et les composés substitués sur l'azote de l'indole par CH₂COOH et CH₂COOCH₃, et leurs sels pharmaceutiquement acceptables.

7. N-[(diméthoxy-2,6 phényl)-4 thiazolyl-2] indole carboxamide-2 et les composés substitués sur l'azote de l'indole par CH₂COOH et CH₂COOCH₃ et leurs sels pharmaceutiquement acceptables.

8. N-[(dichloro-2,6 phényl)-4 thiazolyl-2]-indole carboxamide-2 et les composés substitués à l'azote par CH₂COOH et (CH₂)₂ N(CH₃)₂ et leurs sels pharmaceutiquement acceptables.

9. N-[(méthyl-2-phényl)-4 thiazolyl-2] indole carboxamide-2, N-[(méthoxy-2 phényl)-4 thiazolyl-2] indole carboxamide-2, N-[(chloro-2 phényl)-4 thiazolyl-2] indole carboxamide-2 et les composés substitués à l'azote par CH₂COOH ainsi que leurs sels pharmaceutiquement acceptables.

10. N-[(méthyl-4 phényl)-4 thiazolyl-2] indole carboxamide-2 et N-[(méthoxy-4 phényl)-4 thiazolyl-2] indole carboxamide-2 et leurs sels pharmaceutiquement acceptables.

11. Procédé de préparation des composés de formule I selon l'une des revendications 1 à 10, caractérisé en ce qu'on fait réagir sur l'aminothiazole de formule 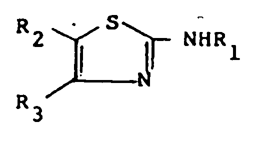 dans laquelle R₁, R₂ et R₃ ont la même signification que dans la formule I,
une forme activée d'un acide de formule Z'COOH dans laquelle Z' représente Z comme dans la formule I ou un dérivé de Z dans lequel les fonctions sensibles ont éventuellement été protégées, puis le cas échéant on effectue une réaction de déprotection.

12. Procédé de préparation des composés selon l'une des revendications 1 à 10, de formule I dans laquelle Z représente le noyau indolyle, non substitué à l'azote et éventuellement substitué sur le phényle, caractérisé en ce que l'on prépare le composé correspondant de formule I dans laquelle Z représente le noyau indolinyle par acylation de l'aminothiazole avec une forme activée de l'acide de formule 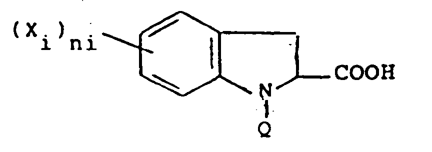 dans laquelle Q représente un groupe protecteur et Xᵢ représentent chacun, halogène, (C₁-C₃)alkyle, (C₁-C₃)alkoxy, NO₂ ou CF_{3,} et ni vaut 0 à 3 puis que l'on déshydrogène le composé obtenu pour obtenir le dérivé indolyle correspondant, après avoir déprotégé l'azote.

13. Procédé selon la revendication 12, caractérisé en ce que Q représente COO(t-C₄H₉) et la déshydrogénation est effectuée par action de la tétrachloro-2,3,5,6 benzoquinone-1,4 de la dichloro-2,3 dicyano-5,6 benzoquinone-1,4 ou du cyclohexène en présence de Pd à une température supérieure à 100°C, dans un solvant inerte.

14. Composition pharmaceutique, caractérisée en ce qu'elle comprend au moins un composé selon l'une des revendications 1 à 10 associé à au moins un excipient.

## Revendications (Revendications pour l'(les) Etat(s) contractant(s) suivant(s): ES, GR)

1. Procédé de préparation d'acylamino-2 thiazoles de formule 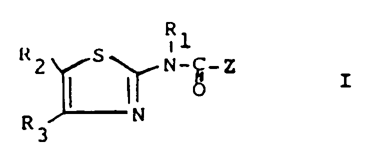 dans laquelle
R₁ représente H; alkyle en C₁ à C_{4,} phénylalkyle (C₁-C₃); un aminoalkyle -Z₁-NR₄R₅ dans laquelle Z_{1,} représente un alkylène en C₂ à C₄ et R₄ et R₅ représentent indépendamment H ou alkyle en C₁ à C₄ ou forment avec N un hétérocycle saturé et représentent morpholino, pyrrolidinyle, pipéridino, pipérazinyle ou (C₁-C₃)alkyl-4 pipérazinyle; carboxyalkyle -Z₂-COOR₆ dans laquelle Z₂ représente alkylène en C₁ à C₄ et R₆ représente H ou alkyle en C₁ à C₆; cyanoalkyle en C₂ à C₅; carbamoylalkyle -Z₃-CONR₇R₆ dans laquelle Z₃ représente un alkylène en C₁ à C₄ et R₇ et R₈ représentent indépendamment H ou un alkyle en C₁ à C₄ ou, avec N, un hétérocycle comme NR₄R₅; hydroxyalkyle en C₂ à C₆ ou alkoxyalkyle en C₂ à C₁₀;
R₂ représente H ou alkyle en C₁ à C₄;
R₃ représente un cycloalkyle en C₅ à C₈, éventuellement substitué par un ou des alkyles en C₁ à C₄; un groupe aromatique, tel qu'un phényle portant éventuellement un ou plusieurs substituants choisis parmi halogène, (C₁-C₆)alkyle, (C₁-C₃)alkoxy et (C₁-C₃)thioalkoxy, nitro, trifluorométhyle ou tel qu'un hétérocycle comportant au moins un hétéroatome choisi parmi O, S et N, et R₃ représente alors furyle, thiényle, pyrrolyle, pyrazolyle, imidazolyle, pyridyle, pyraziinyle, oxazolyle et thiazolyle, éventuellement substitués par (C₁ à C₃) alkyle ou halogène
ou R₂ et R₃ considérés ensemble représentent le groupe 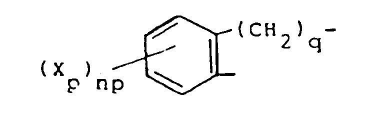 fixé par le carbone du phényle en position 4 du thiazolyle et dans lequel q vaut 1 à 4, et Xₚ représente les éventuels substituants choisis parmi halogène, (C₁-C₃)alkyle, (C₁-C₃)alkoxy, nitro et trifluorométhyle et np représente 0 à 3, et
Z représente un hétérocycle comportant un ou plusieurs hétéroatomes choisis parmi S et N, condensé avec un noyau aromatique qui peut comporter un hétéroatome et qui peut être substitué par un ou des groupes choisis parmi halogène, (C₁-C₃)alkyle, (C₁-C₃)alkoxy, benzyloxy, nitro, amino et trifluorométhyle, ainsi que les sels d'addition des composés de formule I avec des acides et des bases minérales ou organiques;
caractérisé en ce qu'on fait réagir sur l'aminothiazole de formule 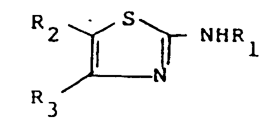 dans laquelle R₁, R₂ et R₃ ont la même signification que dans la formule I, une forme activée d'un acide de formule Z'COOH dans laquelle Z' représente Z comme dans la formule I ou un dérivé de Z dans lequel les fonctions sensibles ont éventuellement été protégées, puis le cas échéant on effectue une réaction de déprotection et une salification.

2. Procédé selon la revendication 1, caractérisé en ce que l'on prépare des composés de formule I dans laquelle Z représente benzothiényle, benzimidazolyle, benzothiazolyle, quinolyle, isoquinolyle, quinoxalinyle, quinazolinyle, cinnolinyle et thiéno/2,3-c/ ou /3,2-c/pyridyle, isoindolyle, isoindolinyle, indolyle ou indolinyle, éventuellement substitués par halogéno, (C₁-C₃)alkyle, (C₁-C₃)alkoxy, benzyloxy, nitro, amino et trifluorométhyle, les groupes indolyle et indolinyle étant éventuellement substitués sur l'azote par (C₁-C₄)alkyle, (C₂-C₆)hydroxyalkyle; (C₂-C₁₀)alkoxyalkyle; aminoalkyle -Z₄-NR₁₀R₁₁ dans laquelle Z₄ représente (C₂-C₄₎alkylène et R₁₀ et R₁₁ représentent indépendamment H, (C₁-C₄)alkyle ou R₁₀ et R₁₁ représentent avec N un hétérocycle saturé tel que morpholino, pyrrolindinyle, pipéridino, piperazinyle ou (C₁-C₃)alkyl-4 pipérazinyle; carboxyalkyle Z₅-COOR₁₂ dans laquelle Z₅ représente (C₁-C₄) alkylène et R₁₂ est H, benzyle ou (C₁-C₆) alkyle; carbamoylalkyle -Z₆-CONR₁₃R₁₄ dans laquelle Z₆ représente (C₁-C₄)alkylène et R₁₃ et R₁₄ représentent indépendamment H, (C₁-C₆)alkyle, ou forment avec N un hétérocycle saturé comme NR₁₀R₁₁; acyle -COR₁₅ avec R₁₅ représente (C₁-C₄)alkyle ou phényle; alkoxycarbonyle -COOR₁₆ avec R₁₆ étant tert-butyle ou benzyle.

3. Procédé selon la revendication 2, caractérisé en ce que l'on prépare des composés de formule I dans laquelle R₂ représente H, R₁ représente H, (C₁-C₄)alkyle ou -Z₁-NR₄R₅ avec Z₁, R₄, R₅ ayant les mêmes significations que dans la revendication 1, et Z représente un groupe indolyle substitué ou non sur l'azote comme à la revendication 2 et R₃ représente un phényle au moins orthosubstitué.

4. Procédé selon la revendication 1, caractérisé en ce que l'on prépare le N-[(triméthyl-2,4,6 phényl)-4 thiazolyl-2] indole carboxamide-2 et les composés substitués sur l'azote de l'indole par CH₃, CH₂COOH, CH₂COOCH₃, (CH₂)₂ N(CH₃)₂ et leurs sels pharmaceutiquement acceptables.

5. Procédé selon la revendication 1, caractérisé en ce que l'on prépare le N-[(triméthoxy-2,4,6 phényl)-4 thiazolyl-2] indole carboxamide-2 et les composés substitués sur l'azote de l'indole par CH₃, CH₂COOH, CH₂COOCH₃, (CH₂)₂ N(CH₃)₂ et leurs sels pharmaceutiquement acceptables.

6. Procédé selon la revendication 1, caractérisé en ce que l'on prépare le N-[(diméthyl-2,6 phényl)-4 thiazolyl-2] indole carboxamide-2 et les composés substitués sur l'azote de l'indole par CH₂COOH et CH₂COOCH₃, et leurs sels pharmaceutiquement acceptables.

7. Procédé selon la revendication 1, caractérisé en ce que l'on prépare le N-[(diméthoxy-2,6 phényl)-4 thiazolyl-2] indole carboxamide-2 et les composés substitués sur l'azote de l'indole par CH₂COOH et CH₂COOCH₃ et leurs sels pharmaceutiquement acceptables.

8. Procédé selon la revendication 1, caractérisé en ce que l'on prépare le N-[(dichloro-2,6 phényl)-4 thiazolyl-2]indole carboxamide-2 et les composés substitués à l'azote par CH₂COOH et (CH₂)₂ N(CH₃)₂ et leurs sels pharmaceutiquement acceptables.

9. Procédé selon la revendication 1, caractérisé en ce que l'on prépare le N-[(méthyl-2 phényl)-4 thiazolyl-2] indole carboxamide-2, N-[(méthoxy-2-phényl)-4-thiazolyl-2]indole carboxamide-2, N-[chloro-2 phényl)-4 thiazolyl-2] indole carboxamide-2 et les composés substitués à l'azote par CH₂COOH ainsi que leurs sels pharmaceutiquement acceptables.

10. Procédé selon la revendication 1, caractérisé en ce que l'on prépare le N-[(méthyl-4 phényl) -4 thiazolyl-2] indole carboxamide-2 et N-[(méthoxy-4 phényl)-4 thiazolyl-2] indole carboxamide-2 et leurs sels pharmaceutiquement acceptables.

11. Procédé de préparation des composés des revendications 1 a 10, de formule I dans laquelle Z représente le noyau indolyle, non substitué à l'azote et éventuellement substitué sur le phényle par halogéno, (C₁-C₃) alkyle, (C₁-C₃)alkoxy, benzyloxy, nitro, amino et trifluorométhyle caractérisé en ce que l'on prépare le composé de formule I dans laquelle Z représente le noyau indolinyle par acylation de l'aminothiazole avec une forme activée de l'acide de formule 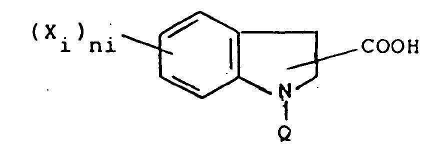 dans laquelle Q représente un groupe protecteur et Xᵢ représentent chacun, halogène, (C₁-C₃)alkyle, C₁-C₃)alkoxy, NO₂ ou CF₃, et ni vaut 0 à 3 puis que l'on déshydrogène le composé obtenu pour obtenir le dérivé indolyle correspondant, après avoir déprotége l'azote.

12. Procédé selon la revendication 11, caractérisé en ce que Q représente COO(t-C₄H₉) et que la déshydrogénation est effectuée par action de la tétrachloro-2,3,5,6 benzoquinone-1,4, de la dichloro-2,3 dicyano-5,6 benzoquinone-1,4 ou du cyclohexène en présence de Pd à une température supérieure à 100°C, dans un solvant inerte.

13. Procédé de préparation d'une composition pharmaceutique, caractérisé en ce que l'on associe au moins un composé obtenu selon l'une des revendications 1 à 12 avec au moins un excipient.

## Claims (Claims for the following Contracting State(s): AT, BE, CH, DE, DK, FR, GB, IT, LI, LU, NL, SE)

1. 2-Acylamino-thiazoles of formula 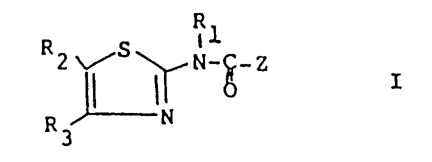 in which
R₁ represents H, C₁to C₄ alkyl, phenyl(C₁ to C₃)alkyl; an aminoalkyl-Z₁-NR₄R₅ in which Z₁ represents a C₂ to C₄ alkylene and R₄ and R₅ independently represent H or C₁ to C₄ alkyl or form with N a saturated heterocycle and represent morpholino, pyrrolidinyl, piperidino, piperazinyl or 4-(C₁ to C₃) alkyl-piperazinyl; carboxyalkyl-Z₂-COOR₆ in which Z₂ represents C₁ to C₄ alkylene and R₆ represents H or C₁ to C₆ alkyl; C₂ to C₅ cyanoalkyl; carbamoylalkyl-Z₃-CONR₇R₈ in which Z₃ represents a C₁ to C₄ alkylene and R₇and R₈ independently represent H or a C₁ to C₄ alkyl or, with N, a heterocycle NR₄R₅; C₂ to C₆ hydroxyalkyl or C₂ to C₁₀ alkoxyalkyl;
R₂ represents H or C₁ to C₄ alkyl;
R₃ represents a C₅ to C₈ cycloalkyl, optionally substituted by one or more C₁ to C₄ alkyl; an aromatic group, such as a phenyl optionally carrying one or more substituents chosen from halogen, (C₁-C₆) alkyl, (C₁-C₃) alkoxy and (C₁-C₃)thioalkoxy, nitro, trifluoromethyl or such as a heterocycle comprising at least one heteroatom chosen form O, S and N, and R₃ then represents furyl, thienyl, pyrrolyl, pyrazolyl, imidazoyl pyridyl, pyrazinyl, oxazolyl and thiazolyl, optionally substituted by (C₁ to C₃) alkyl or halogen
or R₂ and R₃ considered together represent the group 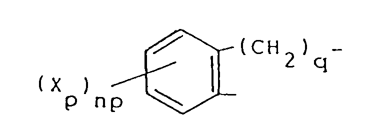 fixed by the phenyl carbon in position 4 of the thiazolyl and in which q is 1 to 4 and Xₚ represents the optional substituents chosen from halogen, (C₁-C₃) alkyl, (C₁-C₃) alkoxy, nitro and trifluoromethyl and np represents 0 to 3 and
Z represents a heterocycle comprising one or more heteroatoms chosen from S and N, fused with an aromatic ring which may comprise a heteroatom and which may be substituted by one or more of the groups chosen from halogen, (C₁-C₃) alkyl, (C₁-C₃) alkoxy, benzyloxy, nitro, amino and trifluormethyl, as well as the addition salts of the compounds of formula I with inorganic or organic acids and bases.

2. Compounds according to claim 1 of formula I in which Z represents benzothienyl, benzimidazolyl, benzothiazolyl, quinolyl,
isoquinolyl, quinoxalinyl, quinazolinyl, cinnolinyl and [2,3-c] or [3,2-c] thienopyridyl, isoindolyl, isoindolinlyl, indolyl, optionally substituted, and the indolyl and indolinyl groups being optionally substituted on the nitrogen by (C₁-C₄) alkyl, (C₂-C₆) hydroxyalkyl, (C₂-C₁₀) alkoxyalkyl, aminoalkyl-Z₄-NR₁₀R₁₁ in which Z₄ represents (C₂-C₄) alkylene and R₁₀ and R₁₁ independently represent H, (C₁-C₄) alkyl or R₁₀ and R₁₁ represents with N a saturated hetero group such as morpholino, pyrrolidinyl, piperidino, or 4-(C₁-C₃) alkylpiperazinyl, carboxyalkyl -Z₅COOR₁₂ in which Z₅ represents (C₁-C₄) alkylene and R₁₂ is H, benzyl or (C₁-C₆) alkyl, carbamoylalkyl - Z₆-CONR₁₃R₁₄ in which Z₆ represents (C₁-C₄) alkylene and R₁₃ and R₁₄ independently represent H, (C₁-C₆) alkyl, or form with N a saturated heterocycle such as NR₁₀R₁₁, acyl -COR₁₅ with R₁₅ representing (C₁-C₄) alkyl or phenyl, alkoxycarbonyl -COOR₁₆ with R₁₆ being tert-butyl or benzyl, and their salts.

3. Compounds according to claim 2, of formula I, in which R₂ represents H, (C₁-C₄) alkyl or -Z₁-NR₄R₅ with Z₁, R₄, R₅ having the same meanings as in claim 1, and Z representing an indolyl group which is substituted or unsubstituted on the nitrogen as in claims 2 and R₃ represents an at least ortho-substituted phenyl.

4. N-[4-(2,4,6-trimethylphenyl)-2- thiazolyl]-indole-2-carboxamide and its derivatives substituted on the indole nitrogen by CH₃, CH₂COOH, CH₂COOCH₃, (CH₂)₂N(CH₃)₂, and their pharmaceutically acceptable salts.

5. N-[4-(2,4,6-trimethoxyphenyl)-2- thiazolyl]-indole-2-carboxamide and its derivatives substituted on the indole nitrogen by CH₃, CH₂COOH, CH₂COOCH₃, (CH₂)₂N(CH₃)_{2,} and their pharmaceutically acceptable salts.

6. N-[4-(2,6-dimethylphenyl)-2-thiazolyl]-indole-2-carboxamide and its derivatives substituted on the indole nitrogen by CH₂COOH, CH₂COOCH_{3,} and their pharmaceutically acceptable salts.

7. N-[4-(2,6-dimethoxyphenyl)-2- thiazolyl]-indole-2-carboxamide and its derivatives substituted on the indole nitrogen by CH₂COOH, CH₂COOCH_{3,} and their pharmaceutically acceptable salts.

8. N-[4-(2,6-dichlorophenyl)-2-thiazolyl]-indole-2-carboxamide and its derivatives substituted on the nitrogen by CH₂COOH, (CH₂)₂N(CH₃)₂, and their pharmaceutically acceptable salts.

9. N-[4-(2-methylphenyl)-2-thiazolyl]-indole-2-carboxamide, N-[4-(2-methoxyphenyl)-2-thiazolyl]-indole-2-carboxamide, N-[4-(2-chlorophenyl)-2-thiazolyl]-indole-2-carboxamide, and their derivatives substituted on the nitrogen by CH₂COOH, and their pharmaceutically acceptable salts.

10. N-[4-(4-methylphenyl)-2-thiazolyl]-indole-2-carboxamide, and N-[4-(4-methoxyphenyl)-2-thiazolyl]-indole-2-carboxamide, and their pharmaceutically acceptable salts.

11. Process for the preparation of compounds of formula I according to one of claims 1 to 10, characterised in that an activated form of an acid of formula Z'COOH in which Z' represents Z as in the formula I or a derivative of Z in which the reactive groups have been protected if necessary, is reacted with the aminothiazole of formula 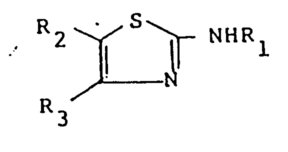 in which R₁, R₂ and R₃ have the same meaning as in formula I, and then if necessary a deprotection reaction is carried out.

12. Process for the preparation of compounds according to one of claims 1 to 10, of formula I in which Z represents the indolyl ring which is unsubstituted on the nitrogen and optionally substituted on the phenyl, characterised in that the compound corresponding to formula I in which Z represents the indolinyl ring is prepared by acylation of the aminothiazole with an activated form of the acid of formula 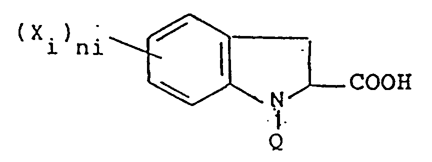 in which Q represents a protective group and Xᵢ represents halogen, (C₁-C₃) alkyl, (C₁-C₃) alkoxy, NO₂ or CF₃, and ni is 0 to 3, and the compound obtained is then dehydrogenated to obtain the corresponding indolyl derivative, after having deprotected the nitrogen.

13. Process according to claim 12, characterised in that Q represents COO(t-C₄H₉) and the dehydrogenation is carried out by reacting 2,3,5,6-tetrachloro-1-4-benzoquinone or cyclohexane in the presence of Pd at a temperature higher than 100° C, in a inert solvent.

14. Pharmaceutical composition, characterised in that it comprises at least one compound according to one of claims 1 to 10 in combination with at least one excipient.

## Claims (Claims for the following Contracting State(s): ES, GR)

1. Process for the preparation of 2-acylamino-thiazoles of formula 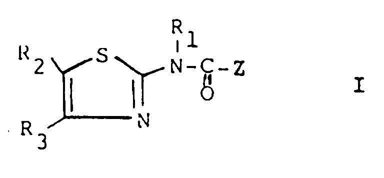 in which
R₁ represents H, C₁to C₄ alkyl, phenyl(C₁ to C₃)alkyl; an aminoalkyl-Z₁-NR₄R₅ in which Z₁ represents a C₂ to C₄ alkylene and R₄ and R₅ independently represent H or C₁ to C₄ alkyl or form with N a saturated heterocycle and represent morpholino, pyrrolidinyl, piperidino, piperazinyl or 4-(C₁ to C₃) alkyl-piperazinyl; carboxyalkyl-Z₂-COOR₆ in which Z₂ represents C₁ to C₄ alkylene and R₆ represents H or C₁ to C₆ alkyl; C₂ to C₅ cyanoalkyl; carbamoylalkyl-Z₃-CONR₇R₈ in which Z₃ represents a C₁ to C₄ alkylene and R₇and R₈ independently represent H or a C₁ to C₄ alkyl or, with N, a heterocycle NR₄R₅; C₂ to C₆ hydroxyalkyl or C₂ to C₁₀ alkoxyalkyl;
R₂ represents H or C₁ to C₄ alkyl;
R₃ represents a C₅ to C₈ cycloalkyl, optionally substituted by one or more C₁ to C₄ alkyl; a aromatic group, such as a phenyl optionally carrying one or more substituents chosen from halogen, (C₁-C₆) alkyl, (C₁-C₃) alkoxy and (C₁-C₃)thioalkoxy, nitro, trifluoromethyl or such as a heterocycle comprising at least one heteroatom chosen from O, S and N, and R₃ then represents furyl, thienyl, pyrrolyl, pyrazolyl, imidazoyl, pyridyl, pyrazinyl, oxazolyl and thiazolyl, optionally substituted by (C₁ to C₃) alkyl or halogen
or R₂ and R₃ considered together represent the group 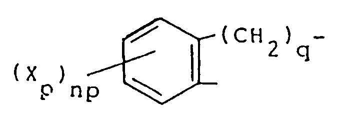 fixed by the phenyl carbon in position 4 of the thiazolyl and in which q is 1 to 4 and Xₚ represents the optional substituents chosen from halogen, (C₁-C₃) alkyl, (C₁-C₃) alkoxy, nitro and trifluoromethyl and np represents 0 to 3, and
Z represents a heterocycle comprising one or more heteroatoms chosen from S and N, fused with an aromatic ring which may comprise a heteroatom and which may be substituted by one or more of the groups chosen from halogen, (C₁-C₃) alkyl, (C₁-C₃) alkoxy, benzyloxy, nitro, amino and trifluoromethyl, as well as the addition salts of the compounds of formula I with inorganic or organic acids and bases, characterised in that an activated form of an acid of formula Z'COOH in which Z' represents Z as in the formula I or a derivative of Z in which the reactive groups have been protected if necessary, is reacted with the aminothiazole of formula 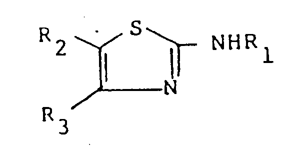 in which R₁, R₂ and R₃ have the same meaning as in formula I, and then if necessary a deprotection and desalification reaction is carried out.

2. Process according to claim 1, characterised in that compounds of formula I are prepared in which Z represents benzothienyl,
benzimidazolyl, benzothiazolyl, quinolyl, isoquinolyl, quinoxalinyl, quinazolinyl cinnolinyl and [2,3-c] or [3,2-c] thienopyridyl, isoindolyl, isoindolinyl, indolyl, optionally substituted, and the indolyl and indolinyl groups being optionally substituted on the nitrogen by (C₁-C₄) alkyl, (C₂-C₆) hydroxyalkyl, (C₂-C₁₀) alkoxyalkyl, aminoalkyl-Z₄-NR₁₀R₁₁ in which Z₄ represents (C₂-C₄) alkylene and R₁₀ and R₁₁ independently represent H, (C₁-C₄) alkyl or R₁₀ and R₁₁ represent with N a saturated hetero group such as morpholino, pyrrolidinyl, piperidino, or 4-(C₁-C₃) alkylpiperazinyl, carboxyalkyl -Z₅COOR₁₂ in which Z₅ represents (C₁-C₄) alkylene and R₁₂ is H, benzyl or (C₁-C₆) alkyl, carbamoylalkyl, -Z₆-CONR₁₃R₁₄ in which Z₆ represents (C₁-C₄) alkylene and R₁₃ and R₁₄ independently represent H, (C₁-C₆) alkyl, or form with N a saturated heterocycle such as NR₁₀R₁₁, acyl -COR₁₅ with R₁₅ representing (C₁-C₄) alkyl or phenyl, alkoxycarbonyl -COOR₁₆ with R₁₆ being tert-butyl or benzyl.

3. Process according to claim 2, characterised in that compounds of formula I are prepared, in which R₂ represents H, (C₁-C₄) alkyl or -Z₁-NR₄R₅ with Z₁, R₄, R₅ having the same meanings as in claim 1, and Z representing an indolyl group which is substituted or unsubstituted on the nitrogen as in claims 2 and R₃ represents an at least ortho-substituted phenyl.

4. Process according to claim 1, characterised in that N-[4-(2,4,6-trimethylphenyl)-2- thiazoly]-indole-2-carboxamide and its derivatives substituted on the indole nitrogen by CH₃, CH₂COOH, CH₂COOCH₃, (CH₂)₂N(CH₃)₂, and their pharmaceutically acceptable salts are prepared.

5. Process according to claim 1, characterised in that N-[4-(2,4,6-trimethoxyphenyl)-2- thiazolyl]-indole-2-carboxamide and its derivatives substituted on the indole nitrogen by CH₃, CH₂COOH, CH₂COOCH₃, (CH₂)₂N(CH₃)_{2,} and their pharmaceutically acceptable salts are prepared.

6. Process according to claim 1, characterised in that N-[4-(2,6-dimethylphenyl)-2- thiazolyl]-indole-2-carboxamide and its derivatives substituted on the indole nitrogen by CH₂COOH, CH₂COOCH_{3,} and their pharmaceutically acceptable salts are prepared.

7. Process according to claim 1, characterised in that N-[4-(2,6-dimethoxyphenyl)-2- thiazolyl]-indole-2-carboxamide and its derivatives substituted on the indole nitrogen by CH₂COOH, CH₂COOCH_{3,} and their pharmaceutically acceptable salts are prepared.

8. Process according to claim 1, characterised in that N-[4-(2,6-dichlorophenyl)-2- thiazolyl]-indole-2-carboxamide and its derivatives substituted on the nitrogen by CH₂COOH, (CH₂)₂N(CH₃)₂, and their pharmaceutically acceptable salts are prepared..

9. Process according to claim 1, characterised in that N-[4-(2-methylphenyl)-2-thiazolyl]-indole-2-carboxamide, N-[4-(2-methoxyphenyl)-2-thiazoly]-indole-2-carboxamide, N-[4-(2-chlorophenyl)-2-thiazolyl]-indole-2-carboxamide, and their derivatives substituted on the nitrogen by CH₂COOH, and their pharmaceutically acceptable salts are prepared.

10. Process according to claim 1, characterised in that N-[4-(4-methylphenyl)-2-thiazoly]-indole-2-carboxamide, and N-[4-(4-methoxyphenyl)-2-thiazolyl]-indole-2-carboxamide, and their pharmaceutically acceptable salts are prepared.

11. Process for the preparation of compounds according to one of claims 1 to 10, of formula I in which Z represents the indolyl ring which is unsubstituted on the nitrogen and optionally substituted on the phenyl by halogen, (C₁₋₃)alkyl, (C₁₋₃)alkoxy, benzyloxy, nitro, amino and trifluoromethyl, characterised in that the compound corresponding to formula I in which Z represents the indolinyl ring is prepared by acylation of the aminothiazole with an activated form of the acid of formula 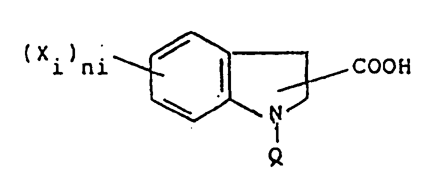 in which Q represents a protective group and each Xᵢ represents halogen, (C₁-C₃)alkyl, (C₁-C₃)alkoxy, NO₂ or CF₃, and ni is 0 to 3, and the compound obtained is then dehydrogenated to obtain the corresponding indolyl derivative, after having deprotected the nitrogen.

12. Process according to claim 11, characterised in that Q represents COO(t-C₄H₉) and the dehydrogenation is carried out by reacting 2,3,5,6-tetrachloro-1,4-benzoquinone, 2,3-dichloro-5,6-dicyano-benzoquinone or cyclohexene in the presence of Pd at a temperature higher than 100°C, in an inert solvent.

13. Process for preparing a pharmaceutical composition, characterised in that at least one compound according to one of claims 1 to 12 is combined with at least one excipient.

## Patentansprüche (Patentansprüche für folgende(n) Vertragsstaat(en): AT, BE, CH, DE, DK, FR, GB, IT, LI, LU, NL, SE)

1. 2-Acylamino-thiazole der Formel 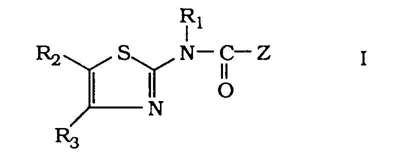 in der
R₁ H; C₁-C₄-Alkyl; Phenyl-(C₁-C₃)-alkyl; Aminoalkyl -Z₁-NR₄R₅, worin Z₁ C₂-C₄-Alkylen und R₄ and R₅ unabhängig voneinander H oder C₁-C₄-Alkyl oder gemeinsam mit N einen gesättigten Heterocyclus bilden und Morpholino, Pyrrolidinyl, Piperidino, Piperazinyl oder 4-(C₁-C₃)-Alkyl-piperazinyl darstellen; Carboxyalkyl -Z₂-COOR₆, worin Z₂ C₁-C₄-Alkylen und R₆ H oder C₁-C₆-Alkyl; C₂-C₅-Cyanoalkyl: Carbamoylalkyl -Z₃-CONR₇R₈, worin Z₃ C₁-C₄-Alkylene und R₇ und R₈ unabhängig voneinander H oder C₁-C₄-Alkyl oder zusammen mit N einen Heterocyclus NR₄R₅; C₂-C₆-Hydroxyalkyl oder C₂-C₁₀-Alkoxyalkyl;
R₂ H oder C₁-C₄-Alkyl;
R₃ C₅-C₈-Cycloalkyl, das gegebenenfalls durch ein oder mehrere C₁-C₄-Alkyl substituiert ist; eine aromatische Gruppe, wie eine Phenylgruppe, die gegebenenfalls einen oder mehrere Substituenten ausgewählt aus Halogen, (C₁-C₆)-Alkyl, (C₁-C₃)-Alkoxy und (C₁-C₃)-Thioalkoxy, Nitro- oder Trifluoromethyltragen kann, oder einen Heterocyclus, der mindestens ein Heteroatom ausgewählt aus S und N aufweist und R₃ dann Furyl, Thienyl, Pyrrolyl, Pyrazolyl, Imidazolyl, Pyridyl, Pyrazinyl, Oxazolyl und Thiazolyl, die gegenenenfalls durch (C₁-C₃)-Alkyl oder Halogen substituiert sind, darstellt,
oder R₂ und R₃ gemeinsam die Gruppe 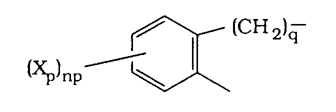 die über das Kohlenstoffatom des Phenylrestes in der 4-Stellung des Thiazolylrests gebunden ist und in der q 1 bis 4, Xₚ gegebenenfalls vorhandene Substituenten ausgewählt aus Halogen, (C₁-C₃)-Alkyl, (C₁-C₃)-Alkoxy, Nitro- und Trifluormethyl, und np 0 bis 3 darstellen und
Z einen Heterocyclus, der ein oder mehrere Heteroatome ausgewählt aus S und N aufweist, der mit einem aromatischen Rest kondensiert ist, welcher ein Heteroatom aufweisen kann und der durch ein oder mehrere Gruppen ausgewählt aus Halogen, (C₁-C₃)-Alkyl, (C₁-C₃)-Alkoxy, Benzyloxy, Nitro, Amino und Trifluormethyl substituiert sein kann,
bedeuten, sowie die Additionssalze der Verbindungen der Formel I mit anorganischen oder organischen Säuren und Basen.

2. Verbindungen nach Anspruch 1 der Formel I, worin Z Benzothienyl, Benzimidazolyl, Benzothiazolyl, Chinolyl, Isochinolyl, Chinoxalinyl, Chinazolinyl, Cinnolinyl und Thieno[2,3-c] oder [3,2-c]pyridyl, Isoindolyl, Isoindolinyl, Indolyl oder Indolinyl darstellt, die gegebenenfalls substituiert sind, wobei die Indolyl- und Indolinyl-Gruppen gegebenenfalls am Stickstoff substituiert sind, durch (C₁-C₄)-Alkyl, (C₂-C₆)-Hydroxyalkyl; (C₂-C₁₀)-Alkoxyalkyl; Aminoalkyl -Z₄-NR₁₀R₁₁, worin Z₄ (C₁-C₄)-Alkylen und R₁₀ und R₁₁ unabhängig voneinander H, (C₁-C₄)-Alkyl darstellen oder R₁₀ und R₁₁ zusammen mit N einen gesättigten Heterocyclus bilden, wie Morpholino, Pyrrolidinyl, Piperidino, Piperazinyl oder 4-(C₁-C₃)-Alkyl-piperazinyl; Carboxyalkyl -Z₅-COOR₁₂, worin Z₅ (C₁-C₄)-Alkylen und R₁₂ H, Benzyl oder (C₁-C₆)-Alkyl darstellen; Carbamoylalkyl -Z₆-CONR₁₃R₁₄, worin Z₆ (C₁-C₄)-Alkylen und R₁₃ und R₁₄ unabhängig voneinander H, (C₁-C₆)-Alkyl oder gemeinsam mit N einen gesättigten Heterocyclus, wie NR₁₀R₁₁ darstellen; Acyl -COR₁₅, worin R₁₅ (C₁-C₄)-Alkyl oder Phenyl darstellt; Alkoxycarbonyl COOR₁₆, worin R₁₆ tert.Butyl oder Benzyl darstellt; und deren Salze.

3. Verbindungen nach Anspruch 2 der Formel I, worin R₂ H, R₁, H, (C₁-C₄)-Alkyl oder -Z₁-NR₄R₅, worin Z₁, R₄ und R₅ die in Anspruch 1 angegebenen Bedeutungen besitzen, und Z eine gegebenenfalls am Stickstoffatom substituierte Indo-lylgruppe, wie es in Anspruch 2 angegeben ist, und R₃ eine mindestens orthosubstituierte Phenylgruppe bedeuten.

4. N-[4-(2,4,6-Trimethyl-phenyl)-thiazol-2-yl]-indol-2-carboxamid und dessen am Indol-Stickstoff durch CH₃, CH₂COOH, CH₂COOCH₃, (CH₂)₂ N(CH₃)₂ substituierten Verbindungen sowie deren pharmazeutisch annehmbare Salze.

5. N-[4-(2,4,6-Trimethoxy-phenyl)-thiazol-2-yl]-indol-2-carboxamid und dessen am Indol-Stickstoff durch CH₃, CH₂COOH, CH₂COOCH₃ oder (CH₂)₂ N(CH₃)₂ substituierten Verbindungen sowie deren pharmazeutisch annehmbare Salze.

6. N-[4-(2,6-Dimethyl-phenyl)-thiazol-2-yl]-indol-2-carboxamid und dessen am Indol-Stickstoff durch CH₂COOH und CH₂COOCH₃ substituierten Verbindungen sowie deren pharmazeutisch annehmbare Salze.

7. N-[4-(2,6-Dimethoxy-phenyl)-thiazol-2-yl]-indol-2-carboxamid und dessen am Indol-Stickstoff durch CH₂COOH und CH₂COOCH₃ substituierten Verbindungen sowie deren pharmazeutisch annehmbare Salze.

8. N-[4-(2,6-Dichlor-phenyl)-thiazol-2-yl]-indol-2-carboxamid und die am Stickstoff durch CH₂COOH und (CH₂)₂ N(CH3)2 substituierten Verbindungen und deren pharmazeutisch annehmbare Salze.

9. N-[4-(2-Methyl-phenyl)-thiazol-2-yl]-indol-2-carboxamid, N-[4-(2-Methoxy-phenyl)-thiazol-2-yl]-indol-2-carboxamid, N-[4-(2-Chlor-phenyl)-thiazol-2-yl]-indol-2-carboxamid und deren am Stickstoff durch CH₂COOH substituierten Verbindungen sowie deren pharmazeutisch annehmbare Salze.

10. N-[4-(4-Methyl-phenyl)-thiazol-2-yl]-indol-2-carboxamid und N-[4-(4-Methoxy-phenyl)-thiazol-2-yl]-indol-2-carboxamid und deren pharmazeutisch annehmbare Salze.

11. Verfahren zur Herstellung der Verbindungen der Formel I nach einem der Ansprüche 1 bis 10, **dadurch gekennzeichnet**, daß man auf ein Aminothiazol der Formel 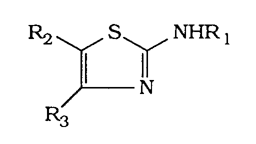 in der R₁, R₂ und R₃ die bezüglich der Formel I angegebenen Bedeutungen besitzen,
eine aktivierte Form einer Säure der Formel Z'COOH, worin Z' die für Z bezüglich der Formel I angegebenen Bedeutungen besitzt oder ein Derivat von Z, worin die empfindlichen Gruppen gegebenenfalls geschützt sind, bedeutet, einwirken läßt und gegebenenfalls eine Reaktion zur Abspaltung der Schutzgruppen durchführt.

12. Verfahren zur Herstellung der Verbindungen nach einem der Ansprüche 1 bis 10 der Formel I, worin Z einen Indolylkern bedeutet, der am Stickstoff nicht substituiert ist und gegebenenfalls am Phenylrest substituiert ist, **dadurch gekennzeichnet**, daß man die entsprechende Verbindung der Formel I, in der Z den Indolinylkern bedeutet, durch Acylieren des Aminothiazols mit einer aktivierten Form der Säure der Formel 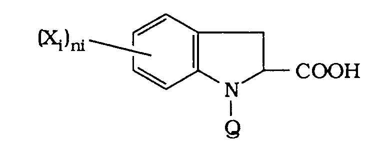 in der Q eine Schutzgruppe und X₁ jeweils Halogen, (C₁-C₃)-Alkyl, (C₁-C₃)-Alkoxy, NO₂ oder CF₃ und ni 0 bis 3 bedeuten, herstellt und dann die erhaltene Verbindung dehydriert, so daß man nach dem Abspalten der Schutzgruppe des Stickstoffs das entsprechende Indolylderivat erhält.

13. Verfahren nach Anspruch 12, **dadurch gekennzeichnet**, daß Q eine Gruppe der Formel COO(t-C₄H₉) darstellt und die Dehydrierung durch Einwirkung von 2,3,5,6-Tetrachlor-1,4-benzochinon, 2,3-Dichlor-5,6-dicyano-1,4-benzochinon oder Cyclohexen in Gegenwart von Pd bei einer Temperatur oberhalb 100°C in einem inerten Lösungsmittel bewirkt wird.

14. Pharmazeutische Zubereitung, **dadurch gekennzeichnet**, daß sie mindestens eine Verbindung nach einem der Ansprüche 1 bis 10 in Kombination mit mindestens einem Trägermaterial umfaßt.

## Patentansprüche (Patentansprüche für folgende(n) Vertragsstaat(en): ES, GR)

1. Verfahren zur Herstellung von 2-Acylamino-thiazolen der Formel 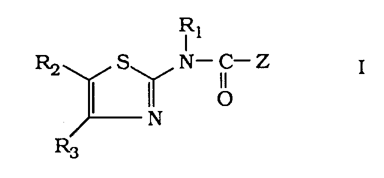 in der
R₁ H; C₁-C₄-Alkyl; Phenyl-(C₁-C₃)-alkyl; Aminoalkyl -Z₁-NR₄R₅, worin Z₁ C₂-C₄-Alkylen und R₄ und R₅ unabhängig voneinander H oder C₁-C₄-Alkyl oder gemeinsam mit N einen gesättigten Heterocyclus bilden und Morpholino, Pyrrolidinyl, Piperidino, Piperazinyl oder 4-(C₁-C₃)-Alkyl-piperazinyl darstellen; Carboxyalkyl -Z₂-COOR₆, worin Z₂ C₁-C₄-Alkylen und R₆ H oder C₁-C₆-Alkyl; C₂-C₅-Cyanoalkyl; Carbamoylalkyl-Z₃-CONR₇R₈, worin Z₃ C₁-C₄-Alkylen und R₇ und R₈ unabhängig voneinander H oder C₁-C₄-Alkyl oder zusammen mit N einen Heterocyclus NR₄R₅; C₂-C₆-Hydroxyalkyl oder C₂-C₁₀-Alkoxyalkyl;
R₂ H oder C₁-C₄-Alkyl;
R₃ C₅-C₈-Cycloalkyl, das gegebenenfalls durch ein oder mehrere C₁-C₄-Alkyl substituiert ist; eine aromatische Gruppe, wie eine Phenylgruppe, die gegebenenfalls einen oder mehrere Substituenten ausgewählt aus Halogen, (C₁-C₆)-Alkyl, (C₁-C₃)-Alkoxy und (C₁-C₃)-Thioalkoxy, Nitro- oder Trifluormethyl tragen kann, oder einen Heterocyclus, der mindestens ein Heteroatom ausgewählt aus O, S und N aufweist und R₃ dann Furyl, Thienyl, Pyrrolyl, Pyrazolyl, Imidazolyl, Pyridyl, Pyrazinyl, Oxazolyl und Thiazolyl, die gegebenenfalls durch (C₁-C₃)-Alkyl oder Halogen substituiert sind, darstellt,
oder R₂ und R₃ gemeinsam die Gruppe 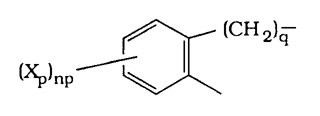 die über das Kohlenstoffatom des Phenylrestes in der 4-Stellung des Thiazolylrests gebunden ist und in der q 1 bis 4, Xₚ gegebenenfalls vorhandene Substituenten ausgewählt aus Halogen, (C₁-C₃)-Alkyl, (C₁-C₃)-Alkoxy, Nitro- und Trifluormethyl, und np 0 bis 3 darstellen und
Z einen Heterocyclus, der ein oder mehrere Heteroatome ausgewählt aus S und N aufweist, der mit einem aromatischen Rest kondensiert ist, welcher ein Heteroatom aufweisen kann und der durch ein oder mehrere Gruppen ausgewählt aus Halogen, (C₁-C₃)-Alkyl, (C₁-C₃)-Alkoxy, Benzyloxy, Nitro, Amino und Trifluormethyl substituiert sein kann,
bedeuten, sowie von den Additionssalzen der Verbindungen der Formel I mit anorganischen oder organischen Säuren und Basen, **dadurch gekennzeichnet**, daß man auf ein Aminothiazol der Formel 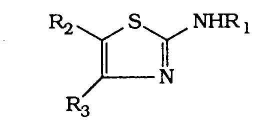 in der R₁, R₂ und R₃ die bezüglich der Formel I angegebenen Bedeutungen besitzen,
eine aktivierte Form einer Säure der Formel Z'COOH, worin Z' die für Z bezüglich der Formel I angegebenen Bedeutungen besitzt oder ein Derivat von Z, worin die empfindlichen Gruppen gegebenenfalls geschützt sind, bedeutet, einwirken läßt und gegebenenfalls eine Reaktion zur Abspaltung der Schutzgruppen durchführt.

2. Verfahren nach Anspruch 1, **dadurch gekennzeichnet**, daß man Verbindungen der Formel I herstellt, in der Z Benzothienyl, Benzimidazolyl, Benzothiazolyl, Chinolyl, Isochinolyl, Chinoxalinyl, Chinazolinyl, Cinnolinyl und Thieno[2,3-c] oder [3,2-c]pyridyl, Isoindolyl, Isoindolinyl, Indolyl oder Indolinyl darstellt, die gegebenenfalls substituiert sind, wobei die Indolyl- und Indolinyl-Gruppen gegebenenfalls am Stickstoff substituiert sind, durch (C₁-C₄)-Alkyl, (C₂-C₆)-Hydroxyalkyl; (C₂-C₁₀)-Alkoxyalkyl; Aminoalkyl -Z₄-NR₁₀R₁₁, worin Z₄ (C₁-C₄)-Alkylen und R₁₀ und R₁₁ unabhängig voneinander H, (C₁-C₄)-Alkyl darstellen oder R₁₀ und R₁₁ zusammen mit N einen gesättigten Heterocyclus bilden, wie Morpholino, Pyrrolidinyl, Piperidino, Piperazinyl oder 4-(C₁-C₃)-Alkylpiperazinyl; Carboxyalkyl -Z₅-COOR₁₂, worin Z₅ (C₁-C₄)-Alkylen und R₁₂ H, Benzyl oder (C₁-C₆)-Alkyl darstellen; Carbamoylalkyl -Z₆-CONR₁₃R₁₄, worin Z₆ (C₁-C₄)-Alkylen und R₁₃ und R₁₄ unabhängig voneinander H, (C₁-C₆)-Alkyl oder gemeinsam mit N einen gesättigten Heterocyclus, wie NR₁₀R₁₁ darstellen; Acyl -COR₁₅, worin R₁₅ (C₁-C₄)-Alkyl oder Phenyl darstellt; Alkoxycarbonyl COOR₁₆, worin R₁₆ tert.-Butyl oder Benzyl darstellt; und deren Salze.

3. Verfahren nach Anspruch 2, **dadurch gekennzeichnet**, daß man Verbindungen der Formell herstellt, in der R₂ H, R₁ H, (C₁-C₄)-Alkyl oder -Z₁-NR₄R₅, worin Z₁, R₄ und R₅ die in Anspruch 1 angegebenen Bedeutungen besitzen, und Z eine gegebenenfalls am Stickstoffatom substituierte Indolylgruppe, wie es in Anspruch 2 angegeben ist, und R₃ eine mindestens orthosubstituierte Phenylgruppe bedeuten.

4. Verfahren nach Anspruch 1, **dadurch gekennzeichnet**, daß man N-[4-(2,4,6-Trimethyl-phenyl)-thiazol-2-yl]-indol-2-carboxamid und die am Indol-Stickstoff durch CH₃, CH₂COOH, CH₂COOCH₃, (CH₂)₂ N(CH₃)₂ substituierten Verbindungen und deren pharmazeutisch annehmbare Salze herstellt.

5. Verfahren nach Anspruch 1, **dadurch gekennzeichnet**, daß man N-[4-(2,4,6-Trimethoxy-phenyl)-thiazol-2-yl]-indol-2-carboxamid und dessen am Indol-Stickstoff durch CH₃, CH₂COOH, CH₂COOCH₃ oder (CH₂)₂N(CH₃)₂ substituierten Verbindungen sowie deren pharmazeutisch annehmbare Salze herstellt.

6. Verfahren nach Anspruch 1, **dadurch gekennzeichnet**, daß man N-[4-(2,6-Dimethyl-phenyl)-thiazol-2-yl]-indol-2-carboxamid und dessen am Indol-Stickstoff durch CH₂COOH und CH₂COOCH₃ substituierten Verbindungen sowie deren pharmazeutisch annehmbare Salze herstellt.

7. Verfahren nach Anspruch 1, **dadurch gekennzeichnet**, daß man N-[4-(2,6-Dimethoxy-phenyl)-thiazol-2-yl]-indol-2-carboxamid und dessen am Indol-Stickstoff durch CH₂COOH und CH₂COOCH₃ substituierten Verbindungen sowie deren pharmazeutisch annehmbare Salze herstellt.

8. Verfahren nach Anspruch 1, **dadurch gekennzeichnet**, daß man N-[4-(2,6-Dichlor-phenyl)-thiazol-2-yl]-indol-2-carboxamid und die am Stickstoff durch CH₂COOH und (CH₂)₂ N(CH3)2 substituierten Verbindungen und deren pharmazeutisch annehmbare Salze herstellt.

9. Verfahren nach Anspruch 1, **dadurch gekennzeichnet**, daß man N-[4-(2-Methyl-phenyl)-thiazol-2-yl]-indol-2-carboxamid, N-[4-(2-Methoxy-phenyl)-thiazol-2-yl]-indol-2-carboxamid, N-[4-(2-Chlor-phenyl)-thiazol-2-yl]-indol-2-carboxamid und deren am Stickstoff durch CH₂COOH substituierten Verbindungen sowie deren pharmazeutisch annehmbare Salze herstellt.

10. Verfahren nach Anspruch 1, **dadurch gekennzeichnet**, daß man N-[4-(4-Methyl-phenyl)-thiazol-2-yl]-indol-2-carboxamid und N-[4-[4-Methoxy-phenyl)-thiazol-2-yl]-indol-2-carboxamid und deren pharmazeutisch annehmbare Salze herstellt.

11. Verfahren zur Herstellung der Verbindungen der Ansprüche 1 bis 10 der Formel I, In der Z einen Indolylrest darstellt, der am Stickstoffatom nicht substituiert ist und gegebenenfalls am Phenylrest durch Halogen, (C₁-C₃)-Alkyl, (C₁-C₃)-Alkoxy, Benzyloxy, Nitro, Amino und Trifluormethyl substituiert ist, **dadurch gekennzeichnet**, daß man die entsprechende Verbindung der Formel I, in der Z den Indolinylkern bedeutet, durch Acylieren des Aminothiazols mit einer aktivierten Form der Säure der Formel 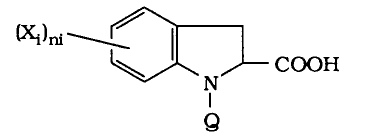 in der Q eine Schutzgruppe und Xₗ jeweils Halogen, (C₁-C₃)-Alkyl, (C₁-C₃)-Alkoxy, NO₂ oder CF₃ und ni 0 bis 3 bedeuten, herstellt und dann die erhaltene Verbindung dehydriert, so daß man nach dem Abspalten der Schutzgruppe des Stickstoffs das entsprechende Indolylderivat erhält.

12. Verfahren nach Anspruch 11, **dadurch gekennzeichnet**, daß Q eine Gruppe der Formel COO(t-C₄H₉) darstellt und die Dehydrierung durch Einwirkung von 2,3,5,6-Tetrachlor-1,4-benzochinon, 2,3-Dichlor-5,6-dicyano-1,4-benzochinon oder Cyclohexen in Gegenwart von Pd bei einer Temperatur oberhalb 100°C in einem inerten Lösungsmittel bewirkt wird.

13. Verfahren zur Herstellung einer pharmazeutischen Zubereitung, **dadurch gekennzeichnet**, daß man mindestens eine nach einem der Ansprüche 1 bis 12 erhaltene Verbindung mit mindestens einem Trägermaterial vereinigt.
